# EUROPEAN PATENT SPECIFICATION

(11) **EP 2 853 532 B1**
(45) Date of publication and mention of the grant of the patent: **09.12.2020**
(21) Application number: 13460066.7
(22) Date of filing: 28.09.2013
(51) Int. Cl.: C07D 413/12, C07D 271/06, A61K 31/4245, A61K 31/4439, A61K 31/506, A61P 25/00, A61P 29/00

(54) **1,2,4-oxadiazole derivatives as allosteric modulators of metabotropic glutamate receptors belonging to group III**
1,2,4-Oxadiazoldrivate als allosterische Modulatoren von metabotropen Glutamatrezeptoren, die zur Gruppe-III gehören
Dérivés 1,2,4-oxadiazoliques comme modulateurs allostériques des récepteurs du glutamate métabotropique du groupe III

(43) Date of publication of application: 01.04.2015
(73) Proprietor: Instytut Farmakologii Polskiej Akademii Nauk, 31-343 Krakow (PL)
(72) Inventor: Pilc, Andrzej, 31-352 Kraków (PL); Bojarski, Andrzej J., 32-080 Zabierzów (PL); Stankiewicz, Anna, 30-033 Kraków (PL); Branski, Piotr, 30-209 Kraków (PL); Burnat, Grzegorz, 30-837 Kraków (PL); Bugno, Ryszard, 30-149 Kraków (PL)
(74) Representative: Patpol Kancelaria Patentowa Sp. z o.o.

(56) References cited:
- EP-A1- 0 504 574
- WO-A1-01/12627
- WO-A1-2008/076356
- WO-A1-2009/041972
- WO-A1-2009/146343
- WO-A1-2009/151529
- WO-A2-02/068417
- WO-A2-03/024448
- WO-A2-2004/054973
- WO-A2-2004/110351
- WO-A2-2009/043889
- DE-A1- 4 425 794
- JP-A- 2011 195 467
- US-A- 6 034 106
- US-A1- 2009 176 842
- US-A1- 2009 298 894
- US-A1- 2010 249 085
- TSUYOSHI NAKAMURA ET AL: "Discovery of CS-2100, a potent, orally active and S1P-sparing S1Pagonist", BIOORGANIC & MEDICINAL CHEMISTRY LETTERS, PERGAMON, AMSTERDAM, NL, vol. 22, no. 4, 3 December 2011 (2011-12-03), pages 1788-1792, XP028398245, ISSN: 0960-894X, DOI: 10.1016/J.BMCL.2011.12.019 [retrieved on 2012-01-02]
- DALIP KUMAR ET AL: "Design and Synthesis of 3,5-Disubstituted-1,2,4-Oxadiazoles as Potent Inhibitors of Phosphodiesterase4B2", CHEMICAL BIOLOGY & DRUG DESIGN, vol. 79, no. 5, 9 May 2012 (2012-05-09), pages 810-818, XP055110314, ISSN: 1747-0277, DOI: 10.1111/j.1747-0285.2011.01304.x
- MOLONEY ET AL: "Synthesis and cannabinoid activity of 1-substituted-indole-3-oxadiazole derivatives: Novel agonists for the CB1 receptor", EUROPEAN JOURNAL OF MEDICINAL CHEMISTRY, EDITIONS SCIENTIFIQUE ELSEVIER, PARIS, FR, vol. 43, no. 3, 1 March 2008 (2008-03-01), pages 513-539, XP022511502, ISSN: 0223-5234, DOI: 10.1016/J.APCATA.2007.12.019
- SEVERSON W E ET AL: "High-throughput screening of a 100,000-compound library for inhibitors of influenza A virus (H3N2)", JOURNAL OF BIOMOLECULAR SCREENING, SAGE; LIEBERT, US, vol. 13, no. 9, 1 October 2008 (2008-10-01), pages 879-887, XP002733966, ISSN: 1087-0571, DOI: 10.1177/1087057108323123 [retrieved on 2008-09-23]
- RECEVEUR JEAN-MARIE ET AL: "4-Acylamino-and 4-ureidobenzamides as melanin-concentrating hormone (MCH) receptor 1 antagonists", BIOORGANIC & MEDICINAL CHEMISTRY LETTERS, vol. 14, no. 20, 20 August 2004 (2004-08-20), pages 5075-5080, XP029246114, ISSN: 0960-894X, DOI: 10.1016/J.BMCL.2004.07.077
- WANG YING ET AL: "Rapid and efficient synthesis of 1,2,4-oxadiazoles utilizing polymer-supported reagents under microwave heating", ORGANIC LETTERS,, vol. 7, no. 5, 3 March 2005 (2005-03-03), pages 925-928, XP002540749, DOI: 10.1021/OL050007R
- Werner Krone: "Ueber p-Oxybensenylamidoxim und Abkommlinge desselben", Berichte der deutschen chemischen Gesellschaft, vol. 24, 1 January 1891 (1891-01-01), pages 834-841, XP055336744, DOI: 10.1002/cber.189102401158

## Description

### Technical Field

The present invention relates to 1,2,4-oxadiazole derivatives for use as positive allosteric modulator ligands of metabotropic glutamate receptors of group III subtype 4 (mGluR4) in the treatment of the central nervous disorders modulated by mGluR4 receptors. In accordance to the invention revealed compounds might be use, optionally in the form of pharmaceutically approved salts, in the treatment, either alone or in combination with already existing drugs, of the central nervous disorders such as Parkinson's disease, anxiety, schizophrenia, pain all other disorders modulated by mGlu4 receptors. Additionally, disclosed are other 1,2,4-oxadiazole derivatives of general Formula I, wherein A, B, M, X, Y, R₁, R₂ and R₃ are further defined

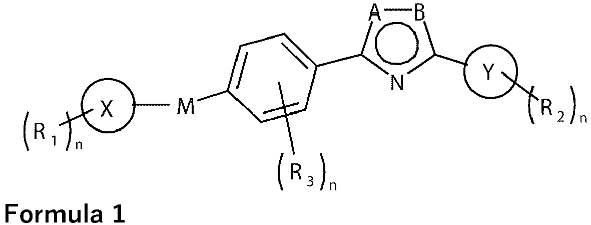

### Background Art

L-glutamate (referred to herein simply as glutamate) is the main excitatory neurotransmitter in the mammalian central nervous system (CNS) and acts in three different cell compartments - the pre- and postsynaptic neurons and glia-that together characterize the "tripartite glutamate synapse" [Machado-Vieira et al., (2009) Neuroscientist, 15:525-539]. Together with GABA, the major inhibitory transmitter, they ensure the appropriate functioning of the brain [Slawinska et al., (2013) Neuropharmacology, 66: 225-235]. Glutamate is produced from *α*-ketoglutarate, an intermediate in the Krebs cycle, and is packaged into secretory vesicles in the presynaptic neuron by glutamate transporters. After release in an activity-dependent process, glutamate is taken by astrocytes and converted to glutamine by the enzyme glutamine synthetase. Glutamine released by astrocytes is transported back to presynaptic neurons, oxidized back into glutamate by the enzyme glutaminase, and repackaged [Machado-Vieira et al., (2012) Pharmacol. Biochem. Behav. 100:678-687].

Glutamate mediates synaptic neurotransmission trough the activation of two classes of receptors, the multi-subunit ion channel ionotropic glutamate receptors (iGluRs) and the G-protein coupled (GPCRs) metabotropic glutamate receptors (mGluRs) [O'Connor et al., (2013) Pharmacol. Biochem. Behav., 103:561-567]. The first class, the ionotropic glutamate receptors, is responsible for fast excitatory transmission. Three subtypes of ionotropic glutamate receptors are named after their respective selective ligands/agonists: AMPA, NMDA and kainite receptors [Machado-Vieira et al., (2012) Pharmacol. Biochem. Behav., 100:678-687].

The second class, the metabotropic glutamate receptors, mediate a slower, more modulatory response to glutamate signaling, based on their location (pre- or post-synaptic). The mGluRs consist of an extracellular N-terminal domain, seven-strand transmembrane spanning (7TM) region, and an intracellular C-terminus. These receptors belong to family C GPCRs, which presence of a large (∼560 amino acid) endogenous ligand-binding site ("Venus Fly Trap"; VFT) in the N-terminal domain. In contrast to the family C, in the family A and B GPCRs the agonist binding domain are located within the 7TM domain or within the extracellular loops that connect the strands to this region [Niswender and Conn, (2010) Ann. Rev. Pharmacol. Toxicol., 50:295-322].

Glutamate activates the mGluRs trough binding to the VFT domain of the receptor, termed the orthosteric binding domain. This activation induces a conformational changes of the receptor which results in the activation of the G-protein and intracellular signaling pathways.

The seven transmembrane domain of the metabotropic glutamate receptors is the binding site for the majority of characterized allosteric modulators of mGluRs that positively and negatively affect glutamate activity. Positive allosteric modulators (PAMs) do not activate the receptor directly in most systems but potentiate the response of the receptor to orthosteric agonists. Allosteric agonists bind to a site other than the orthosteric site and directly activate the receptor. Negative allosteric modulators (NAMs) antagonize the activity of agonists in a noncompetitive fashion by binding to a site other than the agonist, in this case glutamate, binding site [Niswender and Conn, (2010) Ann. Rev. Pharmacol. Toxicol., 50:295-322].

The binding domain overlap in a complex manner such that slight changes in the chemical structure can result in a "molecular switch" from NAM to PAM activity [Wood et al., (2011) Biochemistry, 50: 2403-2410]. Moreover, activation of the allosteric site may result in changes to the orthosteric site [Gregory et al., (2011) Neuropharmacology, 60:66-81].

The mGluRs family consists of eight members. Based on DNA sequence homology, pharmacology and nature of intracellular signalling cascades activated, the mGluRs have been divided into three groups: group I (mGluR₁ and mGluR₅), group II (mGluR₂ and mGluR₃) and group III (mGluR₄, mGluR₆, mGluR₇ and mGluR₈) [Pilc et al., (2013) Biol. Psychiatry Feb 28. doi:pii: S0006-3223(13)00092-9]. Group I mGlu receptors preferentially couple to Gq/G₁₁ proteins and activate phospholipase C, resulting in the hydrolysis of phosphotinositides and generation of inositol 1,4,5-triposphate (IP3) and diacyl-glycerol (DAG). This pathway leads to calcium mobilization and activation of protein kinase C (PKC). These receptors are primarily located post-synaptically and are functionally and physically linked to ionotropic glutamate receptors [Pilc et al., (2013) Biol. Psychiatry Feb 28. doi:pii: S0006-3223(13)00092-9]. Group II and III mGlu receptors are coupled mostly to Gi/o proteins and inhibit adenylyl cyclase. These receptors are predominantly presynaptically localized and are linked to neurotransmitters release, such as glutamate.

Glutamate plays an important role in many physiological functions, such as learning and memory as well as sensory perception, development of synaptic plasticity, motor control, respiration and regulation of cardiovascular function. Seven of the eight mGluRs subtypes are expressed within the central nervous system therefore they are attractive targets for a variety of psychiatric and neurological disorders [Gregory et al., (2013) Prog. Mol. Biol. Transl. Sci., 115: 61-121].

Among eight subtypes of metabotropic glutamate receptors, mGluR4 was found to be involved in pathogenesis of numerous disorders including anxiety [Stachowicz et al., (2006) Pharmacol. Rep., 58:820-826; Lavreysen and Dautzenberg, (2008) Curr. Med. Chem., 15: 671-684], depression (Cryan et al., (2003) Eur. J. Neurosci., 17:2409-2417; Kogo et al., (2004) Eur. J. Neurosci., 19:2727-2740; Klak et al., (2007) Amino Acids. 32:169-172], neuropathic pain [Lavreysen and Dautzenberg, (2008) Curr. Med. Chem., 15: 671-684], Parkinson's disease [Lavreysen and Dautzenberg, (2008) Curr. Med. Chem., 15: 671-684; Hopkins et al., (2009) Future Med. Chem., 1:501-513] and several types of epilepsies [Snead et al., (2000), J. Neurosci., 20:6218-6224; Chen et al., (2005), Neurosci. Lett., 375:192-197; Marino et al., (2005) Curr. Top. Med. Chem., 9:885-895].

mGlu4 receptors belongs to the group III mGluR subfamily and are expressed in most brain regions, particularly in the cerebellar cortex [Makoff et al., (1996) Mol. Brain Res. 37: 239-248], basal ganglia [Bradley et al., (1999) J. Comp. Neurol. 407:33-46], sensory relay of the thalamus and hippocampus [Corti et al., (2002) Neuroscience, 110:403-420].

Orthosteric agonists of mGluR4 are not selective and also activate the other mGluRs or ionotropic glutamate receptors. The prototypical highly selective agonist of group III mGluRs - L-AP4 has submicromolar to low micromolar potencies at mGluRs 4, 6 and 8 but submillimolar to millimolar potency at mGluR7 [Schoepp et al., (1999) Neuropharmacology, 38:1431-1476]. Many related compounds have provided additional group III mGluR agonists, but most have the similar selectivity profile as L-AP4 [Schoepp et al., (1999) Neuropharmacology, 38:1431-1476]. Recently have been synthesized a novel agonist (S)-3,4-DCPG with 100-fold selectivity for mGluR8 over mGluR4 [Thomas et al., (2001) Neuropharmacology, 40: 311-318; Zhai et al., (2002) Neuropharmacology, 43:223-230]. However several L-AP4 analogs (i.e. Z-cyclopentyl AP4) have been shown to have some selectivity for mGluR4 compared with other group III mGluRs [Kroona et al., (1991) J. Med. Chem., 34:1692-1699; Johansen et al., (1995) Mol. Pharmacol., 48:140-149; Ayala et al., (2008) Neuropharmacology, 54:804-814]. Besides the several available orthosteric antagonists have shown high selectivity for group III mGluRs (i.e. CPPG, MAP4) [Schoepp et al., (1999) Neuropharmacology, 38:1431-1476], but exhibit low potencies for antagonizing group III mGluRs, and in some cases have no effect even at high concentrations [Niswender et al., (2008) Mol. Pharmacol., 73:1213-1224]. The most potent group III antagonist is LY341495, followed CPPG [Niswender et al., (2008) Mol. Pharmacol., 73:1213-1224].

The first brain penetrating group III orthosteric agonist was ACPT-I [Acher et al., (1997) J. Med. Chem., 40:3119-3129]. This compound exert both antidepressant and anxiolytic-like effect in preclinical studies [Tatarczyńska et al., (2002) Pol. J. Pharmacol., 707-710; Pa 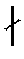 ucha et al., (2004) *Neuropharmacology*, 46:151-159]. However, ACPT-I is not subtype-selective, as it activates all members of group III receptors. Similar effect has a novel compound LSP1-2111, which activates group III mGlu receptors with some preference towards mGluR4 receptor [Beurrier et al., (2009) FASEB J., 23:804-814].

A new avenue for developing selective compounds acting at mGluRs (including group III mGluRs) is to identify molecules that act trough allosteric mechanisms, modulating the receptor by interacting with sites topographically distinct from the orthosteric binding site. Allosteric modulation of these class of receptors represents a novel approach to facilitate development of mGlu subtype-selective probes and therapeutics. In particular for central nervous system therapeutics, allosteric modulators have the potential to maintain the spatial and temporal aspects of endogenous neurotransmission. The past 15 years have seen the discovery of numerous subtype-selective allosteric modulators for the majority of the mGluR family members, including positive, negative, and neutral allosteric modulators, with a number of mGluR allosteric modulators now in clinical trials.

mGluR7 is primarily located on presynaptic terminals where it is thought to regulate neurotransmitter release [Ohishi et al., (1995) Neurosci. Lett. 202:85; Kinoshita et al (1998) J. Comp. Neurol. 393:332]. Mitsukawa reported the identification of AMN082 with selective mGluR7 agonist activity, and found that the compound is orally active and penetrates the blood-brain barrier [Mitsukawa et al. (2005) Proc. Natl. Acad. Sci. U.S.A. 102:18712].

Few years later the discovery of novel allosteric mGluR7 antagonists and the comprehensive in vitro pharmacological characterization of isoxazolopyridone derivatives as selective against mGluR1, mGluR2, mGluR3, mGluR4, mGluR5, mGluR6, and mGluR8 at 1000 nM were reported [Nakamura et al. (2007) J. Pharmacol. Exp. Ther. 323:147]. Recently, Nakamura group described SAR studies on isoxazolopyridone series with development of novel solid support synthesis of this scaffold. Subsequent chemical modification led to the identification of several potent derivatives with improved physicochemical properties [Nakamura et al. (2010) Bioorg. Med. Chem. Lett. 20:726].

Only one molecule was described as positive allosteric modulator of mGluR8 so far. The compound, AZ12216052 was found to potentiate the activity of glutamate at the human mGluR8 receptor expressed in GHEK cells. Authors showed that pharmacological modulation of mGluR8 has anxiolytic properties when given systemically in mouse models of anxiety. The specificity of AZ12216052 remains undefined. It was not effective on mGluR4 and initial screening with all other mGluRs was also negative [Duvoisin et al. (2010) Behav. Brain Res. 212:168].

The first mGluR4 positive allosteric modulators were SIB-1893, MPEP (Mathiesen et al. (2003) Brit. J. Pharmacol. 138:1026-1030) and (-)PHCCC (N-phenyl-7-(hydroxyimino)cyclopropan[*b*]chromen-*1a*-carboxamide). (-)PHCCC was derived from the mGluR1 negative allosteric modulator (-)CPCCOEt [Maj et al. (2003) Neuropharmacology 45:895-906]. (-)PHCCC has no direct agonist activity at mGluR4 but increases potency to glutamate at mGluR4. Furthermore this compound is a partial antagonist (30%) of mGluR1 [Niswender et al. (2008) Bioorg. Med. Chem. Lett., 18:5626-5630]. (-)-PHCCC has been shown to be efficacious in animal models of Parkinson's desease [Marino et al. (2003) Proc. Nat. Acad. Sci. USA, 100:13668-13673, Marino et al. (2005) Curr. Topics Med. Chem. 5:885-895, Valenti et al. (2005) J. Pharmacol. Exp. Ther., 313;1296-1304, Battaglia et al. (2006) J. Neurosci. 26:7222-7229] as well as in animal model of anxiety [Stachowicz et al. (2004) Eur. J. Pharmacol. 498:153-156]

More recently a functional high-throughput screening (HTS) initiated at Vanderbilt University led to identification of a number of hits with mGluR4 activity [Niswander et al. (2008) Mol. Pharmacol. 74:1345-1358, Niswander et al. (2009) Curr. Topics Med. Chem. 9:949-963]. This discovery was a starting point for a research program in which extensive SAR studies were performed revealing a numerous group of new mGluR4 allosteric modulators of different structure based on: *cis*-2-(3,5-dichlorophenylcarbamoyl) cyclohexanecarboxylic acid (VU0155041) [Niswender et al. (2009) Bioorg. Med. Chem. Lett. 19:4967-4970], simple biheteroarylamides [Engers et al. (2009) J. Med. Chem. 52:4115-4118], benzylidene hydrazinyl-3-methylquinazoline and *bis*-2,3-dihydro-quinazolin-4(1H)-one scaffold [Williams et al. (2009) Bioorg. Med. Chem. Lett. 19:949-963], substituted 1,1,3,3-tetraoxidobenzod]-1,3-dithia-2-azoles [international patent publication WO2010/088406A1], N-(4-acetamido)-phenylpicolinamides series [Niswender et al. (2011) J. Med. Chem. 54:1106-1110], substituted benzoimidazolesulfonamides and indolesulfonamides [international patent publication WO2011/011722A1], substituted heteroaryl or arylsulfonyl-1H-pyrazol-4-yl heteroarylamides [international patent publication WO2011/050305A1], dioxoimidazole core substituted with phenylheteroarylamides [WO2011/029104A1] and other pyrazolopyridine, pyrazolopyrazine, pyrazolopyrimidine, pyrazolothiophene, pyrazolothiazole derivatives [international patent publication WO2011/00607A1].

Independently Addex research group has discovered and developed a novel series of mGluR4 PAMs and demonstrated its efficacy in different models of anxiety and Parkinson's disease. International patent publications WO2009/010454A2 and WO2009/010455A2 revealed series of O-aryl substituted heteroaryloamide derivatives and heteroaryl substituted tiazoles and thiadiazoles as positive allosteric mGluR4 modulators. Further SAR studies led to enhancement of mGluR4 PAM activity by introducing pyrazole moiety into thiazole or thiadiazole scaffold [patent publications: US2010/0144756A1, WO2010/079238A1, WO2010/079239A1, WO2011/010222A1, WO2012/009001A2].

A series of tricyclic thiazolopyrazole derivatives was identified in Lundbeck Research as mGluR4 PAMs through medicinal chemistry designed aided by molecular modelling. The detailed SAR of this series was explored resulting discovering few potent and orally bioavailable compounds with excellent brain penetration and good physiochemical properties [Doller et al. (2011), J. Med. Chem. 54:5070-5081].

3,5-diarylosubstituted 1,2,4-oxadiazoles were disclosed in documents WO01/12627 and WO02/068417 as inhibitors of mGlu5 receptors belonging to group I of metabotropic receptors. These post-synaptically located receptors activate Gq/G₁₁ class of G-proteins and pathway leads to calcium mobilization and activation of protein kinase C (PKC). Revealed structures differs from compounds of present application by lacking a structural element of M and X (Formula 1). This additional structural fragment is responsible and essential for unexpected property which is the ability of allosteric modulation of mGluR4 from group III. Presynaptically localized group III mGlu receptors are coupled to Gi/o proteins and inhibit adenylyl cyclise resulting neurotransmitters release, such as glutamate.

Despite recent advances in mGluR4 PAMs identification, there is still necessity for seeking new molecules that can efficiently modulate mGluR4 activity with acceptable bioavailability. The present inventors have discovered a novel series of compounds bearing 1,2,4-oxadiazole moiety which showed activity on mGluR4 receptor in *in vitro* tests. Also activity in behavioral models was demonstrated in hyperthermia test on mouse where anxiolytic effect was observed.

The present invention relates to compounds useful as allosteric modulators of mGluR4 activity in mammal, including humans, methods of their preparation and they are useful for treating, preventing, controlling or reducing a variety neurological and psychiatric disorders such as: anxiety disorders, posttraumatic stress disorders, addictive disorders, Parkinson's disease and other disorders involving akinesia or bradykinesia, schizophrenia and other disorders associated with glutamate dysfunction.

### Summary of invention

Present description discloses different 1,2,4-oxadiazole derivatives of general formula **1** or pharmaceutically acceptable salts, hydrates or solvates thereof, 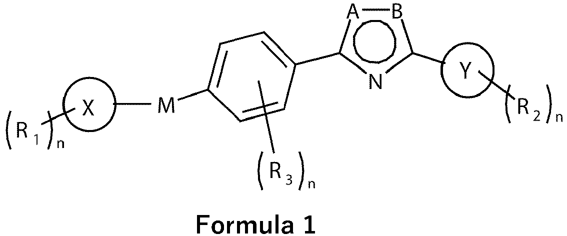 wherein
A, B are each independently selected from N and O forming 5-membered 1,2,4-oxadiazole ring;
**X, Y** represents independently phenyl or 5 or 6-membered heteroaryl ring containing one to three heteroatoms selected independently from N, O or S;
**R₁, R₂, R₃** represents independently hydrogen, halogen, -NO₂, -CN, -OH, - SH, -NH₂ or an optionally substituted (C₁-C₆)alkyl (C₁-C₆)haloalkyl-OR₄, - SR₄,
wherein **R₄** represents independently hydrogen or, (C₁-C₆)alkyl;
**M** represents: -NR₆-,-COO-, -OCO-, -NR₆C(=O)-, -C(=O)NR₆-, wherein **R₆** represents independently hydrogen or (C₁-C₆)alkyl;
**n** is an integer ranging from 1 to 3 that are useful as positive allosteric modulator ligands of metabotropic glutamate receptors of group III subtype 4 (mGluR4) in the treatment of the central nervous disorders modulated by mGluR4 receptors

Some of disclosed of compounds are of particular interest. Therefore, present invention is directed to specific 1,2,4-oxadiazole derivatives of general formula 1 selected from
N-{4-[5-(4-methoxyphenyl)-1,2,4-oxadiazol-3-yl]phenyl}pyridine-4-carboxamide,
N-{4-[5-(2-chlorophenyl)-1,2,4-oxadiazol-3-yl]-3-methoxyphenyl}pyridine-2-carboxamide;
N-{4-[5-(2-chloro-4-fluorophenyl)-1,2,4-oxadiazol-3-yl]-3-methoxyphenyl} pyridine-2-carboxamide;
N-{3-chloro-4-[5-(2-chloro-4-fluorophenyl)-1,2,4-oxadiazol-3-yl]phenyl}pyridine-2-carboxamide;
N-{4-[5-(2-chloro-4-fluorophenyl)-1,2,4-oxadiazol-3-yl]-3-fluorophenyl} pyridine-2-carboxamide;
N-{4-[5-(2-methoxyphenyl)-1,2,4-oxadiazol-3-yl]-3-fluorophenyl}pyridine-2-carboxamide;
N-[4-(5-phenyl-1,2,4-oxadiazol-3-yl)-3-trifluoromethyl)phenyl]pyridine-2-carboxamide;
N-{4-[5-(2-chlorophenyl)-1,2,4-oxadiazol-3-yl]-3-(trifluoromethyl)phenyl} pyridine-2-carboxamide;
N-[3-methyl-4-(5-phenyl-1,2,4-oxadiazol-3-yl)phenyl]pyridine-2-carboxamide;
N-{4-[5-(2-chlorophenyl)-1,2,4-oxadiazol-3-yl]-3-methylphenyl}pyridine-2-carboxamide;
N-{4-[5-(2-chlorophenyl)-1,2,4-oxadiazol-3-yl]-3-methoxyphenyl}-6-fluoropyridine-2-carboxamide;
5-chloro-N-{4-[5-(2-chlorophenyl)-1,2,4-oxadiazol-3-yl]-3-methoxyphenyl}-3-fluoropyridine-2-carboxamide;
6-chloro-N-{4-[5-(2-chlorophenyl)-1,2,4-oxadiazol-3-yl]-3-methoxyphenyl}pyridine-2-carboxamide
N-{4-[5-(2-chlorophenyl)-1,2,4-oxadiazol-3-yl]-3-methoxyphenyl} pyrimidine-4-carboxamide
N-{4-[5-(2-chlorophenyl)-1,2,4-oxadiazol-3-yl]-3-methoxyphenyl}thiophene-2-carboxamide;
6-fluoro-N-{4-[5-(2-fluorophenyl)-1,2,4-oxadiazol-3-yl]-3-methoxyphenyl} pyridine-2-carboxamide;
3,6-dichloro-N-{4-[5-(2-fluorophenyl)-1,2,4-oxadiazol-3-yl]-3-methoxy phenyl}pyridine-2-carboxamide;
N-{3-chloro-4-[5-(2-chlorophenyl)-1,2,4-oxadiazol-3-yl]phenyl}-6-fluoropyridine-2-carboxamide;
N-(2-chlorophenyl)-4-(5-phenyl-1,2,4-oxadiazol-3-yl)benzene-1-sulfonamide;
N-(2,4-difluorophenyl)-4-[5-(pyridin-2-yl)-1,2,4-oxadiazol-3-yl]benzene-1-sulfonamide;
N-[4-(3-phenyl-1,2,4-oxadiazol-5-yl)phenyl]benzamide;
3-chloro-N-[4-(3-phenyl-1,2,4-oxadiazol-5-yl)phenyl]benzamide;
4-methoxy-N-[4-(3-phenyl-1,2,4-oxadiazol-5-yl)phenyl]benzamide;
4-nitro-N-[4-(3-phenyl-1,2,4-oxadiazol-5-yl)phenyl]benzamide;
1-methyl-N-[4-(3-phenyl-1,2,4-oxadiazol-5-yl)phenyl]-1H-pyrrole-2-carboxamide;
for use as positive allosteric modulator ligands of metabotropic glutamate receptors of group III subtype 4 (mGluR4) in the treatment of the central nervous disorders modulated by mGluR4 receptors.

Preferred central nervous disorders modulated by mGlu4 receptor are Parkinson's disease, parkinsonism and other disorders involving akinesia or bradykinesia; dystonia; Huntington's disease and other disorders involving involuntary movements and dyskinesias; schizophrenia and other psychotic disorders; pain; anxiety disorders; mood disorders (including depression, mania, bipolar disorders); addictive disorders; epilepsy; posttraumatic stress disorders; Alzheimer's disease; dementia and other form of neurodegeneration..

Below are listed definitions of terms that were used in the detail description to describe presented invention and disclosure.

As used herein the term "halo" or "halogen" includes fluoro, chloro, bromo and iodo.

The term "alkyl" means linear or branched carbon chain having single carbon-carbon bonds. Thus (C₁-C₆)alkyl means linear or branched chain having from one to six carbon atoms and includes methyl, ethyl, *n*-propyl, isopropyl, *n*-butyl, isobutyl, *sec*-butyl, *tert*-butyl, pentyl, hexyl or the like.

Likewise the term "alkenyl" refers to group such as ethenyl, 1-propenyl, allyl, isopropenyl, 1-butenyl, 3-butenyl, 4-pentenyl or the like.

Similarly "alkynyl" includes groups such as ethynyl, propynyl, butynyl, pentynyl or the like.

The term "haloalkyl" refers to defined alkyl group substituted with one or more halogens and may include but is not limited to groups such as fluoromethyl, difluoromethyl, trifluoromethyl, fluoroethyl, difluoroethyl.

The term "alkylene" means linear or branched difunctional carbon chain having single carbon-carbon bonds. Thus (C₁-C₆)alkylene means linear or branched difunctional chain having one to six carbon atoms and includes methylene, ethylene, *n*-propylene, isopropylene, *n*-butylene, isobutylene, *sec*-butylene, *tert*-butylene, pentylene, hexylene or the like.

The term "cycloalkyl" refers to carbocycle containing no heteroatoms, including mono-, bi- or tricyclic saturated carbocyclic ring as well as fused rings containing one ring that is partially or fully unsaturated. Examples of (C₃-C₁₀)cycloalkyl include cyclopropyl, cyclobutyl, cyclopentyl, cyclohexyl, cycloheptyl, cyclooctyl, norbornyl, adamantyl, decahydronaphtyl, indanyl, 1,2,3,4-tetrahydronaphtyl or the like.

The term "aryl" means an optionally substituted mono- or bicyclic hydrocarbon ring containing from six to ten carbon atoms with at least one unsaturated aromatic ring and includes groups such as phenyl, 1-naphtyl, 2-naphtyl, 1,2,3,4-tetrahydronaphtyl, indyl, indenyl and the like.

The term "arylalkyl" means the substituent that is attached via the alkyl moiety and refers to (C₁-C₃)alkyl connected to (C₆-C₁₀)aryl including groups such as benzyl, 1-phenylethyl, 2-phenylethyl, 1-phenylpropyl, 2-phenylpropyl, 3-phenylpropyl, 1-naphtylmethyl, 2-naphtylmethyl and the like.

The term "heteroaryl" refers to optionally substituted five to twelve membered mono- or bicyclic unsaturated aromatic ring having at least one heteroatom selected independently from N, O or S. Examples of heteroaryl include groups such as furyl, benzofuryl, thienyl, benzothiophenyl, pyrrolyl, imidazolyl, benzoimidazolyl, triazolyl, tertrazolyl, triazinyl, thiazolyl, benzothiazolyl, isothiazolyl, thiadiazolyl, oxadiazolyl, oxazolyl, benzooxazolyl, isooxazolyl, pyrazolyl, oxazolonyl, thiazolonyl, indolyl, isoindolyl, pyridyl, pyrazinyl, pyrimidinyl, pyridazinyl, pyridonyl, quinolyl, phtalazinyl, purinyl, haphthyridinyl, quinoxalinyl, quinazolyl, imidazopyridinyl, oxazolopyridinyl, thiazolopyridinyl, imidazolpyridazinyl, oxazolopyridazinyl, thiazolopyridazinyl, cynnolyl, pteridinyl, furazanyl, benzotriazolyl, pyrazolopyridinyl or the like.

The term "heteroarylalkyl" means the substituent that is attached via the alkyl moiety and refers to (C₁-C₃)alkyl connected to (C₅-C₁₀)heteroaryl including groups such as for example 2-furylmethyl, 3-furylmethyl, 2-thienylmethyl, 3-thienylmethyl, 1-imidazolylmethyl, 2-imidazolylmethyl, 2-thienylmethyl, 3-thienylmethyl, 1-imidazolylmethyl, 2-imidazolylmethyl, 3-imidazolylmethyl, 2-oxazolylmethyl, 3-oxazolylmethyl, 2-thiazolylmethyl, 2-pyridylmethyl, 3-pyridydlmethyl, 4-pyridynylmethyl, 1-quinolylmethyl or the like.

The term "optionally substituted" means that described groups may be further substituted with one or more substituents like (C₁-C₆)alkyl, -O-(C₁-C₆)alkyl, halogen, trifluoromethyl, hydroxyl, mercaptyl, cyano, nitro, amino, amido, carboxyl, ester, sulfonyl, sulfonamide, aryl and heteroaryl. When term "optionally substituted" refers to heteroaryl group, substituent may be bonded to a ring carbon atom or on a ring heteroatom (*i.e.* nitrogen, sulfur).

The term "pharmaceutically acceptable salts" means salts prepared from pharmaceutically acceptable non-toxic base and acids including organic or inorganic base and acids. When the compound of the present invention is basic, salts may be prepared from non-toxic organic or inorganic acids such as acetic, benzoic, methanesulfonic, benzenesulfonic, p-toulenesulfonic, citric, fumaric, glutamic, gluconic, glutamic, lactic, maleic, malic, mandelic, succinic, tartaric, hydrobromic, hydrochloric, phosphoric, sulfuric or the like. The term "solvates" refers to a complex formed by the solute which is any compound of Formula I and a solvent. Solvates can have variable stechiometry. Preferably solvates relates to hydrates with water as a solvent that do not interfere with the biological activity of the solute.

### METHODS OF SYNTHESIS

The compounds of general Formula 1 described herein can be synthesized by methods known in the art of organic synthesis according to the processes presented in Schemes I-IV.

Obtained compounds were isolated from the reaction mixtures and purified according to standard procedures and techniques known to a person skilled in the art such as extraction, chromatography and crystallization.

The compounds of the invention can be prepared according to Scheme I, starting from substituted p-nitrobenzylonitriles which in reaction with hydroxylamine hydrochloride in the presence of base such as sodium hydroxide or triethylamine are converted to corresponding benzamide oximes. The next step was a coupling reaction with acid chlorides in the presence of carbonate (for example: sodium bicarbonate, sodium carbonate, potassium carbonate, cesium carbonate) resulting in the formation of 1,2,4-oxadiazole ring. This reaction can be carried out in the boiling solvent, preferably toluene, benzene, acetonitrile, for several hours (5 - 15h) under normal pressure or in the pressure vessel at 150-170°C using microwave irradiation in much shorter period of time (10 - 15 minutes). Subsequently in the third step, the nitro group is reduced with application of one of the reducing agents (for example: iron in the presence of acetic acid, tin chloride in the presence of hydrochloric acid, hydrogen in the presence of catalytic amount of palladium on charcoal). In the last step obtained anilines were substituted with various acid chlorides or carboxylic acids to give final amides or with boronic acids to obtain biaryl amines. Reaction with acid chlorides can be proceeded in pyridine at room temperature or in a solvent (for example: dichloromethane, tetrahydrofuran) in the presence of other organic or inorganic amine (for example: triethylamine). In other way anilines can be reacted with various carboxylic acids with appropriate coupling agent (for example: BOP in the presence of triethylamine). Biarylamines can be synthesized in coupling reaction of arylamines with various boronic acids in presence of catalyst such as copper diacetate.

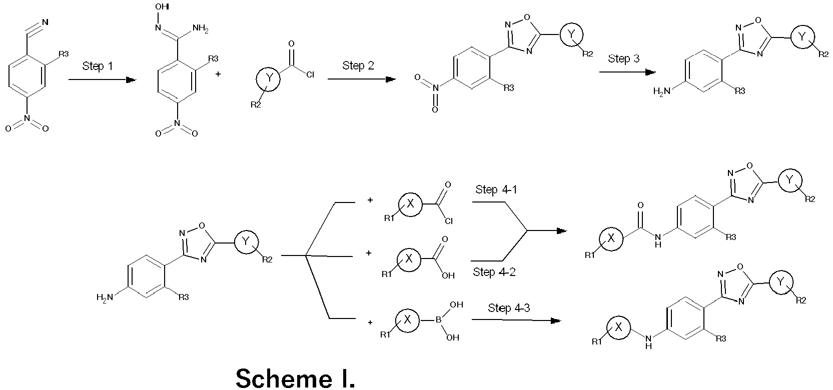

### Reagents and conditions:

Step 1
   (a) NH₂OH·HCl, NaOH_{aq}, EtOH, reflux, 1-5h,
   (b) NH₂OH·HCl, TEA, EtOH, rt, 20 min, reflux, 1-5h;
Step 2
   (a) acid chloride, toluene, K₂CO₃, MW 170°C, 10 min,
   (b) acid chloride, toluene, K₂CO₃, rt, 1h than reflux 5-15h;
Step 3
   (a) Fe, CH₃COOH, EtOH, water, 60° C, 1-2h
   (b) SnCl₂, 5N HCl, EtOH, reflux, 2h;
Step 4-1
   (a) acid chloride, py, rt, overnight,
   (b) acid chloride, TEA, THF, rt, overnight,
Step 4-2 carboxylic acid, BOP, TEA, DCM, rt, 1-3 days;
Step 4-3 boronic acid, DIPEA, Cu(OAc)₂, MeCN, rt, 1-3 days.

The invention compounds of the Formula 1 may be prepared according to the synthetic route presented in Scheme II from substituted p-aminobenzonitriles. In the first step formation of amide was carried out in reaction of starting p-aminobenzonitriles with acid chlorides in the presence of base (for example: triethylamine). The next step was to convert a nitrile group into amide oxime in reaction with hydroxylamine hydrochloride in the presence of base (for example: aqueous sodium hydroxide solution). Prepared benzamide oximes were coupled with various acid chlorides resulting the formation of 1,2,4-oxadizaole ring in the final product.

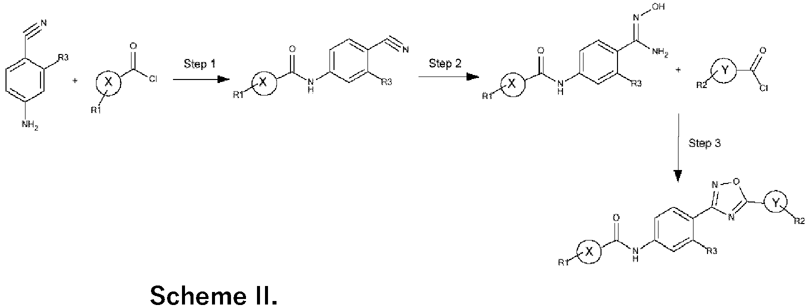

### Reagents and conditions:

Step 1 acid chloride, TEA, THF, rt, overnight;
Step 2 NH₂OH·HCl, NaOH_{aq}, EtOH, reflux, 1-5h;
Step 3 acid chloride, toluene, K₂CO₃, rt, 1h than reflux 5-15 h.

The invention compounds of the Formula 1 may be prepared according to the synthetic route presented in Scheme 3 starting from p-cyanobenzenesulfonyl chlorides. In the first step formation of sulfonamide was carried out in reaction of starting compound with various anilines in the presence of base as a solvent (for example: pyridine). The next step was to convert a nitrile group into amide oxime in reaction with hydroxylamine hydrochloride in the presence of base (for example: aqueous sodium hydroxide solution). Prepared benzamide oximes were coupled with various acid chlorides in the presence of carbonate (for example: sodium bicarbonate, sodium carbonate, potassium carbonate, cesium carbonate) resulting the formation of 1,2,4-oxadizaole ring in the final product. Coupling reaction could be performed in solvent (for example: toluene, benzene, acetonitrile) in reflux under normal pressure for several hours (5 - 15 hours) or in the pressure vessel in higher temperatures (150-170°C) using microwave irradiation in much shorter period of time (10 - 15 minutes).

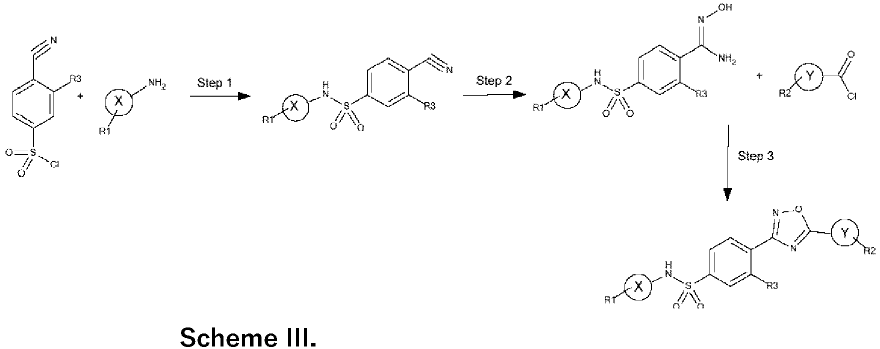

### Reagents and conditions:

Step 1 aniline, py, rt, overnight;
Step 2 NH₂OH·HCl, NaOH_{aq}, EtOH, reflux, 1-5h;
Step 3
   (a) acid chloride, toluene, K₂CO₃, MW 170°C, 10 min,
   (b) acid chloride, toluene, K₂CO₃, rt, 1h than reflux 6h.

The invention the compounds of the invention can be prepared according to Scheme 4 starting from substituted benzylonitriles which in reaction with hydroxylamine hydrochloride in the presence of base (for example: sodium hydroxide, triethylamine) are converted to corresponding benzamide oximes. The following step was a coupling reaction with substituted p-nitrobenzoyl chlorides in the presence of carbonate (for example: sodium bicarbonate, sodium carbonate, potassium carbonate, cesium carbonate) resulting formation of 1,2,4-oxadiazole ring. Reaction can be carried in boiling solvent such as toluene, benzene, acetonitrile under normal pressure for several hours (5 - 15h) or in the pressure vessel in higher temperatures (150-170°C) using microwave irradiation in much shorter period of time (10 - 15 minutes). In the third step nitro group can be reduced with application of one of the reducing agents (for example: iron in the presence of acetic acid, tin chloride in the presence of hydrochloric acid, hydrogen in the presence of catalytic amount of palladium on charcoal). Finally in the last step obtained anilines were substituted with various acid chlorides to give amides or with boronic acids to obtain biaryl amines. Reaction with acid chlorides can be proceeded in pyridine in room temperature or in a solvent (for example: dichloromethane, tetrahydrofuran) in the presence of other organic or inorganic amine (for example: triethylamine). Biarylamines can be synthesized in coupling reaction of arylamines with various boronic acids in the presence of catalyst such as copper diacetate.

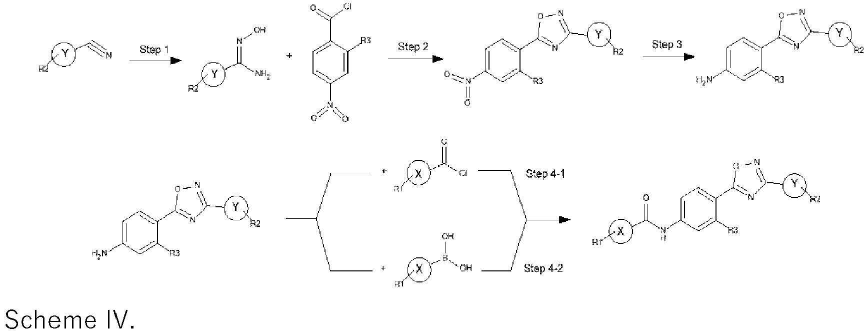

### Reagents and conditions:

Step 1 NH₂OH·HCl, NaOHaq, EtOH, reflux, 1-5h;
Step 2 acid chloride, toluene, K₂CO₃, MW 170°C, 10 min;
Step 3
   1. Fe, CH₃COOH, EtOH, water, 60° C, 1-2h;
   2. Raney Ni, NH₂-NH₂ aq, MeOH/THF, 60° C, 30 min;
Step 4-1 acid chloride, py, rt, overnight;
Step 4-2 boronic acid, DIPEA, Cu(OAc)₂, MeCN, rt, 1-3 days.

All abbreviations that were used in this section and in the experimental part are listed in the Table 1 below.

### Table 1. List of abbreviations

**Table 1**

| | |
|---|---|
| AcOEt | ethyl acetate |
| anh | anhydrous |
| BOP | 1H-benzotriazol-1-yloxytris(dimethylamino) phosphonium hexafluorophosphate |
| CDCl₃ | *d*-chloroform |
| CHCl₃ | chloroform |
| CH₃COOH | acetic acid |
| Cu(OAc)₂ | copper diacetate |
| d | doublet |
| DCM | dichloromethane |
| DIPEA | diisopropylethylamine |
| DMSO-d₆ | *d₆*-dimethylsulfoxide |
| eq | equivalent |
| ESI+ | positive electrospray ionisation |
| EtOH | ethanol |
| HCl | hydrochloric acid |
| NH₂-NH₂ | hydrazine |
| i-PrOH | isopropanol |
| m | multiplet |
| MeCN | acetonitrile |
| MeOH | methanol |
| MW | microwave irradiation |
| NaCl | sodium chloride |
| NaOH_{aq} | aqueous sodium hydroxide |
| NH_{3aq} | aqueous ammonium |
| NH₂OH·HCl | hydroxylamine hydrochloride |
| NMR | nuclear magnetic resonance |
| K₂CO₃ | potassium carbonate |
| py | pyridine |
| rt | room temperature |
| RT | retention time |
| s | singlet |
| s br. | singlet broad |
| SnCl₂ | tin chloride |
| t | tryplet |
| TEA | triethylamine |
| THF | tetrahydrofurane |
| TLC | thin layer chromatography |
| UPLC/MS | ultra performance liquid chromatography / mass spectroscopy |

### Description of embodiments

### Table 2. Exemplary compounds of present disclosure (examples 6, 17, 19, 22, 26-34, 38-42, 44, 46-50 and 54 belong to the invention)

**Table 2**

| Example | Structure | Synthetic procedures | LC/MS (M+H) (Method) |
|---|---|---|---|
| 1 | 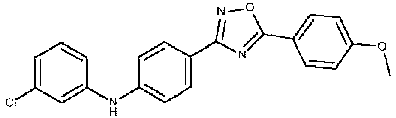 | Scheme I Steps 1a, 2a, 3a, 4-3 | 378.1 (B) |
| 2 | 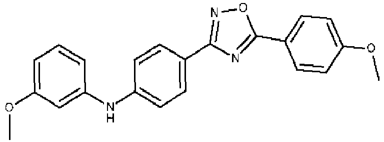 | Scheme I Steps 1a, 2a, 3a, 4-3 | 374.2 (B) |
| 3 | 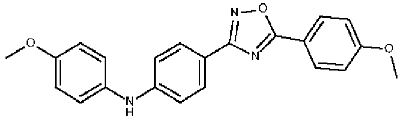 | Scheme I Steps 1a, 2a, 3a, 4-3 | 374.2 (B) |
| 4 | 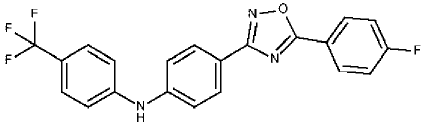 | Scheme I Steps 1a, 2a, 3a, 4-3 | 400.0 (B) |
| 5 | 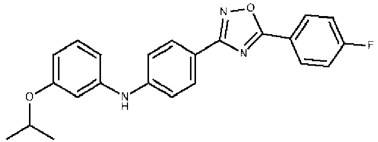 | Scheme I Steps 1a, 2a, 3a, 4-3 | 390.1 (B) |
| 6 | 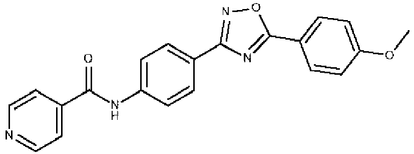 | Scheme I Steps 1a, 2a, 3a, 4-1a | 373.4 (B) |
| 7 | 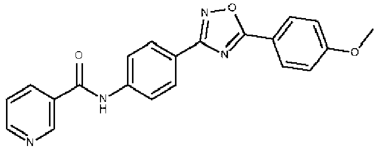 | Scheme I Steps 1a, 2a, 3a, 4-1a | 372.8 (B) |
| 8 | 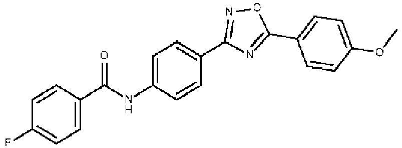 | Scheme I Steps 1a, 2a, 3a, 4-1a | 390.1 (B) |
| 9 | 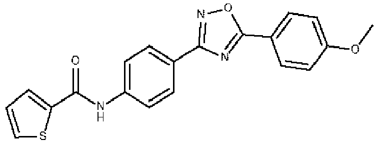 | Scheme I Steps 1a, 2a, 3a, 4-1a | 378.1 (B) |
| 10 | 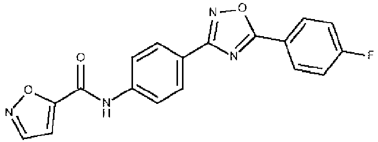 | Scheme I Steps 1a, 2a, 3a, 4-1a | 351.1 (B) |
| 11 | 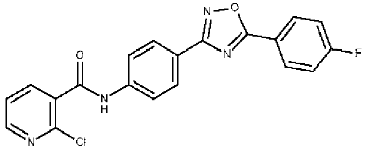 | Scheme I Steps 1a, 2a, 3a, 4-1a | 395.0 (B) |
| 12 | 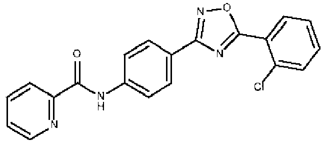 | Scheme I Steps 1a, 2a, 3b, 4-1a | 377.1 (A) |
| 13 | 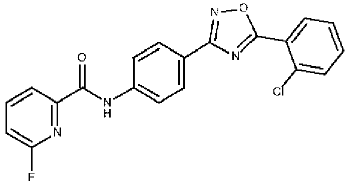 | Scheme I Steps 1a, 2a, 3b, 4-2 | 394.8 (A) |
| 14 | 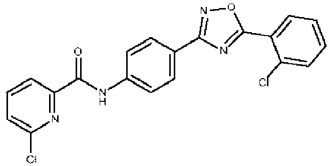 | Scheme I Steps 1a, 2a, 3b, 4-2 | 410.7 (A) |
| 15 | 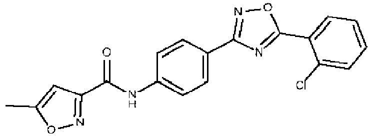 | Scheme I Steps 1a, 2a, 3b, 4-1a | 381.1 (A) |
| 16 | 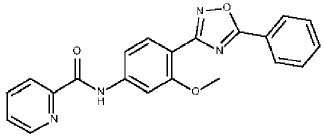 | Scheme I Steps 1a, 2b, 3b, 4-1a | 374.4 (A) |
| 17 | 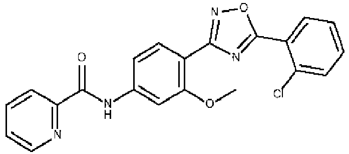 | Scheme I Steps 1a, 2b, 3b, 4-1a | 406.3 (A) |
| 18 | 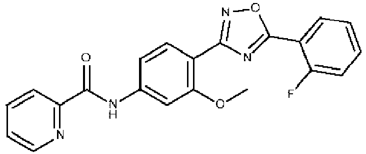 | Scheme I Steps 1a, 2b, 3b, 4-1a | 390.3 (A) |
| 19 | 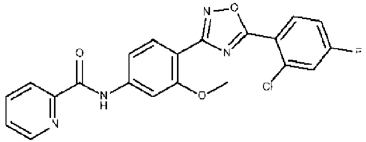 | Scheme I Steps 1a, 2b, 3b, 4-1a | 424.2 (A) |
| 20 | 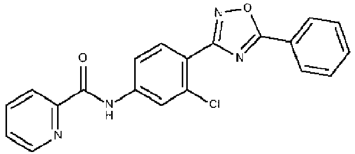 | Scheme I Steps 1a, 2b, 3b, 4-1b | 376.4 (A) |
| 21 | 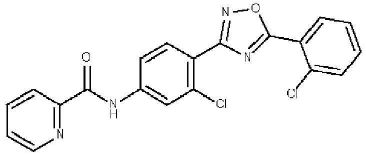 | Scheme II Steps 1, 2, 3 | 410.8 (A) |
| 22 | 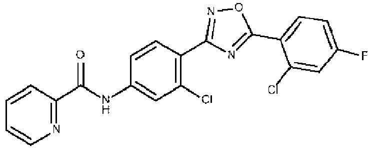 | Scheme II Steps 1, 2, 3 | 428.7 (A) |
| 23 | 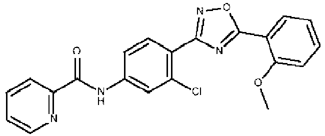 | Scheme II Steps 1, 2, 3 | 406.5 (A) |
| 24 | 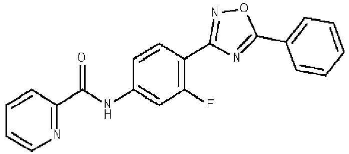 | Scheme II Steps 1, 2, 3 | 361.3 (A) |
| 25 | 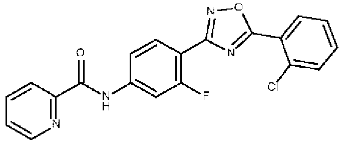 | Scheme II Steps 1, 2, 3 | 395.0 (B) |
| 26 | 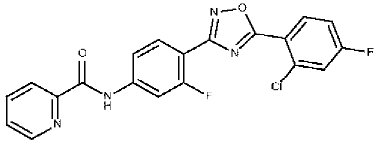 | Scheme II Steps 1, 2, 3 | 413.1 (B) |
| 27 | 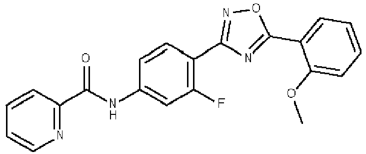 | Scheme II Steps 1, 2, 3 | 390.8 (B) |
| 28 | 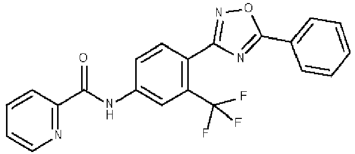 | Scheme I Steps 1b, 2b, 3b, 4-1b | 411.6 (B) |
| 29 | 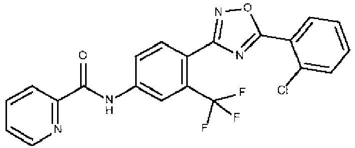 | Scheme I Steps 1b, 2b, 3b, 4-1b | 445.0 (B) |
| 30 | 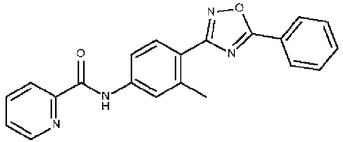 | Scheme I Steps 1a, 2b, 3b, 4-1b | 357.0 (B) |
| 31 | 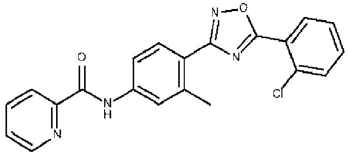 | Scheme I Steps 1a, 2b, 3b, 4-1a | 390.5 (A) |
| 32 | 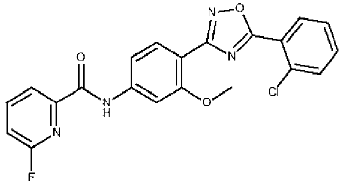 | Scheme I Steps 1a, 2b, 3b, 4-2 | 424.6 (A) |
| 33 | 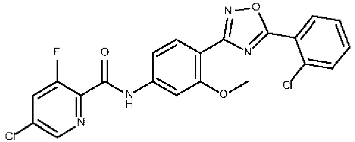 | Scheme I Steps 1a, 2b, 3b, 4-2 | 458.7 (A) |
| 34 | 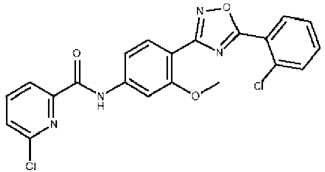 | Scheme I Steps 1a, 2b, 3b, 4-2 | 440.8 (A) |
| 35 | 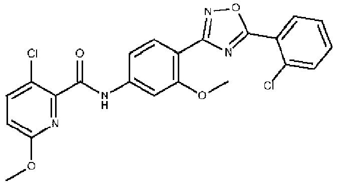 | Scheme I Steps 1a, 2b, 3b, 4-1a | 470.4 (A) |
| 36 | 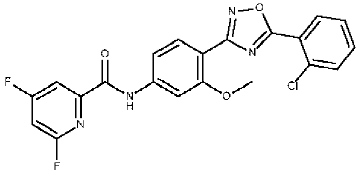 | Scheme I Steps 1a, 2b, 3b, 4-2 | 442.4 (A) |
| 37 | 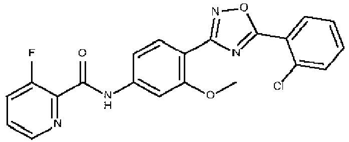 | Scheme I Steps 1a, 2b, 3b, 4-2 | 424.5 (A) |
| 38 | 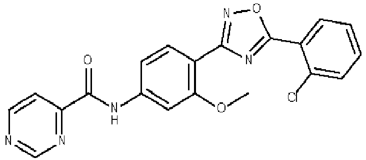 | Scheme I Steps 1a, 2b, 3b, 4-2 | 408.1(B) |
| 39 | 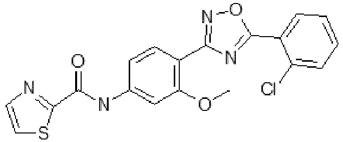 | Scheme I Steps 1a, 2b, 3b, 4-2 | 413.0 (A) |
| 40 | 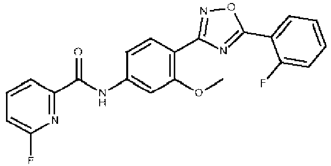 | Scheme I Steps 1a, 2b, 3b, 4-2 | 408.5 (A) |
| 41 | 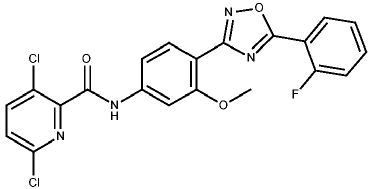 | Scheme I Steps 1a, 2b, 3b, 4-1a | 458.7 (A) |
| 42 | 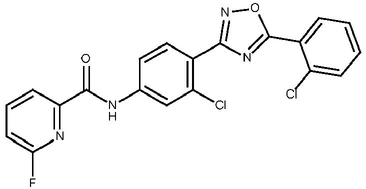 | Scheme I Steps 1a, 2b, 3b, 4-2 | 428.6 (A) |
| 43 | 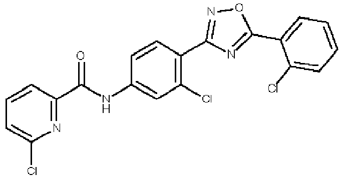 | Scheme I Steps 1a, 2b, 3b, 4-2 | 445.0 (A) |
| 44 | 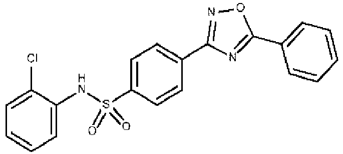 | Scheme III Steps 1, 2, 3a | 411.3 (A) |
| 45 | 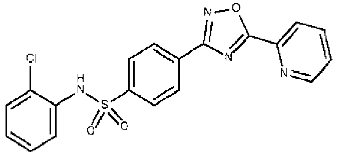 | Scheme III Steps 1, 2, 3a | 412.3 (A) |
| 46 | 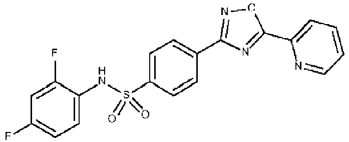 | Scheme III Steps 1, 2, 3b | 414.2 (B) |
| 47 | 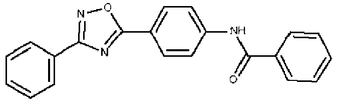 | Scheme IV Steps 1, 2, 3a, 4-1 | 342.1 (B) |
| 48 | 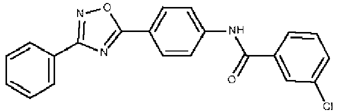 | Scheme IV Steps 1, 2, 3a, 4-1 | 375.9 (B) |
| 49 | 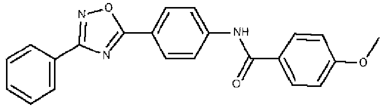 | Scheme IV Steps 1, 2, 3a, 4-1 | 372.7 (B) |
| 50 | 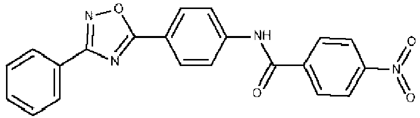 | Scheme IV Steps 1, 2, 3a, 4-1 | 387.1 (B) |
| 51 | 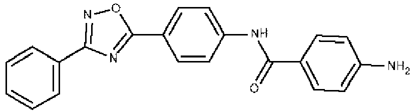 | Scheme IV Steps 1, 2, 3a, 4-1 than reduction as in step 3a | 357.1 (B) |
| 52 | 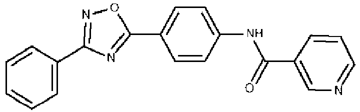 | Scheme IV Steps 1, 2, 3a, 4-1 | 342.9 (B) |
| 53 | 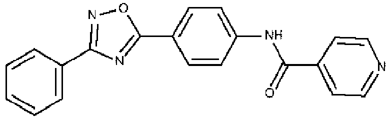 | Scheme IV Steps 1, 2, 3a, 4-1 | 343.4 (B) |
| 54 | 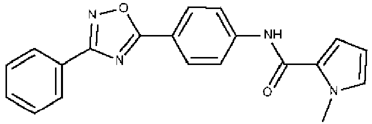 | Scheme IV Steps 1, 2, 3a, 4-1 | 345.0 (B) |
| 55 | 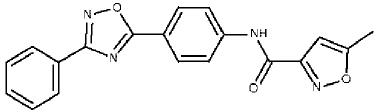 | Scheme IV Steps 1, 2, 3a, 4-1 | 347.0 (B) |
| 56 | 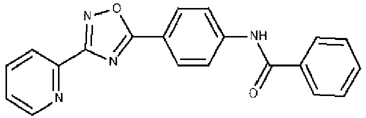 | Scheme IV Steps 1, 2, 3b, 4-1 | 343.2 (B) |
| 57 | 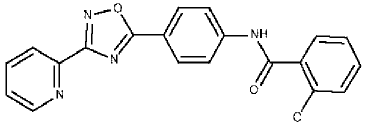 | Scheme IV Steps 1, 2, 3b, 4-1 | 376.8 (B) |
| 58 | 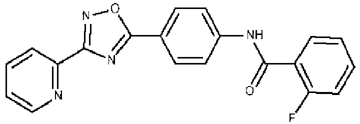 | Scheme IV Steps 1, 2, 3b, 4-1 | 360.8 (B) |
| 59 | 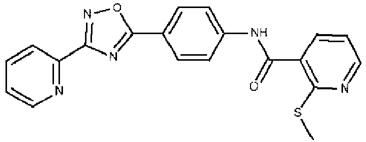 | Scheme IV Steps 1, 2, 3b, 4-1 | 389.8 (B) |
| 60 | 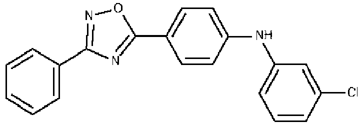 | Scheme IV Steps 1, 2, 3a, 4-2 | 348.1 (B) |
| 61 | 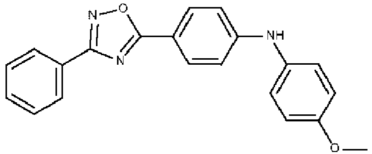 | Scheme IV Steps 1, 2, 3a, 4-1 | 344.1 (B) |
| 62 | 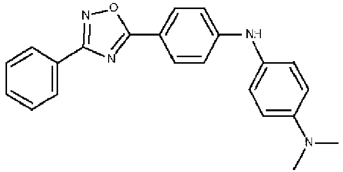 | Scheme IV Steps 1, 2, 3a, 4-1 | 357.2 (B) |

### EXPERIMENTAL DATA

All starting materials were purchased from commercial suppliers and were used without purification.

Reaction progress was monitored by TLC on Merck Silica Gel 60 F₂₅₄ on aluminium plates or Merck Aluminum oxide 60 F₂₅₄, neutral on aluminium plates and visualized with UV light (254 nm).

Column chromatography was performed on Merck Silica Gel 60 (0.063-0.200 mm; 70-230 mesh ASTM) for column chromatography or on Merck Aluminum oxide 90 active neutral (0.063-0.200 mm; 70-230 mesh ASTM).

¹H NMR spectra were measured at 300 MHz and ¹³C NMR spectra at 75 MHz on a Varian Mercury-VX (300 MHz) spectrophotometer in CDCl₃ or d₆-DMSO solutions with TMS as an internal standard. The spectral data of new compounds refer to their free bases. Chemical shifts were expressed in δ (ppm). For compounds containing fluorine atoms couplings from C-F were observed on ¹³C carbon spectra. In cases where it was possible multiplets in ¹³C NMR were identified and denoted in experimental part.

UPLC/MS analysis was performed on Waters TQD spectrometer combined with UPLC Acquity H-Class with PDA eLambda detector. Waters Aquity UPLC BEH C18 1,7 µm 2,1x100 mm chromatographic column was used with temperature of 40° C, flow rate of 0.300 ml/min and injection volume was 1.0 µL. All mass spectra were recorded under electrospray ionization in positive mode (ESI+) and chromatograms were recorded with UV detection in range 190-300 nm.

Method A: The gradient conditions used are: 100% phase A (80% water + 20% acetonitrile + 0.1% formic acid) to 100% phase B (acetonitrile + 0.1% formic acid) at 3.5 minutes, equilibrated to initial conditions until 4.0 minutes and kept for additional 2.0 minutes. Total time of analysis - 6.0 minutes.

Method B: The gradient conditions used are: 80% phase A (water + 0.1% formic acid) and 20% phase B (acetonitrile + 0.1% formic acid) to 100% phase B (acetonitrile + 0.1% formic acid) at 3.0 minutes, kept till 3.5 minutes, then equilibrated to initial conditions until 4.0 minutes and kept for additional 2.0 minutes. Total time of analysis - 6.0 minutes.

The compounds in the Table 2 have been obtained according to methods presented in Schemes 1-4 in previous section. Column "synthetic procedure" shows synthetic procedure which was applied. The compounds that are not subject of the present invention are indicated as Reference Example.

### Intermediate 1

### 3-chloro-4-(5-phenyl-1,2,4-oxadiazol-3-yl)aniline

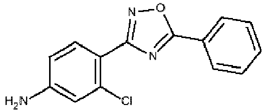

2-chloro-N'-hydroxy-4-nitrobenzene-1-carboximidamide (Scheme I, Step 1a): 2-chloro-4-nitrobenzonitrile (3.50 g, 19.20 mmol, 1 eq) was dissolved in 80 ml of ethanol and hydroxylamine hydrochloride (2.67 g, 38.4 mmol, 2 eq) was added followed by solution of NaOH (1.54 g, 38.4 mmol, 2 eq) in 20 ml of water. Reaction mixture was refluxed for 6 h (TLC control) and ethanol was evaporated in vacuo. 100 ml of water was added and reaction mixture was acidified with 1N HCl Precipitated solid was filtered and identified as byproduct 2-chloro-4-nitrobenzamide (1.80 g, 47%). Filtrate was extracted with AcOEt water layer was alkalized with NH₃aq. Precipitated solid was filtered and purified by maceration from i-PrOH/hexane (1:3) resulting with 0.65 g of product as a white solid (16%).
LCMS (method A) RT=0.86 min, MS (m/z ESI+) 215.8.
¹H NMR (300 MHz, CDCl₃): 9.75 (s, 1H), 8.29 (d, 1H), 8.17 (dd, 2H), 7.67 (dd, 1H), 6.02 (s br., 2H).
¹³C NMR (300 MHz, CDCl₃): 149.8, 148.4, 140.2, 133.8, 132.7, 124.9, 122.4. 3-(2-chloro-4-nitrophenyl)-5-phenyl-1,2,4-oxadiazole (Scheme I, Step 2b): 2-chloro-N'-hydroxy-4-nitrobenzene-1-carboximidamide (0.50 g, 2.32 mmol, 1 eq) was suspended in 20 ml of toluene and benzoyl chloride (0.35 ml, 0.42 g, 3.02 mmol, 1.3 eq) was added followed by K₂CO₃ anh (0.42 g, 3.02 mmol, 1.3 eq). After 30 min of stirring in rt reaction mixture was refluxed for 25 h. reaction mixture was poured into 50 ml of water and was extracted with CHCl₃. After maceration in i-PrOH/hexane (1:2) pure product was filtered as a pale yellow solid (0.60 g, 85.7%).
LCMS (method B) RT=3.77 min, MS (m/z ESI+) 302.4.
¹H NMR (300 MHz, DMSO-d₆): 8.44 (d, 1H), 8.31-8.21 (m, 4H), 7.68-7.55 (m, 3H).
¹³C NMR (300 MHz, DMSO-d₆): 175.9, 166.4, 149.1, 134.9, 133.3, 132.7, 132.1, 129.3, 128.3, 126.1, 123.6, 121.7.

3-chloro-4-(5-phenyl-1,2,4-oxadiazol-3-yl)aniline (Scheme I, Step 3b): To a solution of 3-(2-chloro-4-nitrophenyl)-5-phenyl-1,2,4-oxadiazole (0.56g, 1.86 mmol, 1 eq) in 30 ml of ethanol solution of SnCl₂ (1.42 g, 7.44 mmol, 4 eq) in 2.3 ml of 5N HCl was added dropwise. Reaction mixture was refluxed for 5 h and solvent was removed in vacuo. 50 ml of water was added and reaction mixture was alkalized with 2M NaOH to pH=9 followed by extraction with AcOEt. Crude product was purified by column chromatography over silica gel using CHCl₃/MeOH (99:1) followed by maceration from i-PrOH/hexane (1:2) to yield product as a white solid (0.28g, 56.0%). LC (method B) RT=3.26 min, MS (m/z ESI+) 271.7.
¹H NMR (300 MHz, CDCl₃): 8.20 (m, 2H), 7.88 (d, 1H), 7.60-7.51 (m, 3H), 6.81 (d, 1H), 6.65 (dd, 1H), 4.03 (s br., 2H).
¹³C NMR (300 MHz, CDCl₃): 174.6, 167.8, 149.4, 134.4, 132.9, 132.6, 129.0, 128.1, 124.3, 116.3, 115.5, 113.1.

### Intermediate 2

### 3-chloro-4-[5-(2-chlorophenyl)-1,2,4-oxadiazol-3-yl]aniline

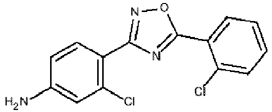

3-(2-chloro-4-nitrophenyl)-5-(2-chlorophenyl)-1,2,4-oxadiazole (Scheme I, Step 2b): Prepared according to procedure described in Intermediate 1 using 2-chloro-N'-hydroxy-4-nitrobenzene-1-carboximidamide) Intermediate 1, Step 1a). Product was purified by maceration from i-PrOH/hexane (1:2) (0.60 g, 85.7%).
LCMS (method A) RT=3.87 min, MS (m/z ESI+) 336.7.
¹H NMR (300 MHz, CDCl₃): 8.45 (d, 1H), 8.33-8.24 (m, 2H), 8.18 (dd, 1H), 7.64-7.57 (m, 2H), 7.47 (dt, 1H).
¹³C NMR (300 MHz, CDCl₃): 174.4, 166.2, 149.2, 134.9, 134.1, 133.6, 132.8, 132.0, 131.8, 131.7, 127.2, 126.1, 122.9, 121.7.

3-chloro-4-[5-(2-chlorophenyl)-1,2,4-oxadiazol-3-yl]aniline (Scheme I, Step 3b): Compound prepared according to procedure described in Intermediate 1 from 3-(2-chloro-4-nitrophenyl)-5-(2-chlorophenyl)-1,2,4-oxadiazole.

Product was purified by maceration from i-PrOH/hexane (1:2) (0.38 g, 69.6%).
LC (method A) RT=3.32 min, MS (m/z ESI+) 306.3.
¹H NMR (300 MHz, CDCl₃): 8.13 (dd, 1H), 7.90 (d, 1H), 7.58 (dd, 1H), 7.50 (dt, 1H), 7.44 (dt, 1H), 6.81 (d, 1H), 6.65 (dd, 1H), 4.03 (s br. 2H). ¹³C NMR (300 MHz, CDCl₃): 173.1, 167.5, 149.5, 134.5, 133.9, 133.0, 132.9, 131.9, 131.4, 127.0, 123.7, 116.3, 115.3, 113.1.

### Intermediate 3

### 3-methoxy-4-(5-phenyl-1,2,4-oxadiazol-3-yl)aniline

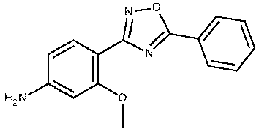

N'-hydroxy-2-methoxy-4-nitrobenzene-1-carboximidamide (Scheme I, Step 1a): Compound was prepared according to procedure described in Intermediate 1 using 2-methoxy-4-nitrobenzonitrile. Precipitated product was purified by maceration from i-PrOH/hexane (1:2) (3.71 g, 62.6%).
LC (method A) RT=0.57 min, MS (m/z ESI+) 212.0
¹H NMR (300 MHz, CDCl₃ + MeOD): 7.72 (d, 1H), 7.69 (m, 1H), 7.64 (d, 1H), 3.88 (s, 3H), 3.82 (s br. 2H).
¹³C NMR (300 MHz, CDCl₃ + MeOD): 157.4, 150.8, 149.1, 130.2, 127.1, 115.6, 106.3, 56.1.

3-(2-methoxy-4-nitrophenyl)-5-phenyl-1,2,4-oxadiazole (Scheme I, Step 2b): Prepared from N'-hydroxy-2-methoxy-4-nitrobenzene-1-carboximid amide according to procedure described in Intermediate 1. Product was purified by maceration from i-PrOH/hexane (1:2) resulting yellow solid (1.71 g, 86.8%).
LCMS (method A) RT=3.49 min, MS (m/z ESI+) 297.5.
¹H NMR (300 MHz, CDCl₃): 8.23-8.16 (m, 3H), 8.01-7.97 (m, 2H), 7.72 (m, 1H), 7.66 (m, 2H), 4.05 (s, 3H).
¹³C NMR (300 MHz, CDCl₃): 175.0, 166.0, 158.7, 150.5, 133.9, 132.4, 130.0, 128.4, 123.6, 121.6, 115.9, 107.6, 57.2.

3-methoxy-4-(5-phenyl-1,2,4-oxadiazol-3-yl)aniline (Scheme I, Step 3b): Compound prepared according to procedure described in Intermediate 1 using 3-(2-methoxy-4-nitrophenyl)-5-phenyl-1,2,4-oxadiazole. Purification of final product was performed by maceration from i-PrOH/hexane (1:2) to yield product as a white solid (1.22g, 79.7%).
LC (method A) RT=2.77 min, MS (m/z ESI+) 267.7
¹H NMR (300 MHz, CDCl₃): 8.20 (m, 2H), 7.97 (d, 1H), 7.55-7.52 (m, 3H), 6.38 (dd, 1H), 6.32 (d, 1H), 4.02 (s br. 2H), 3.94 (s, 3H).
¹³C NMR (300 MHz, CDCl₃): 173.9, 167.4, 159.7, 150.5, 132.7, 132.3, 128.9, 128.1, 124.6, 107.1, 106.0, 98.0, 55.8.

### Intermediate 4

### 4-[5-(2-chlorophenyl)-1,2,4-oxadiazol-3-yl]-3-methoxyaniline

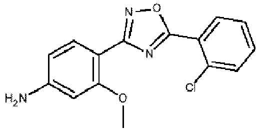

5-(2-chlorophenyl)-3-(2-methoxy-4-nitrophenyl)-1,2,4-oxadiazole (Scheme I, Step 2b): Prepared from N'-hydroxy-2-methoxy-4-nitrobenzene-1-carboximidamide (Intermediate 3, Step 1a) according to procedure described in Intermediate 1. Product was purified by maceration from i-PrOH/hexane (1:2) resulting yellow solid (1.90 g, 88.0%).
LCMS (method A) RT=3.57 min, MS (m/z ESI+) 331.8.
¹H NMR (300 MHz, DMSO): 8.20 (m, 1H), 8.15 (d, 1H), 8.00-7.97 (m, 2H), 7.77-7.68 (m, 2H), 7.60 (dt, 1H), 4.04 (s, 3H).
¹³C NMR (300 MHz, DMSO): 173.4, 165.8, 158.7, 150.6, 134.8, 132.9, 132.7, 132.5, 131.9, 128.5, 122.9, 121.3, 116.0, 107.8, 57.3.

4-[5-(2-chlorophenyl)-1,2,4-oxadiazol-3-yl]-3-methoxyaniline (Scheme I, Step 3b): Compound prepared according to procedure described in Intermediate 1 using 5-(2-chlorophenyl)-3-(2-methoxy-4-nitrophenyl)-1,2,4-oxadiazole. Purification of final product was performed by maceration from i-PrOH/hexane (1:2) to yield product as a white solid (1.33 g, 81.6%). LCMS (method A) RT=2.91 min, MS (m/z ESI+) 301.5
¹H NMR (300 MHz, DMSO): 8.10 (dd, 1H), 7.75-7.66 (m, 3H), 7.59 (dt, 1H), 6.35 (d, 1H), 6.30 (dd, 1H), 5.84 (s br. 2H), 3.81 (s, 3H).
¹³C NMR (300 MHz, DMSO): 171.5, 166.6, 159.3, 153.3, 133.7, 132.1, 131.9, 131.8, 131.1, 127.8, 123.2, 106.0, 101.7, 96.6, 55.2.

### Intermediate 5

### 4-[5-(2-fluorophenyl)-1,2,4-oxadiazol-3-yl]-3-methoxyaniline

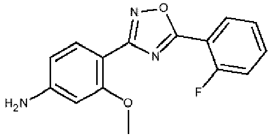

5-(2-fluorophenyl)-3-(2-methoxy-4-nitrophenyl)-1,2,4-oxadiazole (Scheme I, Step 2b): Prepared from N'-hydroxy-2-methoxy-4-nitrobenzene-1-carboximidamide (Intermediate 3, Step 1a) according to procedure described in Intermediate 1. Product was purified by maceration from i-PrOH/hexane (1:2) resulting pale yellow solid (1.81 g, 87.9%).
LCMS (method A) RT=3.43 min, MS (m/z ESI+) 315.4.
¹H NMR (300 MHz, DMSO): 8.19 (m, 2H), 7.99-7.96 (m, 2H), 7.79 (m, 1H), 7.55-7.45 (m, 2H), 4.04 (s, 3H).
¹³C NMR (300 MHz, DMSO): 172.2 (d), 165.8, 162.2, 158.8 (d), 150.7, 136.2 (d), 132.5, 131.3, 125.9 (d), 121.6, 117.7 (d), 115.9, 112.1 (d), 107.8, 57.3.

4-[5-(2-fluorophenyl)-1,2,4-oxadiazol-3-yl]-3-methoxyaniline (Scheme I, Step 3b): Compound prepared according to procedure described in Intermediate 1 using 5-(2-fluorophenyl)-3-(2-methoxy-4-nitrophenyl)-1,2,4-oxadiazole. Purification of final product was performed by maceration from i-PrOH/hexane (1:2) to yield product as a white solid (1.36 g, 88.3%).
LC (method A) RT=2.71 min, MS (m/z ESI+) 285.4.
¹H NMR (300 MHz, DMSO): 8.16 (dt, 1H), 7.74 (m, 1H), 7.70 (d, 1H), 7.54-7.43 (m, 2H), 6.35 (d, 1H), 6.30 (dd, 1H), 5.83 (s br. 2H), 3.81 (s, 3H).
¹³C NMR (300 MHz, DMSO): 169.9 (d), 166.6, 161.5, 159.3, 158.1, 153.3, 135.0 (d), 131.8, 130.7, 125.3 (d), 117.1 (d), 112.1 (d), 106.0, 101.7, 96.6, 55.2. Intermediate 6

### 4-[5-(2-chloro-4-fluorophenyl)-1,2,4-oxadiazol-3-yl]-3-methoxyaniline

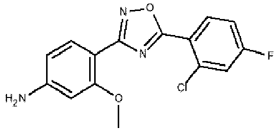

5-(2-chloro-4-fluorophenyl)-3-(2-methoxy-4-nitrophenyl)-1,2,4-oxadiazole (Scheme I, Step 2b): Prepared from N'-hydroxy-2-methoxy-4-nitrobenzene-1-carboximidamide (Intermediate 3, Step 1a) according to procedure described in Intermediate 1. Product was purified by maceration from i-PrOH/hexane (1:2) resulting pale yellow solid (2.10 g, 90.9%).
LCMS (method A) RT=3.66 min, MS (m/z ESI+) 349.6.
¹H NMR (300 MHz, DMSO): 8.27-8.18 (m, 2H), 7.99 (dd, 1H), 7.97 (s, 1H), 7.80 (dd, 1H), 7.51 (ddd, 1H), 4.05 (s, 3H).
¹³C NMR (300 MHz, DMSO): 173.0, 166.3, 165.7, 162.9, 158.7, 150.6, 134.8 (d), 134.6 (d), 132.5, 121.2, 119.8 (d), 119.4 (d), 116.2 (d), 116.0, 107.8, 57.3.

### 4-[5-(2-chloro-4-fluorophenyl)-1,2,4-oxadiazol-3-yl]-3-methoxyaniline

(Method 1, Step 3b): Compound prepared according to procedure described in Intermediate 1 using 5-(2-chloro-4-fluorophenyl)-3-(2-methoxy-4-nitrophenyl)-1,2,4-oxadiazole. Purification of final product was performed by maceration from i-PrOH/hexane (1:2) to yield product as a white solid (1.88 g, 97.9%).
LCMS (method A) RT=3.05 min, MS (m/z ESI+) 319.4.
¹H NMR (300 MHz, DMSO-d₆): 8.16 (dd, 1H), 7.73 (dd, 1H), 7.69 (d, 1H), 7.46 (m, 1H), 6.63 (d, 1H), 6.28 (dd, 1H), 5.82 (s br. 2H), 3.79 (s, 3H).
¹³C NMR (300 MHz, DMSO-d₆): 171.3, 167.1, 164.2 (d), 159.8, 153.8, 134.4 (d), 134.3 (d), 132.3, 120.5 (d), 119.2 (d), 116.0 (d), 106.5, 102.2, 97.1, 55.7.

### Intermediate 7

### 4-(5-phenyl-1,2,4-oxadiazol-3-yl)-3-(trifluoromethyl)aniline

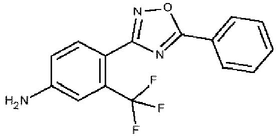

### N'-hydroxy-4-nitro-2-(trifluoromethyl)benzene-1-carboximidamide

(Scheme I, Step 1b): 2-trifluoromethylo-4-nitrobenzonitrile (3.50 g, 16.19 mmol,
1 eq) was dissolved in 80 ml of ethanol and hydroxylamine hydrochloride (5.63 g, 69.5 mmol, 5 eq) was added followed by triethylamine (4.17g, 5.74 ml, 97.14 mmol, 6eq). Reaction mixture was refluxed for 3 h (TLC control) and ethanol was evaporated in vacuo. 100 ml of water was added and reaction mixture was extracted with AcOEt. After evaporation of solvent crude product was purified by column chromatography over silica gel using gradient form CHCl₃ to CHCl₃/MeOH (49:1) followed by maceration from i-PrOH/hexane (1:2)resulting with 1.72 g of white solid (42.7%).
LCMS (method A) RT=1.42 min, MS (m/z ESI+) 249.6.

3-[4-nitro-2-(trifluoromethyl)phenyl]-5-phenyl-1,2,4-oxadiazole (Scheme I, Step 2b): Prepared from N'-hydroxy-4-nitro-2-(trifluoromethyl)benzene-1-carboximidamide according to procedure described in Intermediate 1. Product was purified by maceration from i-PrOH/hexane (1:2) resulting pale yellow solid (0.73 g, 67.6%).
LCMS (method B) RT = 3.76 min, MS (m/z ESI+) 336.6.

4-(5-phenyl-1,2,4-oxadiazol-3-yl)-3-(trifluoromethyl)aniline (Scheme I, Step 3b): Compound prepared according to procedure described in Intermediate 1 using 3-[4-nitro-2-(trifluoromethyl)phenyl]-5-phenyl-1,2,4-oxadiazole. Purification of final product was performed by maceration from i-PrOH/hexane (1:2) to yield product as a white solid (0.45 g, 67.4%).
LCMS (method B) RT=3.31 min, MS (m/z ESI+) 306.6.
¹H NMR (300 MHz, CDCl₃): 8.20 (m, 2H), 7.70 (d, 1H), 7.61-7.54 (m, 3H), 7.09 (d, 1H), 6.88 (dd, 1H), 4.13 (s br. 2H).

### Intermediate 8

### 4-[5-(2-chlorophenyl)-1,2,4-oxadiazol-3-yl]-3-(trifluoromethyl)aniline

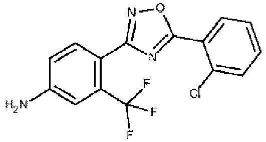

### 5-(2-chlorophenyl)-3-[4-nitro-2-(trifluoromethyl)phenyl]-1,2,4-oxadiazole

(Scheme I, Step 2b): Prepared from N'-hydroxy-4-nitro-2-(trifluoromethyl)benzene-1-carboximidamide (Intermediate 7, Step 1b) according to procedure described in Intermediate 1. Product was purified by column chromatography over silica gel using CHCl₃/hexane (2:1) followed by maceration from i-PrOH/hexane (1:2) to yield product as a pale yellow solid (0.98 g, 40.2%).
LCMS (method A) RT=3.81 min, MS (m/z ESI+) 369.4.
¹H NMR (300 MHz, DMSO-d₆): 8.71 (dd, 1H), 8.66 (d, 1H), 8.30 (d, 1H), 8.19 (m, 1H), 7.80-7.71 (m, 2H).

### 4-[5-(2-chlorophenyl)-1,2,4-oxadiazol-3-yl]-3-(trifluoromethyl)aniline

(Scheme I, Step 3b): Compound prepared according to procedure described in Intermediate 1 using 5-(2-chlorophenyl)-3-[4-nitro-2-(trifluoromethyl)phenyl]-1,2,4-oxadiazole. Purification of final product was performed by maceration from i-PrOH/hexane (1:2) to yield product as a white solid (0.61 g, 75.3%).
LCMS (method A) RT=3.42 min, MS (m/z ESI+) 339.3.
¹H NMR (300 MHz, CDCl₃): 8.14 (m, 1H), 7.72 (d, 1H), 7.57 (m, 1H), 7.52 (dt, 1H), 7.48 (dt, 1H), 7.08 (d, 1H), 6.87 (dd, 1H), 4.14 (s br. 2H).

### Intermediate 9

### 3-methyl-4-(5-phenyl-1,2,4-oxadiazol-3-yl)aniline

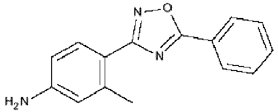

N'-hydroxy-2-methyl-4-nitrobenzene-1-carboximidamide (Scheme I, Step 1a): Compound was prepared according to procedure described in Intermediate 1 using 2-methyl-4-nitrobenzonitrile. Reaction gave mixture of two products: 2-metylo-4-nitrobenzamide isolated after extraction of acidic water layer with AcOEt and N'-hydroxy-2-methyl-4-nitrobenzene-1-carboximidamide isolated after extraction of alkalized water layer with AcOEt. Product was purified by maceration from i-PrOH/hexane (1:2) (0.68 g, 16.2%).
LCMS (method B) RT=0.59 min, MS (m/z ESI+) 196.5.
¹H NMR (300 MHz, DMSO-d₆): 9.64 (s, 1H), 8.10 (d, 1H), 8.05 (dd, 1H), 7.53 (d, 1H), 5.93 (s br. 2H), 2.45 (s, 3H).
¹³C NMR (300 MHz, DMSO-d₆): 150.8, 147.1, 140.6, 138.7, 130.2, 124.6, 120.4, 19.8.

3-(2-methyl-4-nitrophenyl)-5-phenyl-1,2,4-oxadiazole (Scheme I, Step 2b): Prepared from N'-hydroxy-2-methyl-4-nitro-benzene-1-carboximidamide according to procedure described in Intermediate 1. Product was purified by column chromatography over silica gel using CHCl₃ followed by maceration from i-PrOH/hexane (1:2) to yield product as a white solid (0.60 g, 52.2%). LCMS (method B) RT=3.95 min, MS (m/z ESI+) 282.7.

3-methyl-4-(5-phenyl-1,2,4-oxadiazol-3-yl)aniline (Scheme I, Step 3b): Compound prepared according to procedure described in Intermediate 1 using 3-(2-methyl-4-nitrophenyl)-5-phenyl-1,2,4-oxadiazole. Purification of final product was performed by maceration from i-PrOH/hexane (1:2) to yield product as a white solid (0.35 g, 66.0%).
LCMS (method B) RT=3.25 min, MS (m/z ESI+) 252.6.
¹H NMR (300 MHz, CDCl₃): 8.20 (m, 2H), 7.96 (dd, 1H), 7.59-7.51 (m, 3H), 6.62 (m, 2H), 3.89 (s br. 2H), 2.62 (s, 3H).
¹³C NMR (300 MHz, CDCl₃): 174.0, 169.5, 148.5, 140.0, 132.4, 131.8, 129.0, 128.1, 124.6, 117.2, 116.2, 112.2, 22.6.

### Intermediate 10

### 4-[5-(2-chlorophenyl)-1,2,4-oxadiazol-3-yl]-3-methylaniline

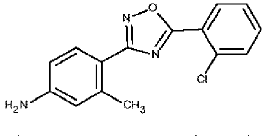

5-(2-chlorophenyl)-3-(2-methyl-4-nitrophenyl)-1,2,4-oxadiazole (Scheme I, Step 2b): Prepared from N'-hydroxy-2-methyl-4-nitro-benzene-1-carboximidamide (Intermediate 9, Step 1a) according to procedure described in Intermediate 1. Product was purified by maceration from i-PrOH/hexane (1:2) to yield product as a white solid (0.83 g, 79.8%).
LCMS (method A) RT = 3.28 min, MS (m/z ESI+) 315.7.
¹H NMR (300 MHz, DMSO-d₆): 8.29-8.22 (m, 3H), 8.18 (dd, 1H), 7.77-7.68 (m, 2H), 7.63-7.58 (m, 1H), 2.72 (s, 3H).
¹³C NMR (300 MHz, DMSO-d₆): 173.9, 167.9, 149.0, 140.6, 134.9, 132.9, 132.7, 131.9, 131.8, 131.7, 128.5, 126.4, 122.8, 121.7, 22.0.

4-[5-(2-chlorophenyl)-1,2,4-oxadiazol-3-yl]-3-methylaniline (Scheme I, Step 3b): Compound prepared according to procedure described in Intermediate 1 using 5-(2-chlorophenyl)-3-(2-methyl-4-nitrophenyl)-1,2,4-oxadiazole. Purification of final product was performed by maceration from i-PrOH/hexane (1:2) to yield product as a white solid (0.55 g, 76.4%).
LCMS (method A) RT=3.37 min, MS (m/z ESI+) 285.4.
¹H NMR (300 MHz, CDCl₃): 8.14 (dd, 1H), 7.98 (m, 1H), 7.56 (m, 1H), 7.49 (dt, 1H), 7.45 (dt, 1H), 6.61 (m, 2H), 3.89 (s br. 2H), 2.63 (s, 3H).
¹³C NMR (300 MHz, CDCl₃): 172.6, 169.2, 148.6, 140.1, 133.8, 132.8, 131.9, 131.8, 131.4, 127.0, 124.0, 117.2, 116.0, 112.3, 22.6.

### Example 1 (Reference Example)

### N-(3-chlorophenyl)-4-[5-(4-methoxyphenyl)-1,2,4-oxadiazol-3-yl]aniline

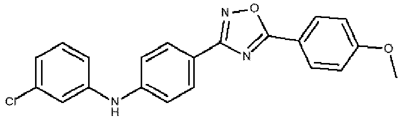

N'-hydroxy-4-nitrobenzene-1-carboximidamide (Scheme I. Step 1a): Compound was prepared according to procedure described in Intermediate 1 using 4-nitrobenzonitrile. After alkalization precipitated solid was filtered and purified by maceration from i-PrOH/hexane (1:3) resulting product as a white solid (9.23 g, 75.5 %).
LCMS (method A) RT=0.65 min, MS (m/z ESI+) 182.2.

5-(4-methoxyphenyl)-3-(4-nitrophenyl)-1,2,4-oxadiazole (Scheme I, Step 2a): N'-hydroxy-4-nitrobenzene-1-carboximidamide (4.00 g, 22.10 mmol, 1.0 eq), 4-methoxybenzoyl chloride (4.52 g, 3.59 ml, 26.52 mmol, 1.2 eq), K₂CO₃ anh. (3.96 g, 28.73 mmol, 1.3 eq) in 40 ml of toluene was heated in MW oven for 10 minutes in 170° C. 20 ml of water was added to reaction mixture and crude product was extracted to CHCl₃. Maceration from EtOH/water (1:1) gave 3.74 g of pure product as a pale yellow solid (57.0%).
¹H NMR (300 MHz, DMSO-d₆): 8.38 (d, 2H), 8.30 (d, 2H), 8.12 (d, 2H), 7.18 (d, 2H), 3.89 (s, 3H).
¹³C NMR (300 MHz, DMSO): 176.5, 167.4, 164.0, 149.8, 132.7, 130.5, 128.9, 124.7, 116.0, 115.6, 56.2.

4-[5-(4-methoxyphenyl)-1,2,4-oxadiazol-3-yl]aniline (Scheme 1, Step 3a): 5-(4-methoxyphenyl)-3-(4-nitrophenyl)-1,2,4-oxadiazole (3.20 g, 10.77 mmol, 1.0 eq) was suspended in 15 ml of ethanol and Fe powder (3.20 g) was added in one portion followed by dropwise addition of 90% solution of CH₃COOH. Reaction mixture was refluxed for 1h and reaction mixture was poured into cold water. Precipitated solid was filtered, dissolved in acetone and refluxed with active carbon for 30 minutes. After filtration and evaporation of solvent water was added and solution was alkalized with NH₃aq. Precipitated solid was filtered and dried resulting with pure product as a white solid (1.88 g, 65.3%).
LCMS (method B) RT= 3.07 min, MS (m/z ESI+) 268.4.
¹H NMR (300 MHz, CDCl₃): 8.14 (d, 2H), 7.96 (d, 2H), 7.02 (d, 2H), 6.75 (d, 2H), 3.95 (s br. 2H), 3.89 (s, 3H).
¹³C NMR (300 MHz, CDCl₃): 175.0, 168.7, 163.0, 149.0, 130.0, 129.0, 117.1, 117.0, 114.7, 114.4, 55.5.

### N-(3-chlorophenyl)-4-[5-(4-methoxyphenyl)-1,2,4-oxadiazol-3-yl]aniline

(Scheme I, Step 4-3): 4-[5-(4-methoxyphenyl)-1,2,4-oxadiazol-3-yl]aniline (0.20 g, 0.75 mmol, 1.0 eq) was dissolved in 10 ml of DCM and 3-chlorobenzeneboronic acid (0.35 g, 2.52 mmol, 3.0 eq), DIPEA (0.39 g, 0.53 ml, 3.00 mmol, 4.0 eq) and Cu(OAc)₂ (0.07 g, 0.40 mmol, 0.5 eq) was added to reaction mixture. After stirring for 24h in rt mixture was evaporated and crude product was purified by column chromatography over silica gel using CHCl₃/MeOH (49:1) followed by maceration from MeOH to yield product as a white solid (0.25g, 88.3%).
LC (method B) RT= 4.16 min, MS (m/z ESI+) 378.1.
¹H NMR (300 MHz, CDCl₃): 8.15 (d, 2H), 8.06 (d, 2H), 7.25 (t, 1H), 7.15 (m, 3H), 7.05-6.98 (m, 4H), 5.99 (s br. 1H), 3.85 (s, 3H).
¹³C NMR (300 MHz, CDCl₃): 175.3, 168.4, 163.1, 145.0, 135.1, 130.0, 129.0, 121.9, 119.7, 118.5, 117.0, 116.8, 114.4, 55.5.

### Example 2 (Reference Example)

### N-(3-methoxyphenyl)-4-[5-(4-methoxyphenyl)-1,2,4-oxadiazol-3-yl]aniline

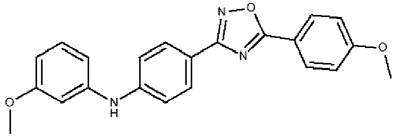

### N-(3-methoxyphenyl)-4-[5-(4-methoxyphenyl)-1,2,4-oxadiazol-3-yl]aniline

(Scheme I, Step 4-3): Compound was prepared from 4-[5-(4-methoxyphenyl)-1,2,4-oxadiazol-3-yl]aniline (Example 1, Step 3a) and 3-methoxybenzeneboronic acid according to procedure described in Example 1. Crude product was purified by column chromatography over silica gel using CHCl₃/MeOH (99:1) followed by maceration from i-PrOH/hexane (1:2) to yield product as a white solid (0.27g, 96.4%).
LCMS (method B) RT= 3.89 min, MS (m/z ESI+) 374.2.
¹H NMR (300 MHz, CDCl₃): 8.16 (d, 2H), 8.14 (d, 2H), 7.23 (t, 1H), 7.15 (d, 2H), 7.13 (d, 2H), 6.73 (m, 1H), 6.57 (dd, 1H), 5.99 (s br. 1H), 3.90 (s, 3H), 3.80 (s, 3H).
¹³C NMR (300 MHz, CDCl₃): 175.2, 168.5, 163.0, 160.7, 145.8, 142.9, 130.2, 130.0, 128.9, 118.8, 117.0, 116.4, 114.4, 111.8, 107.6, 105.0, 55.5, 55.3.

### Example 3 (Reference Example)

### N-(4-methoxyphenyl)-4-[5-(4-methoxyphenyl)-1,2,4-oxadiazol-3-yl]aniline

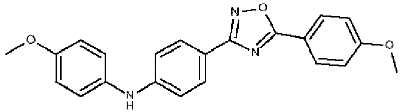

### N-(4-methoxyphenyl)-4-[5-(4-methoxyphenyl)-1,2,4-oxadiazol-3-yl]aniline

(Scheme I, Step 4-3): Compound was prepared from 4-[5-(4-methoxyphenyl)-1,2,4-oxadiazol-3-yl]aniline (Example 1, Step 3a) and 4-methoxybenzeneboronic acid according to procedure described in Example 1. Crude product was purified by column chromatography over silica gel using CHCl₃/MeOH (99:1) followed by maceration from i-PrOH/hexane (1:2) to yield product as a white solid (0.21g, 75.0%).
LCMS (method B) RT= 3.84 min, MS (m/z ESI+) 374.2.
¹H NMR (300 MHz, CDCl₃): 8.15 (d, 2H), 7.98 (d, 2H), 7.14 (d, 2H), 7.03 (d, 2H), 6.91 (m, 4H), 5.78 (s br. 1H), 3.89 (s, 3H), 3.82 (s, 3H).
¹³C NMR (300 MHz, CDCl₃): 175.0, 168.7, 163.0, 156.1, 148.0, 134.1, 130.0, 128.9, 123.7, 117.4, 117.1, 114.7, 114.4, 114.4, 55.5, 55.5.

### Example 4 (Reference Example)

### 4-[5-(4-methoxyphenyl)-1,2,4-oxadiazol-3-yl]-N-[4-(trifluoromethyl)phenyl] aniline

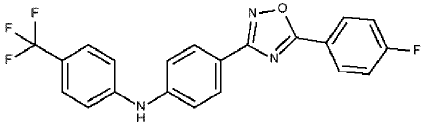

### 5-(4-fluorophenyl)-3-(4-nitrophenyl)-1,2,4-oxadiazole (Scheme I, Step 2a):

Compound was prepared according to procedure described in Example 1 from N'-hydroxy-4-nitrobenzene-1-carboximidamide (Example 1, Step 1a) and 4-fluorobenzoyl chloride. Product was extracted to CHCl₃ and purified by maceration from i-PrOH resulting 4.32 g of pale yellow solid (83.1%).
¹H NMR (300 MHz, DMSO): 8.41 (m, 2H), 8.39-8.22 (m, 4H), 7.49 (m, 2H). ¹³C NMR (300 MHz, DMSO): 175.7, 167.5, 165.6 (d), 149.8, 132.4, 131.4 (d), 129.0, 124.8, 120.3 (d), 117.3 (d).

### 4-[5-(4-fluorophenyl)-1,2,4-oxadiazol-3-yl]aniline (Scheme I. Step 3a):

Compound was synthesized according to procedure described in Example 1 from 5-(4-fluorophenyl)-3-(4-nitrophenyl)-1,2,4-oxadiazole. Precipitated solid was purified by maceration from i-PrOH to yield product as a white solid (2.22 g, 62.0%).
LCMS (method B) RT= 3.13 min, MS (m/z ESI+) 255.7.
¹H NMR (300 MHz, CDCl₃): 8.21 (m, 2H), 7.96 (dd, 2H), 7.24 (t, 2H), 6.76 (d, 2H), 3.98 (s br. 2H).
¹³C NMR (300 MHz, CDCl₃): 174.2, 168.9, 165.3 (d), 149.2, 130.5 (d), 129.0, 120.9, 116.6, 116.4 (d), 114.7.

4-[5-(4-methoxyphenyl)-1,2,4-oxadiazol-3-yl]-N-[4-trifluoromethyl)phenyl]aniline (Scheme I, Step 4-3): Prepared from 4-[5-(4-fluorophenyl)-1,2,4-oxadiazol-3-yl]aniline and 4-trifluoromethylobenzene-boronic acid according to procedure described in Example 1. Crude product was purified by column chromatography over silica gel using CHCl₃/MeOH (99:1) and over aluminum oxide using CHCl₃/hexane (3:1) followed by maceration from i-PrOH/hexane (1:2) to yield product as a white solid (0.13 g, 33.3%).
LCMS (method B) RT= 4.20 min, MS (m/z ESI+) 400.0.
¹H NMR (300 MHz, CDCl₃): 8.23 (dd, 2H), 8.09 (d, 2H), 7.54 (d, 2H), 7.27-7.16 (m, 6H), 6.18 (s br. 1H).

### Example 5 (Reference Example)

### 4-[5-(4-fluorophenyl)-1,2,4-oxadiazol-3-yl]-N-[3-(propan-2-yloxy)phenyl] aniline

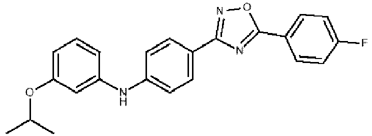

4-[5-(4-fluorophenyl)-1,2,4-oxadiazol-3-yl]-N-[3-propan-2-yloxy)phenyl] aniline (Scheme I, Step 4-3): Prepared from 4-[5-(4-fluorophenyl)-1,2,4-oxadiazol-3-yl]aniline (Example 4, Step 3a) and 3-isopropyloxy-benzeneboronic acid according to procedure described in Example 1. Crude product was purified by column chromatography over aluminum oxide using gradient CHCl₃/hexane (1:1) to CHCl₃/hexane (3:1) followed by maceration from i-PrOH/hexane (1:2) to yield product as a white solid (0.37g, 97.4%). LCMS (method A) RT= 4.21 min, MS (m/z ESI+) 390.1.
¹H NMR (300 MHz, CDCl₃): 8.23 (dd, 2H), 8.04 (d, 2H), 7.24 (m, 3H), 7.14 (d, 2H), 6.72 (m, 2H), 6.58 (m, 1H), 5,97 (s br. 1H), 4.53 (m, 1H), 1.35 (d, 6H). ¹³C NMR (300 MHz, CDCl₃): 174.3, 168.7, 165.4 (d), 159.0, 146.1, 142.7, 130.6 (d), 130.2, 128.9, 120.9 (d), 118.3, 116.6, 116.3 (d), 111.8, 109.7, 107.1, 69.9, 22.1.

### Example 6

### N-{4-[5-(4-methoxyphenyl)-1,2,4-oxadiazol-3-yl]phenyl}pyridine-4-carboxamide

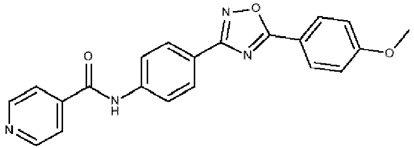

N-{4-[5-(4-methoxyphenyl)-1,2,4-oxadiazol-3-yl]phenyl}pyridine-4-carboxamide (Scheme I, Step 4-1a): 4-[5-(4-methoxyphenyl)-1,2,4-oxadiazol-3-yl]aniline (Example 1, Step 3a) (0.20 g, 0.75 mmol, 1.0 eq) was dissolved in 3 ml of pyridine. Isonicotinoyl chloride, hydrochloride (0.17 g, 0.98 mmol, 1.3 eq) was added in one portion. After stirring overnight in rt reaction mixture was poured into 50 ml of water. Precipitated solid was filtered, washed with water and dried. Crude product was purified by maceration in MeOH to yield product as a white solid (0.26 g, 92.9%). LCMS (method B) RT = 3.03 min, MS (m/z ESI+) 373.4.
¹H NMR (300 MHz, DMSO-d₆): 10.76 (s br. 1H), 8.79 (m, 2H), 8.09 (m, 4H), 8.00 (m, 2H), 7,87 (m, 1H), 7.17 (m, 2H), 3,86 (s, 3H).
¹³C NMR (300 MHz, DMSO-d₆): 175.6, 168.2, 164.8, 163.5, 150.8, 142.1, 141.9, 130.4, 128.3, 122.1, 122.1, 120.9, 116.2, 115.4, 56.1.

### Example 7 (Reference Example)

### N-{4-[5-(4-methoxyphenyl)-1,2,4-oxadiazol-3-yl]phenyl}pyridine-3-carboxamide

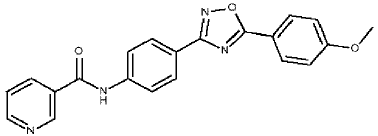

N-{4-[5-(4-methoxyphenyl)-1,2,4-oxadiazol-3-yl]phenyl}pyridine-3-carboxamide (Scheme I, Step 4-1a): Prepared from 4-[5-(4-methoxyphenyl)-1,2,4-oxadiazol-3-yl]aniline (Example 1, Step 3a) and nicotinoyl chloride, hydrochloride according to procedure described in Example 6. Crude product was purified by maceration from MeOH to yield product as a white solid (0.25 g, 89.3%).
LCMS (method B) RT = 3.06 min, MS (m/z ESI+) 372.8.
¹H NMR (300 MHz, DMSO-d₆): 10.70 (s br. 1H), 9.12 (m, 1H), 8.76 (m, 1H), 8.31 (m, 1H), 8.11-7.97 (m, 6H), 7,57 (m, 1H), 7.16 (m, 2H), 3,85 (s, 3H). ¹³C NMR (300 MHz, DMSO-d₆): 175.5, 168.2, 164.8, 163.5, 152.7, 149.2, 142.2, 136.0, 130.8, 130.3, 128.2, 124.0, 121.9, 120.8, 116.2, 115.4, 56.1.

### Example 8 (Reference Example)

### 4-fluoro-N-{4-[5-(4-methoxyphenyl)-1,2,4-oxadiazol-3-yl]phenyl}benzamide

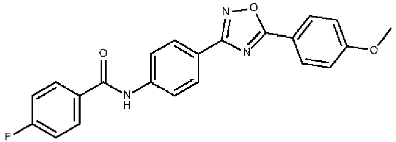

4-fluoro-N-{4-[5-(4-methoxyphenyl)-1,2,4-oxadiazol-3-yl]phenyl}benzamide (Scheme I, Step 4-1a): Prepared from 4-[5-(4-methoxyphenyl)-1,2,4-oxadiazol-3-yl]aniline (Example 1, Step 3a) and 4-fluorobenzoyl chloride according to procedure described in Example 6. Crude product was purified by column chromatography over silica gel using CHCl₃/MeOH (49:1) followed by maceration from MeOH to yield product as a white solid (0.16 g, 73.4%).
LCMS (method B) RT = 3.64 min, MS (m/z ESI+) 390.1.
¹H NMR (300 MHz, DMSO-d₆): 10.53 (s br. 1H), 8.12-7.97 (m, 8H), 7.37 (m, 2H), 7.16 (d, 2H), 3.86 (s, 3H).
¹³C NMR (300 MHz, DMSO-d₆): 175.5, 168.2, 165.2, 164.5 (d), 163.5, 142.5, 131.5 (d), 131.0 (d), 130.3, 128.2, 121.7, 120.8, 116.2, 115.8 (d), 115.4, 56.1.

### Example 9 (Reference Example)

### N-{4-[5-(4-methoxyphenyl)-1,2,4-oxadiazol-3-yl]phenyl}thiophene-2-carboxamide

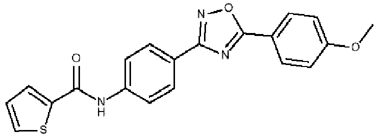

N-{4-[5-(4-methoxyphenyl)-1,2,4-oxadiazol-3-yl]phenyl}thiophene-2-carboxamide (Scheme I, Step 4-1a): Prepared from 4-[5-(4-methoxyphenyl)-1,2,4-oxadiazol-3-yl]aniline (Example 1, Step 3a) and 2-thiophenecarbonyl chloride according to procedure described in Example 6. Crude product was purified by column chromatography over silica gel using CHCl₃/MeOH (49:1) followed by maceration from MeOH to yield product as a white solid (0.17 g, 81.0%).
LCMS (method B) RT = 3.55 min, MS (m/z ESI+) 378.1.
¹H NMR (300 MHz, DMSO-d₆): 10.48 (s br. 1H), 8.13-8.04 (m, 5H), 7.95 (m, 2H), 7.88 (m, 1H), 7.24 (m, 1H), 7.18 (m, 2H), 3.86 (s, 3H).
¹³C NMR (300 MHz, DMSO-d₆): 175.5, 168.2, 163.5, 160.6, 142.1, 140.1, 132.8, 130.4, 130.0, 128.6, 128.2, 121.7, 120.8, 116.2, 115.4, 56.1.

### Example 10 (Reference Example)

### N-{4-[5-(4-fluorophenyl)-1,2,4-oxadiazol-3-yl]phenyl}-1,2-oxazole-5-carboxamide

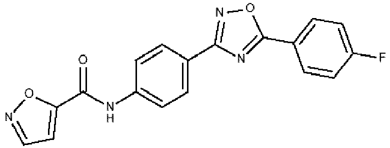

N-{4-[5-(4-fluorophenyl)-1,2,4-oxadiazol-3-yl]phenyl}-1,2-oxazole-5-carboxamide (Scheme I, Step 4-1a): Prepared from 4-[5-(4-fluorophenyl)-1,2,4-oxadiazol-3-yl]aniline (Example 4, Step 3a) and isoxazole-5-carbonyl chloride according to procedure described in Example 6. Crude product was purified by maceration from i-PrOH to yield product as a white solid (0.19 g, 70.4%).
LCMS (method B) RT = 3.34 min, MS (m/z ESI+) 351.1.
¹H NMR (300 MHz, DMSO): 10.99 (s br. 1H), 8.83 (m, 1H), 8.21 (m, 2H), 8.08-7.97 (m, 4H), 7.48 (t, 2H), 7.29 (d, 1H).
¹³C NMR (300 MHz, DMSO): 174.9, 168.3, 165.3 (d), 162.7, 154.7, 152.4, 141.3, 131.2, 128.3, 122.3, 121.2, 120.6, 117.3, 107.6.

### Example 11 (Reference Example)

### 2-chloro-N-{4-[5-(4-fluorophenyl)-1,2,4-oxadiazol-3-yl]phenyl}pyridine-3-carboxamide

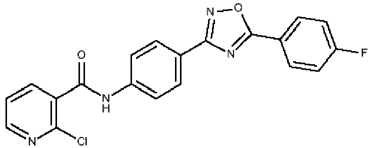

2-chloro-N-{4-[5-(4-fluorophenyl)-1,2,4-oxadiazol-3-yl]phenyl}pyridine-3-carboxamide (Scheme I, Step 4-1a): Prepared from 4-[5-(4-fluorophenyl)-1,2,4-oxadiazol-3-yl]aniline (Example 4, Step 3a) and 2-chloropyridine-3-carbonyl chloride according to procedure described in Example 6. Crude product was purified by column chromatography over silica gel using CHCl₃/MeOH (99:1) followed by maceration from i-PrOH to yield product as a white solid (0.22 g, 71.0%).
LCMS (method B) RT = 3.39 min, MS (m/z ESI+) 395.0.
¹H NMR (300 MHz, CDCl₃): 8.54 (m, 1H), 8.40 (s br. 1H), 8.26-8.18 (m, 5H), 7.81 (m, 2H), 7.42 (m, 1H), 7.25 (m, 2H).
¹³C NMR (300 MHz, CDCl₃): 174.8, 168.3, 167.2 (d), 162.6, 151.6, 140.3, 139.8, 131.0, 130.7 (d), 128.6, 123.1, 122.1 (d), 120.1, 116.5 (d).

### Example 12 (Reference Example)

### N-{4-[5-(2-chlorophenyl)-1,2,4-oxadiazol-3-yl]phenyl}pyridine-2-carboxamide

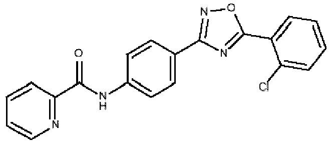

### 5-(2-chlorophenyl)-3-(4-nitrophenyl)-1,2,4-oxadiazole (Scheme I, Step 2a):

Compound was prepared according to procedure described in Example 1 from N'-hydroxy-4-nitrobenzene-1-carboximidamide (Example 1, Step 1a) and 2-chlorobenzoyl chloride. Crude product was purified by maceration in i-PrOH/hexane (1:3) to give 2.48 g of pure product as a pale yellow solid (74.5%).
LCMS (method B) RT = 3.84 min, MS (m/z ESI+) 301.9.

### 4-[5-(2-chlorophenyl)-1,2,4-oxadiazol-3-yl]aniline (Scheme I, Step 3b):

Prepared from 5-(2-chlorophenyl)-3-(4-nitrophenyl)-1,2,4-oxadiazole according to procedure described in Intermediate 1. Crude product was purified by column chromatography over silica gel using CHCl₃/MeOH (99:1) followed by maceration from i-PrOH/hexane (1:3) to yield product as a white solid (0.98 g, 54.1%).
LCMS (method B) RT = 3.21 min, MS (m/z ESI+) 272.3.
¹H NMR (300 MHz, CDCl₃): 8.12 (dd, 1H), 7.98 (m, 2H), 7.57 (dd, 1H), 7.49 (dt, 1H), 7.41 (dt, 1H), 6.75 (m, 2H), 3.98 (s br. 2H).
¹³C NMR (300 MHz, CDCl₃): 173.7, 168.6, 149.3, 133.8, 132.9, 131.9, 131.4, 129.0, 127.1, 123.9, 116.5, 114.7.

N-{4-[5-(2-chlorophenyl)-1,2,4-oxadiazol-3-yl]phenyl}pyridine-2-carboxamide (Scheme I, Step 4-1a): Prepared from 4-[5-(2-chlorophenyl)-1,2,4-oxadiazol-3-yl]aniline and picolinoyl chloride, hydrochloride according to procedure described in Example 6. Crude product was purified by column chromatography over silica gel using CHCl₃/MeOH (99:1) followed by maceration from i-PrOH/hexane (1:2) to yield product as a white solid (0.19 g, 91.8%).
LCMS (method A) RT = 3.82 min, MS (m/z ESI+) 377.1.
¹H NMR (300 MHz, CDCl₃): 10.22 (s br. 1H), 8.64 (m, 1H), 8.32 (m, 1H), 8.23 (m, 2H), 8.15 (m, 1H), 7.97-7.93 (m, 4H), 7.58-7.44 (m, 4H).
¹³C NMR (300 MHz, CDCl₃): 174.2, 168.2, 162.1, 149.4, 148.0, 140.4, 137.8, 133.9, 131.4, 128.6, 127.1, 126.7, 123.7, 122.5, 122.4, 119.6.

### Example 13 (Reference Example)

### N-{4-[5-(2-chlorophenyl)-1,2,4-oxadiazol-3-yl]phenyl}-6-fluoropyridine-2-carboxamide

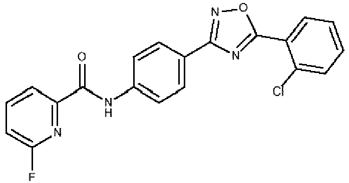

N-{4-[5-(2-chlorophenyl)-1,2,4-oxadiazol-3-yl]phenyl}-6-fluoropyridine-2-carboxamide (Scheme I, Step 4-2): BOP (0.39 g, 0.88 mmol, 1.6 eq) and 6-fluoropyridine-2-carboxylic acid (0.12 g, 0.83 mmol, 1.5 eq) was dissolved under argon in 10 ml of dry MeCN. TEA (0.112 g, 0.154 ml, 1.10 mmol, 2.0 eq) was added dropwise and after 30 minutes of stirring in rt 4-[5-(2-chlorophenyl)-1,2,4-oxadiazol-3-yl]aniline (0.15 g, 0.55 mmol, 1.0 eq) (Example 12, Step 3b) in 5 ml of MeCN was added to reaction mixture. Stirring was continued until TLC showed disappearance of starting aniline. Precipitated solid was filtered directly from reaction mixture and washed with acetonitrile to yield pure product as a white solid (0.19 g, 87.2%).
LCMS (method A) RT=3.86 min, MS (m/z ESI+) 394.8.
¹H NMR (300 MHz, CDCl₃): 9.72 (s, 1H), 8.18 (m, 3H), 8.15 (dd, 1H), 8.03 (dd, 1H), 7.93 (d, 2H), 7.57 (dd, 1H), 7.51 (dt, 1H), 7.42 (dt, 1H), 7.16 (dd, 1H). ¹³C NMR (300 MHz, CDCl₃): 174.2, 168.2, 161.9 (d), 160.7, 148.1 (d), 142.9 (d), 140.0, 133.9, 133.1, 131.9, 131.4, 128.6, 127.1, 123.7, 122.8, 120.2 (d), 119.8, 113.2 (d).

### Example 14 (Reference Example)

### 6-chloro-N-{4-[5-(2-chlorophenyl)-1,2,4-oxadiazol-3-yl]phenyl}pyridine-2-carboxamide

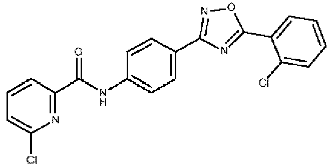

6-chloro-N-{4-[5-(2-chlorophenyl)-1,2,4-oxadiazol-3-yl]phenyl}pyridine-2-carboxamide (Scheme I, Step 4-2): Prepared from 4-[5-(2-chlorophenyl)-1,2,4-oxadiazol-3-yl]aniline (Example 12, Step 3b) and 6-chloropyridine-2-carboxylic acid according to procedure described in Example 13. Precipitated solid was filtered directly from reaction mixture and washed with acetonitrile to yield pure product as a white solid (0.16 g, 70.5%).
LCMS (method A) RT = 3.99 min, MS (m/z ESI+) 410.7.
¹H NMR (300 MHz, CDCl₃): 9.85 (s, 1H), 8.25 (d, 1H), 8.21 (m, 2H), 8.15 (dd, 1H), 7.96-7.87 (m, 3H), 7.60-7.42 (m, 4H).
¹³C NMR (300 MHz, CDCl₃): 174.2, 168.2, 160.7, 150.1, 150.0, 140.4, 140.0, 133.9, 133.1, 131.9, 131.4, 128.6, 127.5, 127.1, 123.7, 122.8, 121.3, 119.9.

### Example 15 (Reference Example)

### N-{4-[5-(2-chlorophenyl)-1,2,4-oxadiazol-3-yl]phenyl}-5-methyl-1,2-oxazole-3-carboxamide

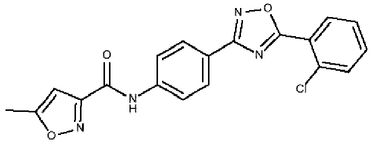

N-{4-[5-(2-chlorophenyl)-1,2,4-oxadiazol-3-yl]phenyl}-5-methyl-1,2-oxadiazole-3-carboxamide (Scheme I, Step 4-1a): Prepared from 4-[5-(2-chlorophenyl)-1,2,4-oxadiazol-3-yl]aniline (Example 12, Step 3b) and 5-methyloxazole-3-carbonyl chloride according to procedure described in Example 6.

Crude product was purified by column chromatography over silica gel using CHCl₃/hexane (3:1) followed by maceration from i-PrOH/hexane (1:2) to yield product as a white solid (0.10 g, 79.4%).
LCMS (method A) RT=3.61 min, MS (m/z ESI+) 381.1.
¹H NMR (300 MHz, CDCl₃): 8.67 (s br. 1H), 8.20 (m, 2H), 8.15 (dd, 1H), 7.82 (m, 2H), 7.61-7.42 (m, 3H), 6.55 (d, 1H).
¹³C NMR (300 MHz, CDCl₃): 174.3, 171.9, 168.1, 158.8, 156.9, 139.7, 133.9, 133.1, 131.9, 131.4, 128.6, 127.1, 123.6, 123.0, 119.8, 101.5, 12.4.

### Example 16 (Reference Example)

### N-[3-methoxy-4-(5-phenyl-1,2,4-oxadiazol-3-yl)phenyl]pyridine-2-carboxamide

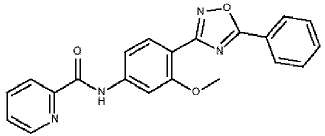

N-[3-methoxy-4-(5-phenyl-1,2,4-oxadiazol-3-yl)phenyl]pyridine-2-carboxamide (Scheme I, Step 4-1a): Prepared from 3-methoxy-4-(5-phenyl-1,2,4-oxadiazol-3-yl)aniline (Intermediate 3, Step 3b) and picolinoyl chloride, hydrochloride. Crude product was purified by column chromatography over silica gel using CHCl₃/hexane (2:1) followed by maceration from i-PrOH/hexane (1:2) to yield product as a white solid (0.15 g, 71.4%).
LC (method A) RT=3.43 min, MS (m/z ESI+) 374.4.
¹H NMR (300 MHz, CDCl₃): 10.23 (s br. 1H), 8.64 (m, 1H), 8.31 (dt, 1H), 8.24-8.16 (m, 3H), 7.97-7.91 (m, 2H), 7.60-7.49 (m, 4H), 7.27 (dd, 1H), 4.09 (s, 3H). ¹³C NMR (300 MHz, CDCl₃): 174.3, 167.1, 162.3, 159.1, 149.4, 148.0, 141.5, 137.9, 132.5, 131.9, 129.0, 128.2, 126.8, 124.4, 122.4, 111.6, 111.4, 102.9, 56.1.

### Example 17

### N-{4-[5-(2-chlorophenyl)-1,2,4-oxadiazol-3-yl]-3-methoxyphenyl}pyridine-2-carboxamide

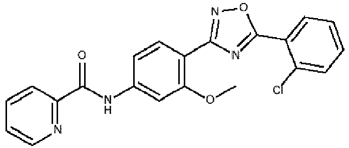

N-{4-[5-(2-chlorophenyl)-1,2,4-oxadiazol-3-yl]-3-methoxyphenyl}pyridine-2-carboxamide (Scheme I, Step 4-1a): Prepared from 4-[5-(2-chlorophenyl)-1,2,4-oxadiazol-3-yl]-3-methoxyaniline (Intermediate 4, Step 3b) and picolinoyl chloride, hydrochloride. Crude product was purified by column chromatography over silica gel using gradient from CHCl₃/hexane (2:1) to CHCl₃/MeOH (99:1) followed by maceration from i-PrOH/hexane (1:2) to yield product as a white solid (0.113 g, 56.5%).
LCMS (method A) RT=3.52 min, MS (m/z ESI+) 406.3.
¹H NMR (300 MHz, CDCl₃): 10.23 (s br. 1H), 8.63 (m, 1H), 8.30 (m, 1H), 8.17 (d, 1H), 8.13 (dd, 1H), 7.98 (dd, 1H), 7.93 (dt, 1H), 7.59-7.42 (m, 4H), 7.26 (dd, 1H).
¹³C NMR (300 MHz, CDCl₃): 172.9, 166.8, 162.3, 159.1, 149.4, 148.0, 141.6, 137.8, 133.9, 132.9, 132.0, 131.9, 131.4, 127.0, 126.8, 123.8, 122.4, 111.4, 102.9, 56.2.

### Example 18 (Reference Example)

### N-{4-[5-(2-fluorophenyl)-1,2,4-oxadiazol-3-yl]-3-methoxyphenyl}pyridine-2-carboxamide

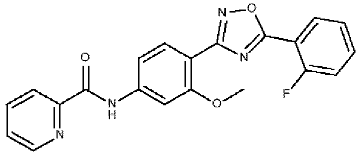

N-{4-[5-(2-fluorophenyl)-1,2,4-oxadiazol-3-yl]-3-methoxyphenyl}pyridine-2-carboxamide (Scheme I, Step 4-1a): Prepared from 4-[5-(2-fluorophenyl)-1,2,4-oxadiazol-3-yl]-3-methoxyaniline (Intermediate 5, Step 3b) and picolinoyl chloride, hydrochloride. Crude product was purified by column chromatography over aluminum oxide using AcOEt/hexane (2:1) followed by maceration from MeOH to yield product as a white solid (0.128 g, 62.4%).
LCMS (method A) RT = 3.33 min, MS (m/z ESI+) 390.3.
¹H NMR (300 MHz, CDCl₃): 10.23 (s br. 1H), 8.63 (m, 1H), 8.30 (m, 1H), 8.23-8.15 (m, 2H), 7.98 (d, 1H), 7.93 (dt, 1H), 7.55 (m, 1H), 7.51 (m, 2H), 7.35-7.24 (m, 3H), 4.08 (s, 3H).
¹³C NMR (300 MHz, CDCl₃): 171.3 (d), 166.9, 162.3, 160.8 (d), 159.1, 149.3, 148.0, 141.6, 137.9, 134.4 (d), 131.9, 130.9 (d), 126.8, 124.6 (d), 122.4, 117.1 (d), 113.0 (d), 111.4, 111.4, 102.9, 56.1.

### Example 19

### N-{4-[5-(2-chloro-4-fluorophenyl)-1,2,4-oxadiazol-3-yl]-3-methoxyphenyl} pyridine-2-carboxamide

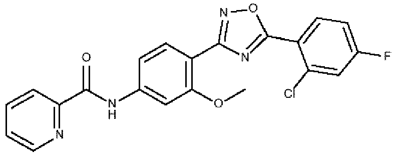

N-{4-[5-(2-chloro-4-fluorophenyl)-1,2,4-oxadiazol-3-yl]-3-methoxyphenyl} pyridine-2-carboxamide (Scheme I, Step 4-1a): Prepared from 4-[5-(2-chloro-4-fluorophenyl)-1,2,4-oxadiazol-3-yl]-3-methoxyaniline (Intermediate 6, Step 3b) and picolinoyl chloride, hydrochloride. Crude product was purified by column chromatography over silica gel using CHCl₃/hexane (2:1) followed by maceration from i-PrOH/hexane (1:2) to yield product as a white solid (0.18 g, 90.0%).
LCMS (method A) RT=3.65 min, MS (m/z ESI+) 424.2.
¹H NMR (300 MHz, CDCl₃): 10.23 (s br. 1H), 8.64 (m, 1H), 8.30 (m, 1H), 8.18 (m, 2H), 7.98 (d, 1H), 7.94 (dt, 1H), 7.51 (m, 1H), 7.32 (dd, 1H), 7.26 (dd, 1H), 7.16 (m, 1H), 4.09 (s, 3H).
¹³C NMR (300 MHz, CDCl₃): 172.1, 166.8, 164.3 (d), 162.3, 159.1, 149.3, 148.0, 141.7, 137.9, 135.5 (d), 133.7 (d), 132.0, 126.8, 122.4, 120.2 (d), 118.9 (d), 114.8 (d), 111.4, 111.3, 102.9, 56.2.

### Example 20 (Reference Example)

### N-[3-chloro-4-(5-phenyl-1,2,4-oxadiazol-3-yl)phenyl]pyridine-2-carboxamide

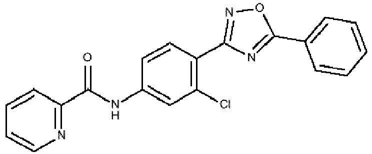

N-[3-chloro-4-(5-phenyl-1,2,4-oxadiazol-3-yl)phenyl]pyridine-2-carboxamide (Scheme I, Step 4-1b): 3-chloro-4-(5-phenyl-1,2,4-oxadiazol-3-yl)aniline (0.12 g, 0.442 mmol, 1.0 eq) (Intermediate 1, Step 3b) was dissolved in 5 ml of THF and TEA (0.174 g, 0.24 ml, 1.77 mmol, 4.0 eq) was added dropwise. Picolinoyl chloride, hydrochloride (0.12 g, 0.66 mmol, 1.5 eq) was added in few portions over 5 minutes. After stirring overnight in rt solvent was removed in vacuo. Water was then added to residual oil and after stirring precipitated solid was filtered, dried and purified by column chromatography over silica gel using CHCl₃/MeOH (99:1) followed by maceration from i-PrOH/hexane (1:2) to yield product as a white solid (0.12 g, 72.3%).
LCMS (method A) RT=3.80 min, MS (m/z ESI+) 376.4.
¹H NMR (300 MHz, CDCl₃): 10.21 (s br. 1H), 8.62 (m, 1H), 8.30 (dd, 1H), 8.22 (m, 2H), 8.12-8.08 (m, 2H), 7.90 (m, 1H), 7.84 (m, 1H), 7.61-7.49 (m, 4H).
¹³C NMR (300 MHz, CDCl₃): 175.0, 167.4, 162.2, 149.1, 148.1, 140.6, 137.8, 134.2, 132.8, 132.4, 129.1, 128.2, 126.9, 124.1, 122.6, 121.6, 121.3, 117.6.

### Example 21 (Reference Example)

### N-{3-chloro-4-[5-(2-chlorophenyl)-1,2,4-oxadiazol-3-yl]phenyl}pyridine-2-carboxamide

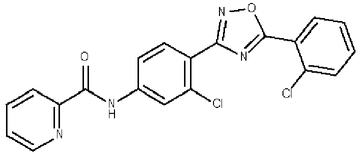

### N-(3-chloro-4-cyanophenyl)pyridine-2-carboxamide (Scheme II, Step 1):

4-amino-2-chlorobenzonitrile (5.00 g, 32.77 mmol, 1.00 eq) was dissolved in 100 ml of THF. TEA (9.92 g, 13.68 ml, 98.31 mmol, 3.0 eq) was added dropwise followed by addition of picolinoyl chloride, hydrochloride (7.00 g, 39.32 mmol, 1.2 eq). Reaction mixture was stirred in RT overnight. Solvent was evaporated, 60 ml of water was added and resulted mixture was extracted with CHCl₃. After evaporation of solvent crude product was purified by column chromatography over silica gel using CHCl₃/MeOH (49:1) to give 3.27 g of product as a pale yellow solid (38.7%).
LCMS (method A) RT = 3.04 min, MS (m/z ESI+) 257.9.

### N-[3-chloro-4-(N'-hydroxycarbamimidoyl]phenyl]pyridine-2-carboxamide (Scheme II, Step 2):

Prepared from N-(3-chloro-4-cyanophenyl)pyridine-2-carboxamide according to procedure described in Example 1. Reaction gave mixture of two products: N-(4-carbamoyl-3-chlorophenyl)pyridine-2-carboxamide (1.90 g, 52%) isolated after extraction of acidic water layer with AcOEt and N-{3-chloro-4-[(Z)-N'-hydroxycarbamimidoyl]phenyl}pyridine-2-carboxamide isolated after extraction of alkalized water layer with AcOEt. Finally crude product was purified by maceration from i-PrOH/hexane (1:2) (0.51 g, 13.9%).
LCMS (method A) RT = 0.48-0.83 min, MS (m/z ESI+) 290.4.
¹H NMR (300 MHz, CDCl₃+MeOD): 8.53 (m, 1H), 8.15 (m, 1H), 7.95 (d, 1H), 7.85 (dt, 1H), 7.53 (dd, 1H), 7.45 (m, 1H), 7.37 (d, 1H), 3.70 (s br. 3H). ¹³C NMR (300 MHz, CDCl₃): 162.5, 151.8, 148.9, 148.1, 139.5, 137.9, 133.3, 131.4, 127.5, 126.9, 122.5, 120.6, 117.7.

N-{3-chloro-4-[5-(2-chlorophenyl)-1,2,4-oxadiazol-3-yl]phenyl}pyridine-2-carboxamide (Scheme II, Step 3): N-[3-chloro-4-(N'-hydroxy carbamimidoyl)phenyl]pyridine-2-carboxamide (0.15 g, 0.516 mmol, 1.0eq) was suspended in 10 ml of toluene and 2-chlorobenzoyl chloride (0.12 g, 0.087 ml, 0.67 mmol, 1.3 eq) was added dropwise followed by addition of K₂CO₃ (0.092 g, 0.67 mmol, 1.3 eq). After stirring for 30 minutes in rt reaction mixture was refluxed over 4h. 50 ml of water was then poured into reaction flask and solution was extracted with chloroform. Organic layers were dried and solvent was removed in vacuo. Crude product was purified by column chromatography over silica gel using CHCl₃ followed by maceration from i-PrOH/hexane (1:2) to yield product as a white solid (0.18 g, 85.7%). LCMS (method A) RT = 3.89 min, MS (m/z ESI+) 410.8.
¹H NMR (300 MHz, CDCl₃): 10.22 (s br. 1H), 8.63 (m, 1H), 8.30 (m, 1H), 8.18-8.10 (m, 3H), 7.94 (dt, 1H), 7.82 (dd, 1H), 7.61-7.44 (m, 4H). ¹³C NMR (300 MHz, CDCl₃): 173.6, 167.2, 162.2, 149.1, 148.1, 140.6, 137.8, 134.3, 133.9, 133.2, 132.5, 132.0, 131.5, 127.1, 126.9, 123.5, 122.6, 121.3, 117.6.

### Example 22

### N-{3-chloro-4-[5-(2-chloro-4-fluorophenyl)-1,2,4-oxadiazol-3-yl]phenyl}pyridine-2-carboxamide

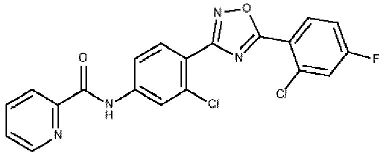

### N-{3-chloro-4-[5-(2-chloro-4-fluorophenyl)-1,2,4-oxadiazol-3-yl]phenyl} pyridine-2-carboxamide

(Scheme II, Step 3): Prepared from N-[3-chloro-4-(N'-hydroxycarbamimidoyl)phenyl]pyridine-2-carboxamide (Example 21, Step 2) and 2-chloro-4-fluorobenzoyl chloride according to procedure described in Example 21. Crude product was purified by column chromatography over silica gel using CHCl₃ followed by maceration from i-PrOH/hexane (1:2) to yield product as a white solid (0.15 g, 68.2%).
LCMS (method A) RT=3.96 min, MS (m/z ESI+) 428.7.
¹H NMR (300 MHz, CDCl₃): 10.23 (s br. 1H), 8.63 (m, 1H), 8.30 (d, 1H), 8.19 (dd, 1H), 8.12 (d, 1H), 8.10 (d, 1H), 7.94 (dt, 1H), 7.82 (dd, 1H), 7.53 (m, 1H), 7.34 (dd, 1H), 7.17 (m, 1H).
¹³C NMR (300 MHz, CDCl₃): 172.8, 167.2, 164.5 (d), 162.7, 162.3, 149.0, 148.1, 140.7, 137.8, 135.6 (d), 134.3, 133.7 (d), 132.5, 126.9, 122.6, 121.2 (d), 119.9 (d), 119.2, 118.9, 117.6, 114.9 (d).

### Example 23 (Reference Example)

### N-{3-chloro-4-[5-(2-methoxyphenyl)-1,2,4-oxadiazol-3-yl]phenyl}pyridine-2-carboxamide

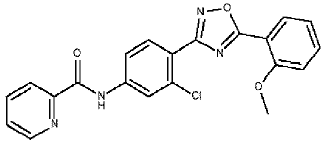

N-{3-chloro-4-[5-(2-methoxyphenyl)-1,2,4-oxadiazol-3-yl]phenyl}pyridine-2-carboxamide (Scheme II, Step 3): Prepared from N-[3-chloro-4-(N'-hydroxycarbamimidoyl)phenyl]pyridine-2-carboxamide (Example 21, Step 2) and 2-methoxybenzoyl chloride according to procedure described in Example 21. Crude product was purified by column chromatography over silica gel using gradient from CHCl₃/hexane (1:1) to CHCl₃/MeOH (99:1) and then over aluminum oxide using AcOEt/hexane (1:2) followed by maceration from i-PrOH/hexane/CHCl₃ (1:1:1) to yield product as a white solid (0.13 g, 41.9%). LCMS (method A) RT=3.63 min, MS (m/z ESI+) 406.5.
¹H NMR (300 MHz, CDCl₃): 10.20 (s br. 1H), 8.62 (m, 1H), 8.16-8.08 (m, 3H), 7.92 (dt, 1H), 7.81 (dd, 1H), 7.59-7.49 (m, 2H), 7.10 (m, 2H), 4.00 (s, 3H). ¹³C NMR (300 MHz, CDCl₃): 174.4, 166.8, 162.2, 158.6, 149.1, 148.1, 140.4, 137.8, 134.2, 134.1, 132.4, 131.7, 126.9, 122.6, 121.8, 121.2, 120.8, 117.6, 113.3, 112.1, 56.1.

### Example 24 (Reference Example)

### N-[3-fluoro-4-(5-phenyl-1,2,4-oxadiazol-3-yl)phenyl]pyridine-2-carboxamide

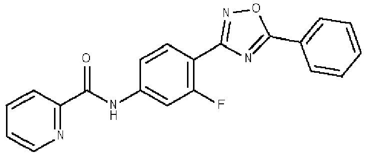

### N-(4-cyano-3-fluorophenyl)pyridine-2-carboxamide (Scheme II, Step 1):

Prepared according to procedure described in Example 21 from 4-amino-2-fluorobenzonitrile. Crude product was purified by column chromatography over silica gel using CHCl₃/MeOH (49:1) to give 3.91 g of product as a pale yellow solid (88.3%).
LCMS (method A) RT = 2.84 min, MS (m/z ESI+) 241.7.
¹H NMR (300 MHz, CDCl₃): 10.33 (s br. 1H), 8.63 (m, 1H), 8.28 (d, 1H), 8.01-7.92 (m, 2H), 7.62-7.52 (m, 2H), 7.48 (dd, 1H).
¹³C NMR (300 MHz, CDCl₃): 163.9 (d), 162.4, 148.5, 148.2, 143.6 (d), 138.0, 133.9 (d), 127.3, 122.7, 115.2 (d), 114.3, 107.0 (d), 96.0 (d).

### N-{3-fluoro-4-[N'-hydroxycarbamimidoyl]phenyl}pyridine-2-carboxamide

(Scheme II, Step 2): Compound synthesized according to procedure described in Intermediate 1 from N-(4-cyano-3-fluorophenyl)pyridine-2-carboxamide. Isolated product was purified by maceration in i-PrOH/hexane (1:2) resulting 1.12 g of white solid (52.6%).
LCMS (method A) RT = 0.71 min, MS (m/z ESI+) 275.0.
¹H NMR (300MHz, CDCl₃ + DMSO-d₆): 10.37 (s br. 1H), 9.57 (s br. 1H), 8.59 (m,1H), 8.15(m,1H), 7.92-7.81(m,2H), 7.59-7.39(m,3H), 5.29(s br.2H).
¹³C NMR (300MHz, CDCl₃ + DMSO-d₆): 161.9, 159.5, 148.7, 148.1(d), 147.6, 139.6(d), 137.3, 129.1(d), 126.3, 121.9, 115.8(d),114.7(d), 106.8(d).

N-[3-fluoro-4-(5-phenyl-1,2,4-oxadiazol-3-yl)phenyl]pyridine-2-carboxamide (Scheme II, Step 3): Prepared from N-{3-fluoro-4-[N'-hydroxycarbamimidoyl]phenyl}pyridine-2-carboxamide and benzoyl chloride according to procedure described in Example 21. Crude product was purified by column chromatography over silica gel using CHCl₃/MeOH (49:1) followed by maceration from i-PrOH/hexane (1:2) to yield product as a white solid (0.16 g, 80.4%).
LCMS (method A) RT=3.65 min, MS (m/z ESI+) 360.7.
¹H NMR (300 MHz, CDCl₃): 10.25 (s br. 1H), 8.63 (m, 1H), 8.30 (m, 1H), 8.24-8.15 (m, 3H), 8.02-7.90 (m, 2H), 7.61-7.49 (m, 5H).
¹³C NMR (300 MHz, CDCl₃): 175.0, 165.5 (d), 162.2, 161.2 (d), 149.1, 148.1, 141.6 (d), 137.8, 132.8, 131.2 (d), 129.1, 128.2, 126.9, 124.1, 122.6, 115.0 (d), 110.6 (d), 107.6 (d).

### Example 25 (Reference Example)

### N-{4-[5-(2-chlorophenyl)-1,2,4-oxadiazol-3-yl]-3-fluorophenyl}pyridine-2-carboxamide

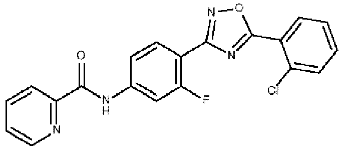

N-{4-[5-(2-chlorophenyl)-1,2,4-oxadiazol-3-yl]-3-fluorophenyl}pyridine-2-carboxamide (Scheme II, Step 3): Prepared from N-{3-fluoro-4-[N'-hydroxycarbamimidoyl]phenyl}pyridine-2-carboxamide (Example 24, Step 2) and 2-chlorobenzoyl chloride according to procedure described in Example 21. Crude product was purified by column chromatography over silica gel using CHCl₃/MeOH (49:1) followed by maceration from i-PrOH/hexane (1:2) to yield product as a white solid (0.17 g, 78.7%).
LCMS (method B) RT = 3.77 min, MS (m/z ESI+) 395.0.
¹H NMR (300 MHz, CDCl₃): 10.98 (s br. 1H), 8.75 (m, 1H), 8.19-8.04 (m, 5H), 7.98 (dd, 1H), 7.75-7.66 (m, 3H), 7.63-7.58 (m, 1H).
¹³C NMR (300 MHz, CDCl₃): 173.8, 165.2 (d), 163.7, 160.5 (d), 149.9, 148.9, 143.4 (d), 138.6, 134.6, 132.9, 132.6, 131.8, 131.2 (d), 128.4, 127.7, 123.2, 123.1, 116.8 (d), 109.5 (d), 108.3 (d).

### Example 26

### N-{4-[5-(2-chloro-4-fluorophenyl)-1,2,4-oxadiazol-3-yl]-3-fluorophenyl} pyridine-2-carboxamide

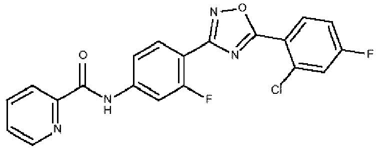

N-{4-[5-(2-chloro-4-fluorophenyl)-1,2,4-oxadiazol-3-yl]-3-fluorophenyl} pyridine-2-carboxamide (Scheme II, Step 3): Prepared from N-{3-fluoro-4-[N'-hydroxycarbamimidoyl]phenyl}pyridine-2-carboxamide (Example 24, Step 2) and 2-chloro-4-fluorobenzoyl chloride according to procedure described in Example 21. Crude product was purified by column chromatography over silica gel using CHCl₃/MeOH (99:1) followed by maceration from i-PrOH/hexane (1:2) to yield product as a white solid (0.18 g, 79.6%).
LCMS (method B) RT = 3.84 min, MS (m/z ESI+) 413.1.
¹H NMR (300 MHz, CDCl₃): 10.80 (s br. 1H), 8.70 (m, 1H), 8.20 (m, 2H), 8.12-7.97 (m, 3H), 7.85 (dd, 1H), 7.60 (m, 1H), 7.50 (dd, 1H), 7.34 (dt, 1H).
¹³C NMR (300 MHz, CDCl₃): 176.2, 172.9, 166.2, 165.2, 163.2, 162.8, 162.4, 159.0, 149.7, 148.5, 143.1 (d), 138.1, 135.0 (d), 131.0 (d), 127.2, 122.9, 120.0 (d), 119.1 (d), 116.3 (d), 115.5 (d), 109.6 (d), 108.0 (d).

### Example 27

### N-{4-[5-(2-methoxyphenyl)-1,2,4-oxadiazol-3-yl]-3-fluorophenyl}pyridine-2-carboxamide

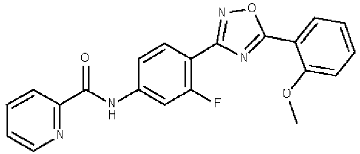

N-{4-[5-(2-methoxyphenyl)-1,2,4-oxadiazol-3-yl]-3-fluorophenyl}pyridine-2-carboxamide (Scheme II, Step 3): Prepared from N-{3-fluoro-4-[N'-hydroxycarbamimidoyl]phenyl}pyridine-2-carboxamide (Example 24, Step 2) and 2-methoxybenzoyl chloride according to procedure described in Example 21. Crude product was purified by column chromatography over silica gel using CHCl₃/MeOH (99:1) followed by maceration from i-PrOH/hexane (1:2) to yield product as a white solid (0.16 g, 74.8%).
LCMS (method B) RT = 3.49 min, MS (m/z ESI+) 390.8.
¹H NMR (300 MHz, CDCl₃): 10.25 (s br. 1H), 8.63 (m, 1H), 8.30 (m, 1H), 8.14 (m, 1H), 8.01-7.93 (m, 2H), 7.59-7.51 (m, 3H), 7.13-7.07 (m, 2H), 4.00 (s, 3H). ¹³C NMR (300 MHz, CDCl₃): 174.5, 164.9 (d), 162.2, 161.2 (d), 158.6, 149.1, 141.4, 137.9, 134.1, 131.7, 131.2 (d), 126.9, 122.6, 120.8, 115.0 (d), 113.3, 112.1, 110.9 (d), 107.6 (d), 56.1.

### Example 28

### N-[4-(5-phenyl-1,2,4-oxadiazol-3-yl)-3-trifluoromethyl)phenyl]pyridine-2-carboxamide

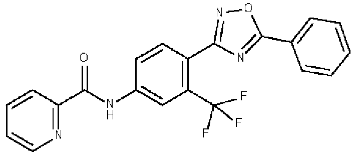

N-[4-(5-phenyl-1,2,4-oxadiazol-3-yl)-3-trifluoromethyl)phenyl]pyridine-2-carboxamide (Scheme I, Step 4-1b): Prepared from 4-(5-phenyl-1,2,4-oxadiazol-3-yl)-3-(trifluoromethyl)aniline (Intermediate 7, Step 3b) and picolinoyl chloride, hydrochloride according to procedure described in Example 20. Crude product was purified by column chromatography over silica gel using CHCl₃ followed by maceration from i-PrOH/hexane (1:2) to yield product as a white solid (0.417 g, 86.2%).
LCMS (method B) RT = 3.80 min, MS (m/z ESI+) 411.6.
¹H NMR (300 MHz, CDCl₃): 10.34 (s br. 1H), 8.65 (m, 1H), 8.33-8.27 (m, 2H), 8.23-8.19 (m, 3H), 7.98-7.92 (m, 2H), 7.65-7.52 (m, 4H).

### Example 29

### N-{4-[5-(2-chlorophenyl)-1,2,4-oxadiazol-3-yl]-3-(trifluoromethyl)phenyl} pyridine-2-carboxamide

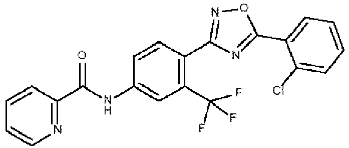

N-{4-[5-(2-chlorophenyl)-1,2,4-oxadiazol-3-yl]-3-(trifluoromethyl)phenyl} pyridine-2-carboxamide (Scheme I, Step 4-1b): Prepared from 4-[5-(2-chlorophenyl)-1,2,4-oxadiazol-3-yl]-3-(trifluoromethyl)aniline (Intermediate 8, Step 3b) and picolinoyl chloride, hydrochloride according to procedure described in Example 20. Crude product was purified by column chromatography over silica gel using CHCl₃ followed by maceration from i-PrOH/hexane (1:2) to yield product as a white solid (0.15 g, 95.5%). LCMS (method A) RT = 3.85 min, MS (m/z ESI+) 445.0.
¹H NMR (300 MHz, CDCl₃): 10.35 (s br. 1H), 8.65 (m, 1H), 8.30 (m, 1H), 8.22-8.15 (m, 2H), 7.99-7.92 (m, 2H), 7.61-7.42 (m, 4H).

### Example 30

### N-[3-methyl-4-(5-phenyl-1,2,4-oxadiazol-3-yl)phenyl]pyridine-2-carboxamide

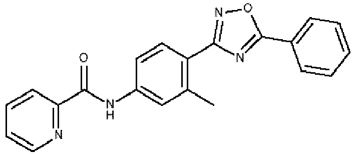

N-[3-methyl-4-(5-phenyl-1,2,4-oxadiazol-3-yl)phenyl]pyridine-2-carboxamide (Scheme I, Step 4-1b): Prepared from 3-methyl-4-(5-phenyl-1,2,4-oxadiazol-3-yl)aniline (Intermediate 9, Step 3b) and picolinoyl chloride, hydrochloride according to procedure described in Example 20. Crude product was purified by column chromatography over silica gel using CHCl₃/MeOH (99:1) followed by maceration from i-PrOH/hexane (1:2) to yield product as a white solid (0.21 g, 75.0%).
LCMS (method B) RT = 3.89 min, MS (m/z ESI+) 357.0.
¹H NMR (300 MHz, CDCl₃): 10.77 (s br. 1H), 8.15 (m, 3H), 8.09-7.98 (m, 4H), 7.70-7.64 (m, 4H), 2.61 (s, 3H).
¹³C NMR (300 MHz, CDCl₃): 174.6, 169.0, 163.2, 150.0, 148.9, 141.0, 138.8, 138.6, 133.7, 130.9, 130.0, 128.3, 127.6, 123.9, 122.9, 121.2, 118.1, 22.6.

### Example 31

### N-{4-[5-(2-chlorophenyl)-1,2,4-oxadiazol-3-yl]-3-methylphenyl}pyridine-2-carboxamide

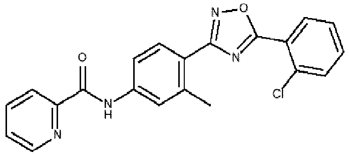

N-{4-[5-(2-chlorophenyl)-1,2,4-oxadiazol-3-yl]-3-methylphenyl}pyridine-2-carboxamide (Scheme I, Step 4-1a): Prepared from 4-[5-(2-chlorophenyl)-1,2,4-oxadiazol-3-yl]-3-methylaniline (Intermediate 10, Step 3b) and picolinoyl chloride, hydrochloride according to procedure described in Example 6. Crude product was purified by column chromatography over silica gel using CHCl₃/MeOH (99:1) followed by maceration from i-PrOH/hexane (1:2) to yield product as a white solid (0.41 g, 93.6%).
LCMS (method A) RT=3.94 min, MS (m/z ESI+) 390.5.
¹H NMR (300 MHz, CDCl₃): 10.15 (s br. 1H), 8.63 (m, 1H), 8.31 (d, 1H), 8.17 (m, 2H), 7.93 (dt, 1H), 7.78 (m, 2H), 7.60-7.41 (m, 4H), 2.75 (s, 3H). ¹³C NMR (300 MHz, CDCl₃): 173.1 168.9, 162.1, 149.5, 148.0, 139.8, 139.7, 137.8, 133.9, 133.0, 131.9, 131.4, 131.3, 127.1, 126.6, 123.7, 122.5, 121.9, 121.8, 116.9, 22.6.

### Example 32

### N-{4-[5-(2-chlorophenyl)-1,2,4-oxadiazol-3-yl]-3-methoxyphenyl}-6-fluoropyridine-2-carboxamide

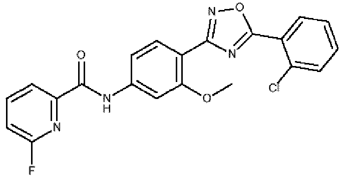

N-{4-[5-(2-chlorophenyl)-1,2,4-oxadiazol-3-yl]-3-methoxyphenyl}-6-fluoropyridine-2-carboxamide (Scheme I, Step 4-2): BOP (0.22 g, 0.50 mmol, 1.0 eq) and 6-fluoropyridine-2-carboxylic acid (0.08 g, 0.55 mmol, 1.1 eq) was dissolved under argon in 10 ml of dry MeCN. TEA (0.10 g, 0.14 ml, 1.00 mmol, 2.0 eq) was added dropwise followed by addition of 4-[5-(2-chlorophenyl)-1,2,4-oxadiazol-3-yl]-3-methoxyaniline (Example 4, Step 3b) (0.15 g, 0.50 mmol, 1.0 eq) in 5 ml of MeCN. Reaction mixture was stirred in rt for 24 hours and another portion of BOP (0.22 g, 0.50 mmol, 1.0 eq) and acid (0.08 g, 0.55 mmol, 1.1 eq) was added (LCMS control). Stirring was continued for additional 12h. Reaction mixture was then quenched with 50 ml of water and extracted with chloroform. Organic layer was washed with saturated sodium bicarbonate and dried. Removal of solvent gave crude product which was purified by column chromatography over aluminum oxide using gradient from AcOEt/hexane (1:2) to AcOEt/hexane (2:1) followed by maceration from i-PrOH/hexane (1:2) to yield product as a white solid (0.10 g, 47.6%).
LCMS (method A) RT = 3.60 min, MS (m/z ESI+) 424.6.
¹H NMR (300 MHz, CDCl₃): 10.56 (s br. 1H), 8.25 (m, 1H), 8.12 (m, 2H), 7.94 (d, 1H), 7.87 (d, 1H), 7.78-7.66 (m, 3H), 7.62 (m, 1H), 7.48 (m, 1H), 3.93 (s, 3H).
¹³C NMR (300 MHz, CDCl₃): 173.0, 170.6, 166.7, 162.2, 162.1 (d), 158.7, 148.9 (d), 144.4 (d), 142.8, 134.4, 132.8, 132.5, 131.6 (d), 128.3, 123.5, 121.2 (d), 113.8 (d), 112.8, 111.2, 105.1.

### Example 33

### 5-chloro-N-{4-[5-(2-chlorophenyl)-1,2,4-oxadiazol-3-yl]-3-methoxyphenyl}-3-fluoropyridine-2-carboxamide

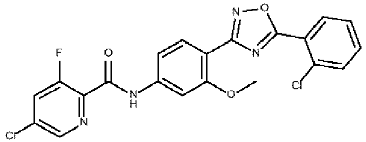

### 5-chloro-N-{4-[5-(2-chlorophenyl)-1,2,4-oxadiazol-3-yl]-3-methoxyphenyl}-3-fluoropyridine-2-carboxamide (Scheme I, Step 4-2):

Compound prepared according to procedure described in Example 32 from 4-[5-(2-chlorophenyl)-1,2,4-oxadiazol-3-yl]-3-methoxyaniline (Intermediate 4, Step 3b) and 5-chloro-3-fluoropyridine -2-carboxylic acid. Crude product was purified by column chromatography over silica gel using gradient from AcOEt/hexane (1:2) to AcOEt/hexane (2:1) followed by maceration from i-PrOH/hexane (1:2) to yield product as a white solid (0.16 g, 70.2%).
LCMS (method A) RT = 3.65 min, MS (m/z ESI+) 258.7.
¹H NMR (300 MHz, DMSO-d₆): 10.87 (s br. 1H), 8.66 (m, 1H), 8.33 (dd, 1H), 8.13 (dd, 1H), 7.96 (d, 1H), 7.81 (d, 1H), 7.76-7.57 (m, 4H), 3.90 (s, 3H). ¹³C NMR (300 MHz, DMSO-d₆): 172.9, 166.6, 161.1 (d), 158.6, 158.0 (d), 143.9 (d), 142.9, 138.1 (d), 134.7 (d), 132.8, 132.5, 131.8, 131.7, 128.4, 127.0 (d), 123.3, 112.2, 110.8 (d), 104.1, 56.3.

### Example 34

### 6-chloro-N-{4-[5-(2-chlorophenyl)-1,2,4-oxadiazol-3-yl]-3-methoxyphenyl}pyridine-2-carboxamide

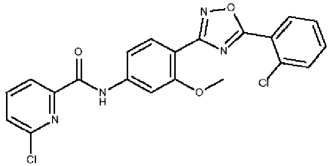

6-chloro-N-{4-[5-(2-chlorophenyl)-1,2,4-oxadiazol-3-yl]-3-methoxyphenyl}-pyridine-2-carboxamide (Scheme I, Step 4-2): Compound prepared according to procedure described in Example 32 from 4-[5-(2-chlorophenyl)-1,2,4-oxadiazol-3-yl]-3-methoxyaniline (Intermediate 4, Step 3b) and 6-chloropyridine-2-carboxylic acid. Solid that was precipitated from reaction mixture was filtered and washed with MeCN. Filtrate was concentrated and rest of the product was isolated by column chromatography over silica gel using CHCl₃/MeOH (1:1). Product after chromatography was combined with precipitated solid and further purified by maceration from i-PrOH/hexane/CHCl₃ (1:2:0.5) to yield pure product as a white solid (0.094 g, 42.9%).
LCMS (method A) RT=3.76 min, MS (m/z ESI+) 440.8.
¹H NMR (300 MHz, CDCl₃): 9.86 (s br. 1H), 8.25-8.12 (m, 3H), 7.91 (m, 2H), 7.60-7.41 (m, 4H), 7.30 (dd, 1H), 4.09 (s, 3H).
¹³C NMR (300 MHz, CDCl₃): 172.9, 166.8, 160.8, 159.1, 150.1, 150.0, 141.2, 140.4, 133.9, 133.0, 132.0, 131.9, 131.4, 127.6, 127.0, 123.8, 121.2, 11.8, 111.6, 103.1, 56.2.

### Example 35 (Reference example)

### 3-chloro-N-{4-[5-(2-chlorophenyl)-1,2,4-oxadiazol-3-yl]-3-methoxyphenyl}-6-methoxypyridine 2-carboxamide

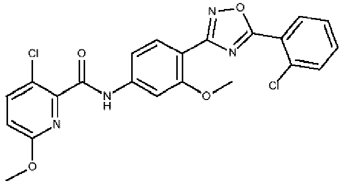

### 3-chloro-N-{4-[5-(2-chlorophenyl)-1,2,4-oxadiazol-3-yl]-3-methoxyphenyl}-6-methoxypyridine-2-carboxamide (Scheme I, Step 4-1a):

Compound prepared according to procedure described in Intermediate 1 from 4-[5-(2-chlorophenyl)-1,2,4-oxadiazol-3-yl]-3-methoxyaniline (Intermediate 4, Step 3b) and 3-chloro-6-methoxypyridine-2-carbonyl chloride. Crude product was purified by column chromatography over silica gel using CHCl₃ followed by maceration from i-PrOH/hexane (1:2) to yield product as a white solid (0.24 g, 100.0%).
LCMS (method A) RT = 3.74 min, MS (m/z ESI+) 470.4.
¹H NMR (300 MHz, CDCl₃): 9.89 (s br. 1H), 8.15-8.07 (m, 3H), 7.72 (d, 1H), 7.56 (dd, 1H), 7.50 (dt, 1H), 7.43 (dt, 1H), 7.00 (dd, 1H), 6.92 (d, 1H), 4.09 (s, 3H), 4.05 (s, 3H).
¹³C NMR (300 MHz, CDCl₃): 172.9, 166.8, 160.9, 160.7, 159.2, 143.6, 141.5, 141.1, 133.8, 132.9, 131.9, 131.7, 131.3, 127.0, 125.1, 123.7, 116.0, 111.4, 111.2, 102.8, 56.2, 54.0.

### Example 36 (Reference example)

### N-{4-[5-(2-chlorophenyl)-1,2,4-oxadiazol-3-yl]-3-methoxyphenyl}-4,6-difluoropyridine-2-carboxamide

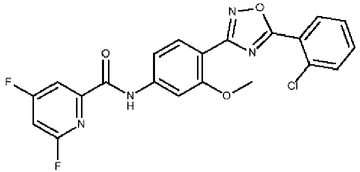

N-{4-[5-(2-chlorophenyl)-1,2,4-oxadiazol-3-yl]-3-methoxyphenyl}-4,6-difluoropyridine-2-carboxamide (Scheme I, Step 4-2): Compound was prepared according to procedure described in Example 32 from 4-[5-(2-chlorophenyl)-1,2,4-oxadiazol-3-yl]-3-methoxyaniline (Intermediate 4, Step 3b) and 3,5-difluoropyridine-2-carboxylic acid. Product was isolated from reaction mixture by column chromatography over silica gel using CHCl₃ and further purified by maceration from i-PrOH/hexane (1:2) to yield pure product as a white solid (0.220 g, 100.0%).
LCMS (method A) RT = 3.53 min, MS (m/z ESI+) 442.4.
¹H NMR (300 MHz, CDCl₃): 9.82 (s br. 1H), 8.37 (d, 1H), 8.16 (d, 1H), 8.13 (dd, 1H), 8.01 (d, 1H), 7.58 (dd, 1H), 7.48 (dt, 1H), 7.46-7.38 (m, 2H), 7.15 (dd, 1H), 4.08 (s, 3H).
¹³C NMR (300 MHz, CDCl₃): 172.9, 166.8, 162.5 (q), 159.2, 159.1, 158.7 (q), 141.3, 133.9, 133.7 (t), 133.0, 132.7 (q), 131.9 (2C), 131.4, 127.0, 123.7, 114.4 (q), 111.6, 111.2, 102.9, 56.2.

### Example 37 (Reference example)

### N-{4-[5-(2-chlorophenyl)-1,2,4-oxadiazol-3-yl]-3-methoxyphenyl}-3-fluoropyridine-2-carboxamide

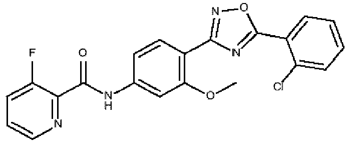

N-{4-[5-(2-chlorophenyl)-1,2,4-oxadiazol-3-yl]-3-methoxyphenyl}-3-fluoropyridine-2-carboxamide (Scheme I, Step 4-2): Compound was prepared according to procedure described in Example 32 from 4-[5-(2-chlorophenyl)-1,2,4-oxadiazol-3-yl]-3-methoxyaniline (Intermediate 4, Step 3b) and 3-fluoropyridine-2-carboxylic acid. Precipitated solid was filtered from reaction mixture and washed with MeCN followed by maceration from i-PrOH/hexane (1:2) to yield pure product as a white solid (0.210 g, 100.0%). LCMS (method A) RT=3.40 min, MS (m/z ESI+) 424.5.
¹H NMR (300 MHz, CDCl₃): 10.07 (s br. 1H), 8.45 (m, 1H), 8.16 (d, 1H), 8.13 (dd, 1H), 8.04 (d, 1H), 7.66-7.39 (m, 5H), 7.16 (dd, 1H), 4.07 (s, 3H). ¹³C NMR (300 MHz, CDCl₃): 172.9, 166.8, 161.0 (d), 159.1, 159.0 (d), 143.7 (d), 141.4, 136.8 (d), 133.9, 133.0, 131.9, 131.8, 131.4, 128.8 (d), 127.0, 126.8, 123.8, 111.4, 111.3, 102.9, 56.2.

### Example 38

### N-{4-[5-(2-chlorophenyl)-1,2,4-oxadiazol-3-yl]-3-methoxyphenyl} pyrimidine-4-carboxamide

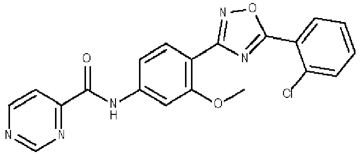

N-{4-[5-(2-chlorophenyl)-1,2,4-oxadiazol-3-yl]-3-methoxyphenyl} pyrimidine-4-carboxamide (Scheme I, Step 4-2): Compound was synthesized according to procedure described in Example 32 from 4-[5-(2-chlorophenyl)-1,2,4-oxadiazol-3-yl]-3-methoxyaniline (Intermediate 4, Step 3b) and pyrimidine-4-carboxylic acid. Precipitated solid was filtered from reaction mixture and purified by column chromatography over silica gel using CHCl₃/hexane (4:1) followed by maceration from i-PrOH/hexane (1:2) to yield pure product as a white solid (0.140 g, 70.0%).
LCMS (method B) RT = 3.23 min, MS (m/z ESI+) 408.1.
¹H NMR (300 MHz, DMSO-d₆): 11.07 (s, 1H), 9.44 (s br. 1H), 9.15 (d, 1H), 8.14 (m, 2H), 7.96 (m, 2H), 7.82-7.67 (m, 3H), 7.60 (m, 1H), 3.91 (s, 3H). ¹³C NMR (300 MHz, DMSO-d₆): 172.9, 166.6, 162.3, 160.5, 158.6, 158.3, 156.8, 142.6, 134.5, 132.8, 132.6, 131.8 (2C), 128.4, 123.3, 119.5, 112.7, 111.1, 104.8, 56.3.

### Example 39

### N-{4-[5-(2-chlorophenyl)-1,2,4-oxadiazol-3-yl]-3-methoxyphenyl}thiophene-2-carboxamide

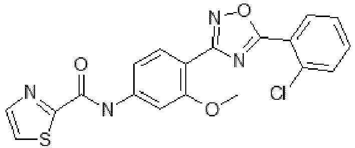

N-{4-[5-(2-chlorophenyl)-1,2,4-oxadiazol-3-yl]-3-methoxyphenyl}thiophene-2-carboxamide (Scheme I, Step 4-2): Compound was synthesized according to procedure described in Example 32 from 4-[5-(2-chlorophenyl)-1,2,4-oxadiazol-3-yl]-3-methoxyaniline (Intermediate 4, Step 3b) and 1,3-thiazole-2-carboxylic acid. Crude product isolated from reaction mixture was purified by column chromatography over silica gel using CHCl₃/hexane (4:1) and further purified by maceration from i-PrOH/hexane (1:2) to yield pure product as a white solid (0.190 g, 92.7%).
LCMS (method A) RT = 3.49 min, MS (m/z ESI+) 413.0.
¹H NMR (300 MHz, CDCl₃): 9.28 (s br. 1H), 8.17 (d, 1H), 8.13 (dd, 1H), 7.93 (d, 1H), 7.87 (d, 1H), 7.67 (d, 1H), 7.58 (dd, 1H), 7.50 (dt, 1H), 7.43 (dt, 1H), 7.20 (dd, 1H), 4.06 (s, 3H).
¹³C NMR (300 MHz, CDCl₃): 172.9, 166.7, 163.4, 159.1, 157.4, 143.6, 140.9, 133.9, 133.0, 132.1, 131.9, 131.4, 127.0, 125.8, 123.7, 111.9, 111.4, 103.0, 56.2.

### Example 40

### 6-fluoro-N-{4-[5-(2-fluorophenyl)-1,2,4-oxadiazol-3-yl]-3-methoxyphenyl} pyridine-2-carboxamide

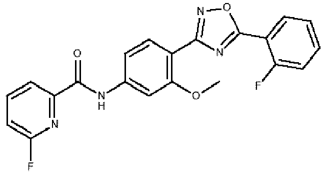

6-fluoro-N-{4-[5-(2-fluorophenyl)-1,2,4-oxadiazol-3-yl]-3-methoxyphenyl} pyridine-2-carboxamide (Scheme I, Step 4-2): Compound was synthesized according to procedure described in Example 32 from 4-[5-(2-fluorophenyl)-1,2,4-oxadiazol-3-yl]-3-methoxyaniline (Intermediate 5, Step 3b) and 6-fluoro-pyridine-2-carboxylic acid. Precipitated solid was filtered from reaction mixture and washed with MeCN. Filtrate was concentrated and rest of the product was isolated by column chromatography over silica gel using AcOEt/hexane (1:1). Product after chromatography was combined with precipitated solid and further purified by maceration from i-PrOH/hexane (1:2) to yield pure product as a white solid (0.168 g, 78.1%).
LCMS (method A) RT = 3.43 min, MS (m/z ESI+) 408.5.
¹H NMR (300 MHz, CDCl₃): 9.73 (s br. 1H), 8.20 (m, 1H), 8.15 (d, 1H), 8.03 (q, 1H), 7.89 (d, 1H), 7.57 (m, 1H), 7.35-7.26 (m, 3H), 7.17 (m, 1H), 4.07 (s, 3H). ¹³C NMR (300 MHz, CDCl₃): 171.4, 165.2 (d), 161.6 (d), 160.3, 159.0, 148.0 (d), 143.0 (d), 141.1, 134.4 (d), 132.0, 130.9, 124.6 (d), 120.1 (d), 117.1 (d), 113.5, 113.1, 112.9 (d), 111.8, 111.5, 103.4, 56.2.

### Example 41

### 3,6-dichloro-N-{4-[5-(2-fluorophenyl)-1,2,4-oxadiazol-3-yl]-3-methoxy phenyl}pyridine-2-carboxamide

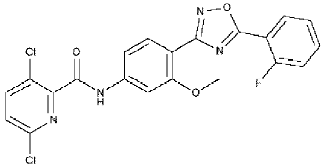

3,6-dichloro-N-{4-[5-(2-fluorophenyl)-1,2,4-oxadiazol-3-yl]-3-methoxyphenyl}pyridine-2-carboxamide (Scheme I, Step 4-1a): Compound prepared according to procedure described in Intermediate 1 from 4-[5-(2-fluorophenyl)-1,2,4-oxadiazol-3-yl]-3-methoxyaniline (Intermediate 5, Step 3b) and 3,6-dichloropyridine-2-carbonyl chloride. Crude product was purified by column chromatography over silica gel using CHCl₃ followed by maceration from i-PrOH/hexane (1:2) to yield product as a white solid (0.18 g, 75.0%).
LCMS (method A) RT = 3.52 min, MS (m/z ESI+) 458.7.
¹H NMR (300 MHz, CDCl₃): 9.80 (s br. 1H), 8.20 (m, 1H), 8.14 (d, 1H), 7.93 (d, 1H), 7.84 (d, 1H), 7.59 (m, 1H), 7.45 (d, 1H), 7.35-7.23 (m, 2H), 7.21 (dd, 1H), 4.07 (s, 3H).
¹³C NMR (300 MHz, CDCl₃): 171.4, 166.8, 162.4, 160.7 (d), 159.6, 159.1, 147.6, 144.9, 143.6, 141.1, 134.4 (d), 131.9, 131.6, 130.9 (d), 128.0, 124.6 (d), 117.0 (d), 112.9 (d), 111.9, 111.6, 103.1, 56.3.

### Example 42

### N-13-chloro-4-[5-(2-chlorophenyl)-1,2,4-oxadiazol-3-yl]phenyl)-6-fluoropyridine-2-carboxamide

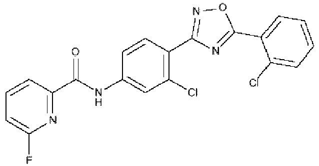

N-{3-chloro-4-[5-(2-chlorophenyl)-1,2,4-oxadiazol-3-yl]phenyl}-6-fluoropyridine-2-carboxamide (Scheme I, Step 4-2): Compound was synthesized according to procedure described in Example 32 from 3-chloro-4-[5-(2-chlorophenyl)-1,2,4-oxadiazol-3-yl]aniline (Intermediate 2, Step 3b) and 6-fluoro-pyridine-2-carboxylic acid. Precipitated solid was filtered from reaction mixture, washed with MeCN, dried and purified by maceration from i-PrOH/hexane (1:2) to yield product as a white solid (0.120 g, 85.7%). LCMS (method A) RT = 3.91 min, MS (m/z ESI+) 428.6.
¹H NMR (300 MHz, DMSO-d₆): 10.93 (s br. 1H), 8.35 (d, 1H), 8.25 (q, 1H), 8.19 (dd, 1H), 8.14-8.04 (m, 3H), 7.79-7.70 (m, 2H), 7.65 (dt, 1H), 7.53 (dd, 1H).
¹³C NMR (300 MHz, DMSO-d₆): 173.7, 167.0, 162.6, 162.1 (d), 148.4 (d), 144.5 (d), 142.2, 134.8, 132.8 (d), 132.6, 132.5, 131.8, 128.5, 122.9, 122.3, 121.5 (d), 120.6, 119.6, 114.0.

### Example 43 (Reference Example)

### 6-chloro-N-{3-chloro-4-[5-(2-chlorophenyl)-1,2,4-oxadiazol-3-yl]phenyl}pyridine-2-carboxamide

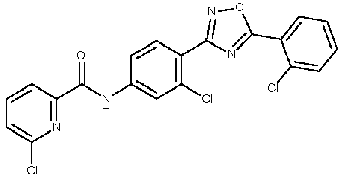

6-chloro-N-{3-chloro-4-[5-(2-chlorophenyl)-1,2,4-oxadiazol-3-yl]phenyl}pyridine-2-carboxamide (Scheme I, Step 4-2): Compound was synthesized according to procedure described in Example 32 from 3-chloro-4-[5-(2-chlorophenyl)-1,2,4-oxadiazol-3-yl]aniline (Intermediate 2, Step 3b) and 6-chloro-pyridine-2-carboxylic acid. Precipitated solid was filtered from reaction mixture, washed with MeCN, dried and purified by maceration from i-PrOH/hexane (1:2) to yield product as a white solid (0.113 g, 77.9%). LCMS (method A) RT = 4.07 min, MS (m/z ESI+) 445.0.
¹H NMR (300 MHz, CDCl₃): 9.86 (s br. 1H), 8.24 (d, 1H), 8.18-8.14 (m, 3H), 7.91 (t, 1H), 7.81 (dd, 1H), 7.59-7.45 (m, 4H).
¹³C NMR (300 MHz, CDCl₃): 173.7, 167.1, 160.8, 150.2, 149.6, 140.5, 140.2, 134.3, 133.9, 133.2, 132.5, 132.0, 131.5, 127.8, 127.1, 123.4, 121.7, 121.6, 121.4, 117.9.

### Example 44

### N-(2-chlorophenyl)-4-(5-phenyl-1,2,4-oxadiazol-3-yl)benzene-1-sulfonamide

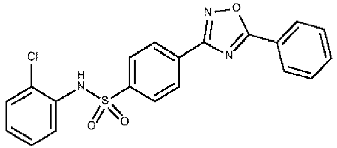

N-(2-chlorophenyl)-4-cyanobenzene-1-sulfonamide (Scheme III, Step 1): To a solution of 2-chloroaniline (1.90 g, 14.88 mmol, 1.2 eq) in pyridine (30 ml) 4-cyanobenzene-1-sulfonyl chloride (2.50 g, 12.40 mmol, 1.0 eq) was added in one portion. After stirring in RT overnight reaction mixture was poured into 100 ml of water. Precipitated solid was filtered off, dried and purified by maceration in i-PrOH/hexane (1:2) to yield product as a white solid (2.43 g, 66.9%).
LCMS (method A) RT = 2.91 min, MS (m/z ESI+) 292.0.

4-[(2-chlorophenyl)sulfamoyl]-N'-hydroxybenzene-1-carboximidamide (Scheme III, Step 2): Compound was prepared from N-(2-chlorophenyl)-4-cyanobenzene-1-sulfonamide according to procedure described in Example 1. After extraction and evaporation of solvent 20 ml of CHCl₃ was added and precipitated white crystals were filtered off and used directly in next steps (2.33 g, 86.3%).
LCMS (method A) RT = 1.76 min, MS (m/z ESI+) 325.9.
¹H NMR (300 MHz, DMSO-d₆): 10.04 (s, 1H), 9.94 (s, 1H), 7.81 (d, 2H), 7.68 (d, 2H), 7.39 (dd, 1H), 7.27-7.18 (m, 3H), 5.95 (s br. 2H).
¹³C NMR (300 MHz, DMSO-d₆): 150.1, 140.7, 137.8, 133.9, 130.4, 129.6, 128.2, 128.1, 127.9, 127.0, 126.3.

N-(2-chlorophenyl)-4-(5-phenyl-1,2,4-oxadiazol-3-yl)benzene-1-sulfonamide (Scheme III, Step 3a): Compound synthesized according to procedure described in Example 1 from 4-[(2-chlorophenyl)sulfamoyl]-N'-hydroxybenzene-1-carboximidamide and benzoyl chloride. Crude product was purified by column chromatography over aluminum oxide using CHCl₃/hexane (3:1) followed by maceration from i-PrOH/hexane (1:3) to yield product as a white solid (0.16 g, 50.6%).
LCMS (method A) RT = 3.71 min, MS (m/z ESI+) 411.3.
¹H NMR (300 MHz, CDCl₃): 8.25-8.18 (m, 4H), 7.88 (m, 2H), 7.71 (dd, 1H), 7.67-7.54 (m, 3H), 7.26 (m, 2H), 7.30-7.23 (m, 1H), 7.10-7.03 (s br. 1H). ¹³C NMR (300 MHz, CDCl₃): 176.3, 167.6, 141.0, 133.1, 132.9, 131.6, 129.5, 129.2, 128.2, 128.1, 128.0, 127.8, 126.5, 125.7, 123.8, 123.2.

### Example 45 (Reference Example)

### N-(2-chlorophenyl)-4-[5-(pyridin-2-yl)-1,2,4-oxadiazol-3-yl]benzene-1-sulfonamide

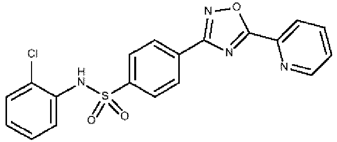

N-(2-chlorophenyl)-4-[5-(pyridin-2-yl)-1,2,4-oxadiazol-3-yl]benzene-1-sulfonamide (Scheme III, Step 3a): Compound was prepared according to procedure described in Example 1 from 4-[(2-chlorophenyl)sulfamoyl]-N'-hydroxybenzene-1-carboximidamide (Example 44, Step 2) and picolinoyl chloride, hydrochloride. Crude product was purified by column chromatography over silica gel using CHCl₃/MeOH (99:1) and then over aluminum oxide using CHCl₃/hexane (3:1) followed by maceration from i-PrOH/hexane (1:3) to yield product as a white solid (0.15 g, 47.4%).
LCMS (method A) RT=3.15 min, MS (m/z ESI+) 412.3.
¹H NMR (300 MHz, DMSO-d₆): 10.26 (s, 1H), 8.86 (m, 1H), 8.33 (m, 1H), 8.28 (d, 2H), 8.14 (dt, 1H), 7.93 (d, 2H), 7.75 (ddd, 1H), 7.41 (dd, 1H), 7.33-7.24 (m, 3H).
¹³C NMR (300 MHz, DMSO-d₆): 174.7, 167.3, 150.6, 142.9, 142.5, 138.1, 133.0, 129.9, 129.8, 129.6, 128.1, 128.0, 127.9, 127.8, 127.6, 127.5, 124.5.

### Example 46

### N-(2,4-difluorophenyl)-4-[5-(pyridin-2-yl)-1,2,4-oxadiazol-3-yl]benzene-1-sulfonamide

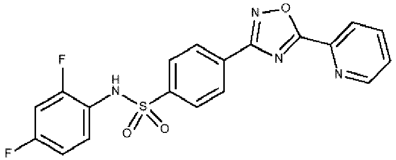

### 4-cyano-N-(2,4-difluorophenyl)benzene-1-sulfonamide (Scheme III, Step1):

Prepared according to procedure described in Example 44 from 4-cyanobenzene-1-sulfonyl chloride and 2,4-difluoroaniline. Reaction mixture was poured into water and precipitated solid was filtered off and dried resulting pale yellow solid (1.46 g, 80.2%).
LCMS (method A) RT = 2.97 min, MS (m/z ESI+) 294.7.

### 4-[(2,4-difluorophenyl)sulfamoyl]-N'-hydroxybenzene-1-carboximidamide

(Scheme III, Step 2): Compound was synthesized from 4-cyano-N-(2,4-difluorophenyl)benzene-1-sulfonamide according to procedure described in Intermediate 1. Crude product was purified by maceration from i-PrOH (0.40 g, 25%).
LCMS (method A) RT = 1.61 min, MS (m/z ESI+) 327.9.
¹H NMR (300 MHz, DMSO-d₆): 10.13 (s, 1H), 9.93 (s, 1H), 7.81 (d, 2H), 7.64 (d, 2H), 7.21 (m, 2H), 7.2 (m, 1H), 5.92 (d, 2H).
¹³C NMR (300 MHz, DMSO-d₆): 160.6 (q), 156.9 (q), 150.0 (q), 140.1, 137.9, 137.8, 129.3 (q), 126.9, 126.3, 121.0 (q), 112.2 (q), 105.1 (q).

N-(2,4-difluorophenyl)-4-[5-(pyridin-2-yl)-1,2,4-oxadiazol-3-yl]benzene-1-sulfonamide (Scheme III, Step 3b): Compound was prepared from 4-[(2,4-difluorophenyl)sulfamoyl]-N'-hydroxybenzene-1-carboximidamide and picolinoyl chloride, hydrochloride according to procedure described in Intermediate 1. Crude product was purified by column chromatography over silica gel using CHCl₃/MeOH (99:1) followed by maceration from i-PrOH/hexane (1:2) to yield product as a white solid (0.13 g, 52.0%).
LCMS (method A) RT = 3.05 min, MS (m/z ESI+) 414.2.
¹H NMR (300 MHz, CHCl₃ + MeOD): 8.76 (m, 1H), 8.24 (m, 1H), 8.20 (d, 2H), 7.93 (dt, 1H), 7.78 (d, 2H), 7.52 (ddd, 1H), 7.42 (dt, 1H), 6.77 (m, 1H), 6.63 (ddd, 1H).
¹³C NMR (300 MHz, CHCl₃ + MeOD): 174.7, 167.9, 160.6 (q), 155.8 (q), 150.6, 142.9, 141.7, 137.7, 130.7, 128.1, 127.7 (q), 127.5, 127.1, 124.4, 111.6 (q), 104.1 (q).

### Example 47

### N-[4-(3-phenyl-1,2,4-oxadiazol-5-yl)phenyl]benzamide

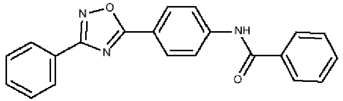

5-(4-nitrophenyl)-3-phenyl-1,2,4-oxadiazole (Scheme IV, Step 2): Prepared from N'-hydroxybenzene-1-carboximidamide and 4-nitrobenzoyl chloride according to procedure described in Example 1. Product was purified by maceration from MeOH to give white crystals (7.88 g, 80.3%).

4-(3-phenyl-1,2,4-oxadiazol-5-yl)aniline (Scheme IV, Step 3): 5-(4-nitrophenyl)-3-phenyl-1,2,4-oxadiazole (3.00 g, 11.24 mmol, 1.0 eq) was dissolved in 75 ml of 90% aqueous CH₃COOH and 15 ml of EtOH. 3.00 g of iron powder was added in one portion and reaction mixture was heated to reflux for 1.0 h. Solution was poured into cold water and precipitated solid was filtered, dissolved in 50 ml of acetone and again filtered. 25 ml of water was then added and pH was adjusted to 9 with NH₃aq. Solution was concentrated and precipitated solid was filtered off and further purified by flash column chromatography over silica gel using CHCl₃ followed by maceration from i-PrOH to give pure product as a white crystals (1.72 g, 64.7%).
LCMS (method B) RT = 3.15 min, MS (m/z ESI+) 237.9.

N-[4-(3-phenyl-1,2,4-oxadiazol-5-yl)phenyl]benzamide (Scheme IV, Step 4-1): Compound synthesized according to procedure described in Example 6 from 4-(3-phenyl-1,2,4-oxadiazol-5-yl)aniline and benzoyl chloride. Crude product was purified by maceration from MeOH/water (2:1) to yield product as a white solid (0.27 g, 93.8%).
LCMS (method B) RT = 3.71 min, MS (m/z ESI+) 342.1.
¹H NMR (300 MHz, DMSO-d₆): 10.68 (s br. 1H), 8.19 (m, 2H), 8.11 (m, 4H), 7.98 (d, 2H), 7.63-7.54 (m, 6H).
¹³C NMR (300 MHz, DMSO-d₆): 174.7, 167.7, 165.6, 143.3, 134.0, 131.5, 131.1, 128.8, 128.4, 128.0, 127.4, 126.6, 125.8, 119.8, 117.5.

### Example 48

### 3-chloro-N-[4-(3-phenyl-1,2,4-oxadiazol-5-yl)phenyl]benzamide

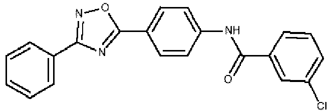

3-chloro-N-[4-(3-phenyl-1,2,4-oxadiazol-5-yl)phenyl]benzamide (Scheme IV, Step 4-1): Compound synthesized according to procedure described in Example 6 from 4-(3-phenyl-1,2,4-oxadiazol-5-yl)aniline (Example 46, Step 3) and 3-chlorobenzoyl chloride. Crude product was purified by maceration from MeOH/water (2:1) to yield product as a white solid (0.29 g, 91.5%). LCMS (method B) RT = 3.98 min, MS (m/z ESI+) 375.9.
¹H NMR (300 MHz, DMSO-d₆): 10.74 (s br. 1H), 8.19 (m, 2H), 8.11-8.03 (m, 5H), 7.95 (m, 1H), 7.69 (m, 1H), 7.59 (m, 4H).
¹³C NMR (300 MHz, DMSO-d₆): 175.1, 168.1, 164.5, 143.4, 136.4, 133.2, 131.7, 131.5, 130.4, 129.2, 128.8, 127.5, 127.0, 126.6, 126.2, 120.3, 118.2.

### Example 49

### 4-methoxy-N-[4-(3-phenyl-1,2,4-oxadiazol-5-yl)phenyl]benzamide

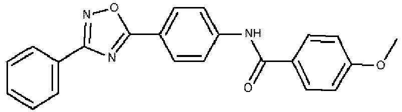

### 4-methoxy-N-[4-(3-phenyl-1,2,4-oxadiazol-5-yl)phenyl]benzamide

(Scheme IV, Step 4-1): Compound synthesized according to procedure described in Example 6 from 4-(3-phenyl-1,2,4-oxadiazol-5-yl)aniline (Example 46, Step 3) and 4-methoxybenzoyl chloride. Crude product was purified by maceration from MeOH to yield product as a white solid (0.30 g, 96.7%).
LCMS (method B) RT = 3.71 min, MS (m/z ESI+) 372.7.
¹H NMR (300 MHz, DMSO-d₆): 10.46 (s br. 1H), 8.13 (d, 2H), 8.04 (m, 4H), 7.96 (d, 2H), 7.57 (m, 3H), 7.04 (d, 2H).
¹³C NMR (300 MHz, DMSO-d₆): 175.1, 168.1, 165.3, 162.1, 143.9, 131.5, 129.8, 129.2, 128.8, 127.0, 126.3, 126.2, 120.1, 117.7, 113.6, 55.4.

### Example 50

### 4-nitro-N-[4-(3-phenyl-1,2,4-oxadiazol-5-yl)phenyl]benzamide

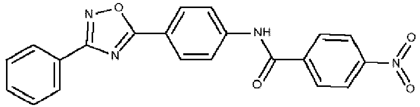

4-nitro-N-[4-(3-phenyl-1,2,4-oxadiazol-5-yl)phenyl]benzamide (Scheme IV, Step 4-1): Compound synthesized according to procedure described in Example 6 from 4-(3-phenyl-1,2,4-oxadiazol-5-yl)aniline (Example 46, Step 3) and 4-nitrobenzoyl chloride. Crude product was purified by maceration from MeOH/water (3:1) to yield product as a white solid (0.63 g, 96.9%). LCMS (method B) RT = 3.75 min, MS (m/z ESI+) 387.1.
¹H NMR (300 MHz, DMSO-d₆): 10.93 (s br. 1H), 8.38 (m, 2H), 8.21 (m, 4H), 8.07 (m, 4H), 7.60 (m, 3H).
¹³C NMR (300 MHz, DMSO-d₆): 175.0, 168.1, 164.3, 149.2, 143.1, 139.9, 131.5, 129.3, 129.2, 128.8, 127.0, 126.2, 123.5, 120.4, 118.4.

### Example 51 (Reference Example)

### 4-amino-N-[4-(3-phenyl-1,2,4-oxadiazol-5-yl)phenyl]benzamide

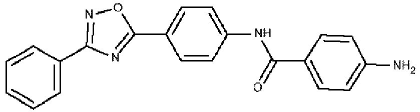

4-amino-N-[4-(3-phenyl-1,2,4-oxadiazol-5-yl)phenyl]benzamide (Scheme IV, reduction as in Step 2): To solution of 4-nitro-N-[4-(3-phenyl-1,2,4-oxadiazol-5-yl)phenyl]benzamide (Example 50, Step 4-1) (0.50 g, 1.30 mmol, 1.0 eq) in 15 ml of 90% aqueous CH₃COOH with 5 ml of EtOH and 0.50 g of iron powder was added in one portion. Reaction mixture was heated to 50° C for 2 hours and poured into 100 ml of water. Precipitated solid was filtered, dissolved in 50 ml of acetone and again filtered. 25 ml of water was then added and pH was adjusted to 9 with NH₃aq. Solution was concentrated and precipitated solid was filtered, washed with water and dried. Crude product was purified by maceration from i-PrOH to yield product as a white solid (0.34 g, 73.9%).
LCMS (method B) RT = 3.34 min, MS (m/z ESI+) 357.1.
¹H NMR (300 MHz, DMSO-d₆): 10.19 (s br. 1H), 8.17-8.06 (m, 6H), 7.77 (d, 2H), 7.61 (m, 3H), 6.61 (d, 2H), 5.87 (s br. 2H).
¹³C NMR (300 MHz, DMSO-d₆): 175.2, 168.1, 165.6, 152.5, 144.4, 131.5, 129.6, 129.2, 128.7, 127.0, 126.2, 120.3, 119.8, 117.1, 112.5.

### Example 52 (Reference Example)

### N-[4-(3-phenyl-1,2,4-oxadiazol-5-yl)phenyl]pyridine-3-carboxamide

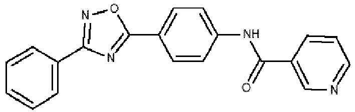

N-[4-(3-phenyl-1,2,4-oxadiazol-5-yl)phenyl]pyridine-3-carboxamide (Scheme IV, Step 4-1): Compound was prepared according to procedure described in Example 6 from 4-(3-phenyl-1,2,4-oxadiazol-5-yl)aniline (Example 46, Step 3) and nicotinoyl chloride, hydrochloride. Crude product was purified by maceration from i-PrOH/water (3:1) to yield product as a white solid (0.26 g, 90.0%).
LCMS (method B) RT = 3.17 min, MS (m/z ESI+) 342.9.
¹H NMR (300 MHz, DMSO-d₆): 10.84 (s br. 1H), 9.14 (d, 1H), 8.79 (dd, 1H), 8.33 (dt, 1H), 8.20 (d, 2H), 8.09 (m, 4H), 7.59 (m, 4H).
¹³C NMR (300 MHz, DMSO-d₆): 175.0, 168.1, 164.6, 152.3, 148.7, 143.3, 135.6, 131.5, 130.1, 129.2, 128.9, 127.0, 126.2, 123.5, 120.3, 118.3.

### Example 53 (Reference Example)

### N-[4-(3-phenyl-1,2,4-oxadiazol-5-yl)phenyl]pyridine-4-carboxamide

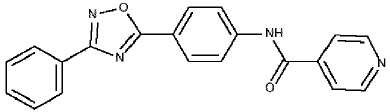

### N-[4-(3-phenyl-1,2,4-oxadiazol-5-yl)phenyl]pyridine-7-carboxamide

(Scheme IV, Step 4-1): Compound was prepared according to procedure described in Example 6 from 4-(3-phenyl-1,2,4-oxadiazol-5-yl)aniline (Example 46, Step 3) and isonicotinoyl chloride, hydrochloride. Crude product was purified by maceration from MeOH/water (2:1) to yield product as a white solid (0.26 g, 89.7%).
LCMS (method B) RT = 3.15 min, MS (m/z ESI+) 343.4.
¹H NMR (300 MHz, DMSO-d₆): 10.89 (s br. 1H), 8.82 (m, 2H), 8.20 (m, 2H), 8.08 (m, 4H), 7.88 (m, 2H), 7.60 (m, 3H).
¹³C NMR (300 MHz, DMSO-d₆): 175.0, 168.1, 164.5, 150.3, 143.0, 141.4, 131.6, 129.2, 128.9, 127.0, 126.2, 121.6, 120.4, 118.5.

### Example 54

### 1-methyl-N-[4-(3-phenyl-1,2,4-oxadiazol-5-yl)phenyl]-1H-pyrrole-2-carboxamide

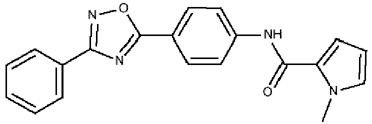

1-methyl-N-[4-(3-phenyl-1,2,4-oxadiazol-5-yl)phenyl]-1H-pyrrole-2-carboxamide (Scheme IV, Step 4-1): Compound was prepared according to procedure described in Example 6 from 4-(3-phenyl-1,2,4-oxadiazol-5-yl)aniline (Example 46, Step 3) and 1-methylpyrrole-2-carbonyl chloride. Crude product was purified by column chromatography over silica gel using CHCl₃/MeOH (49:1) followed by maceration from i-PrOH/water (3:1) to yield product as a white solid (0.22 g, 75.9%).
LCMS (method B) RT = 3.72 min, MS (m/z ESI+) 345.0.
¹H NMR (300 MHz, DMSO-d₆): 10.16 (s br. 1H), 8.16-8.02 (m, 6H), 7.60 (m, 3H), 7.13 (dd, 1H), 7.07 (dd, 1H), 6.13 (dd, 1H), 3.90 (s, 3H).
¹³C NMR (300 MHz, DMSO-d₆): 175.2, 168.1, 159.9, 144.1, 131.5, 129.6, 129.2, 128.7, 127.0, 126.2, 124.8, 119.8, 117.2, 114.6, 107.0, 36.4.

### Example 55 (Reference example)

### 5-methyl-N-[4-(3-phenyl-1,2,4-oxadiazol-5-yl)phenyl]-1,2-oxazole-3-carboxamide

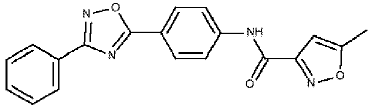

5-methyl-N-[4-(3-phenyl-1,2,4-oxadiazol-5-yl)phenyl]-1,2-oxazole-3-carboxamide (Scheme IV, Step 4-1): Compound was prepared according to procedure described in Example 6 from 4-(3-phenyl-1,2,4-oxadiazol-5-yl)aniline (Example 46, Step 3) and 5-methyloxazole-3-carbonyl chloride. Crude product was purified by maceration from i-PrOH/water (3:1) to yield product as a white solid (0.29 g, 99.3%).
LCMS (method B) RT = 3.68 min, MS (m/z ESI+) 347.0.
¹H NMR (300 MHz, DMSO-d₆): 11.03 (s br. 1H), 8.14 (d, 2H), 8.06 (m, 4H), 7.56 (m, 3H), 6.66 (s, 1H), 2.47 (s, 3H).
¹³C NMR (300 MHz, DMSO-d₆): 175.0, 171.6, 168.1, 158.9, 157.8, 142.5, 131.5, 129.2, 128.8, 127.0, 126.2, 120.5, 118.7, 101.7, 11.8.

### Example 56 (Reference example)

### N-{4-[3-(pyridin-2-yl)-1,2,4-oxadiazol-5-yl]phenyl}benzamide

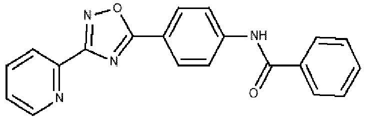

N'-hydroxypyridine-2-carboximidamide (Scheme IV, Step 1): Prepared from pyridine-2-carbonitrile according to procedure described in Intermediate 1. Pure product was precipitated as white crystals from reaction mixture after alkalization and was filtered off and dried (4.09 g, 62.2%).
LCMS (method B) RT = 0.47, MS (m/z ESI+) 138.2.

### 2-[5-(4-nitrophenyl)-1,2,4-oxadiazol-3-yl]pyridine (Scheme IV, Step 2):

Compound was prepared according to procedure described in Example 1 from N'-hydroxypyridine-2-carboximidamide and benzoyl chloride. Crude product was purified by maceration from i-PrOH/hexane (1:2) to give yellow crystals (2.94 g, 75.4%).
LCMS (method A) RT = 2.86 min, MS (m/z ESI+) 269.0.

4-[3-(pyridin-2-yl)-1,2,4-oxadiazol-5-yl]aniline (Scheme IV, Step 3b): 2-[5-(4-nitrophenyl)-1,2,4-oxadiazol-3-yl]pyridine (2.90 g, 10.81 mmol, 1.0 eq) was dissolved in 60 ml of MeOH and 30 ml of THF. Solution was heated to 50°C and 2 ml of hydrazin hydrate followed by portion of freshly prepared Ranney Ni was added. Reaction was stirred for 20 minutes and solvents were evaporated. Crude product was purified by column chromatography over silica gel using CHCl₃/MeOH (49:1) to give pale yellow solid (0.98 g, 38.8%) LCMS (method A) RT = 2.30 min, MS (m/z ESI+) 238.8.
¹H NMR (300 MHz, DMSO-d₆): 8.77 (m, 1H), 8.12 (m, 1H), 8.04 (dd, 1H), 7.85 (m, 2H), 7.60 (m, 1H), 6.71 (m, 2H), 6.23 (s br. 2H).
¹³C NMR (300 MHz, DMSO-d₆): 176.7, 168.3, 154.2, 150.6, 146.7, 138.0, 130.2, 126.3, 123.7, 113.9, 109.8.

N-{4-[3-(pyridin-2-yl)-1,2,4-oxadiazol-5-yl]phenyl}benzamide (Scheme IV, Step 4-1): Compound was prepared according to procedure described in Example 6 from 4-[3-(pyridin-2-yl)-1,2,4-oxadiazol-5-yl]aniline and benzoyl chloride. Crude product was purified by maceration from i-PrOH/hexane (1:2) to yield product as a white solid (0.19 g, 88.4%).
LCMS (method B) RT = 2.96 min, MS (m/z ESI+) 343.2.
¹H NMR (300 MHz, CDCl₃): 8.81 (d, 1H), 8.26 (d, 2H), 8.21 (d, 2H), 7.87 (m, 5H), 7.57-7.44 (m, 4H).
¹³C NMR (300 MHz, CDCl₃): 176.0, 168.7, 165.9, 150.4, 146.4, 142.3, 137.1, 134.4, 132.3, 129.6, 128.9, 127.1, 125.5, 123.3, 119.9, 119.6.

### Example 57 (Reference example)

### 2-chloro-N-{4-[3-(pyridin-2-yl)-1,2,4-oxadiazol-5-yl]phenyl}benzamide

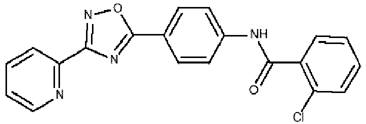

### 2-ch loro- N-{ 4- [3- (pyridin-2-yl) -1,2,4-oxadiazol-5-yl] phenyl}benzamide

(Scheme IV, Step 4-1): Compound was prepared according to procedure described in Example 6 from 4-[3-(pyridin-2-yl)-1,2,4-oxadiazol-5-yl]aniline (Example 56, Step 3) and 2-chlorobenzoyl chloride. Crude product was purified by column chromatography over silica gel using CHCl₃/MeOH (49:1) followed by maceration from i-PrOH/hexane (1:2) to yield product as a white solid (0.20 g, 87.0%).
LCMS (method B) RT = 3.03 min, MS (m/z ESI+) 376.8.
¹H NMR (300 MHz, CDCl₃): 8.85 (m, 1H), 8.28 (d, 2H), 8.22 (d, 1H), 8.20 (s br. 1H), 7.89 (m, 3H), 7.80 (m, 1H), 7.48-7.40 (m, 4H).

### Example 58 (Reference example)

### 2-fluoro-N-{4-[3-(pyridin-2-yl)-1,2,4-oxadiazol-5-yl]phenyl}benzamide

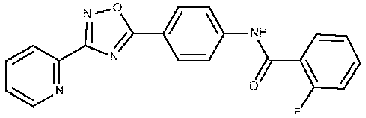

### 2-fluoro-N-{4-[3-(pyridin-2-yl)-1,2,4-oxadiazol-5-yl]phenyl}benzamide

(Scheme IV, Step 4-1): Compound was prepared according to procedure described in Example 6 from 4-[3-(pyridin-2-yl)-1,2,4-oxadiazol-5-yl]aniline (Example 56, Step 3) and 2-fluorobenzoyl chloride. Crude product was purified by column chromatography over silica gel using CHCl₃/MeOH (49:1) followed by maceration from i-PrOH/hexane (1:2) to yield product as a white solid (0.22 g, 96.9%).
LCMS (method B) RT = 2.99 min, MS (m/z ESI+) 360.8.
¹H NMR (300 MHz, CDCl₃): 8.84 (m, 1H), 8.70 (d, 1H), 8.28 (m, 2H), 8.20 (m, 2H), 7.90 (m, 3H), 7.55 (m, 1H), 7.44 (m, 1H), 7.34 (dt, 1H), 7.20 (m, 1H). ¹³C NMR (300 MHz, CDCl₃): 176.0, 168.7, 161.7 (d), 160.1 (d), 150.4, 146.5, 141.9, 137.0, 134.3 (d), 132.3 (d), 129.6, 125.5, 125.3 (d), 123.2, 120.7 (d), 120.3, 119.9, 116.3 (d).

### Example 59 (Reference example)

### 2-(methylsulfanyl)-N-{4-[3-(pyridin-2-yl)-1,2,4-oxadiazol-5-yl]phenyl) pyridine-3-carboxamide

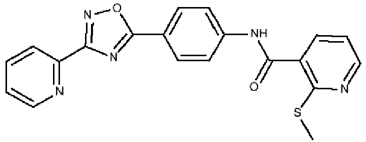

2-(methylsulfanyl)-N-{4-[3-(pyridin-2-yl)-1,2,4-oxadiazol-5-yl]phenyl} pyridine-3-carboxamide (Scheme IV, Step 4-1): Compound was prepared according to procedure described in Example 6 from 4-[3-(pyridin-2-yl)-1,2,4-oxadiazol-5-yl]aniline (Example 56, Step 3) and 2-(methylthio)nicotinoyl chloride. Crude product was purified by maceration from i-PrOH/hexane (1:2) to yield product as a white solid (0.21 g, 85.7%). LCMS (method B) RT = 2.91 min, MS (m/z ESI+) 389.8.
¹H NMR (300 MHz, CDCl₃): 8.80 (m, 1H), 8.71 (s br. 1H), 8.52 (dd, 2H), 8.25 (m, 2H), 8.20 (m, 1H), 7.94 (dd, 1H), 7.87 (m, 3H), 7.44 (m, 1H), 7.07 (dd, 1H), 2.61 (s, 3H).
¹³C NMR (300 MHz, CDCl₃): 175.9, 168.7, 164.8, 157.3, 151.1, 150.3, 146.4, 141.8, 137.1, 136.7, 129.6, 129.1, 125.5, 123.3, 120.0, 119.9, 118.9, 13.8.

### Example 60 (Reference example)

### N-(3-chlorophenyl)-4-(3-phenyl-1,2,4-oxadiazol-5-yl)aniline

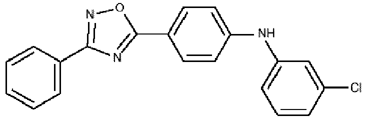

N-(3-chlorophenyl)-4-(3-phenyl-1,2,4-oxadiazol-5-yl)aniline (Scheme IV, Step 4-2): Compound prepared according to procedure described in Example 1 from 4-(3-phenyl-1,2,4-oxadiazol-5-yl)aniline (Example 46, Step 3) and 3-chlorobenzeneboronic acid. Crude product was purified by column chromatography over silica gel using AcOEt/hexane (2:3) followed by maceration from MeOH to yield product as a pale yellow crystals (0.20g, 69.0%).
LCMS (method B) RT = 4.27 min, MS (m/z ESI+) 348.1.
1H NMR (300 MHz, DMSO-d₆): 9.11 (s br. 1H), 8.09-8.03 (m, 4H), 7.60 (m, 3H), 7.35 (t, 1H), 7.23 (m, 4H), 7.03 (m, 1H).
¹³C NMR (300 MHz, DMSO-d₆): 175.3, 167.9, 147.5, 142.8, 133.7, 131.4, 130.9, 129.7, 129.1, 127.0, 126.4, 121.3, 118.2, 117.2, 115.5, 113.7.

### Example 61 (Reference example)

### N-(4-methoxyphenyl)-4-(3-phenyl-1,2,4-oxadiazol-5-yl)aniline

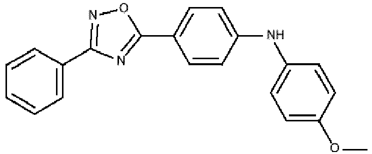

N-(4-methoxyphenyl)-4-(3-phenyl-1,2,4-oxadiazol-5-yl)aniline (Scheme IV, Step 4-2): Compound prepared according to procedure described in Example 1 from 4-(3-phenyl-1,2,4-oxadiazol-5-yl)aniline (Example 46, Step 3) and 4-methoxybenzeneboronic acid. Crude product was purified by column chromatography over silica gel using AcOEt/hexane (2:3) followed by maceration from MeOH/Et₂O (1:1) to yield product as a dark yellow solid (0.18 g, 62.1%).
LCMS (method B) RT = 3.98 min, MS (m/z ESI+) 344.1.
¹H NMR (300 MHz, DMSO): 8.72 (s br. 1H), 8.06 (m, 2H), 7.94 (d, 2H), 7.57 (m, 3H), 7.16 (m, 2H), 7.00 (d, 2H), 6.95 (m, 2H), 3.75 (s, 3H).
¹³C NMR (300 MHz, DMSO): 175.6, 167.8, 155.4, 150.1, 133.4, 131.3, 129.7, 129.1, 126.9, 126.5, 123.1, 114.6, 113.3, 111.5, 55.2.

### Example 62 (Reference example)

### 1-N,1-N-dimethyl-4-N-[4-(3-phenyl-1,2,4-oxadiazol-5-yl)phenyl]benzene-1,4-diamine

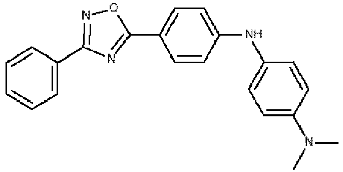

1-N,1-N-dimethyl-4-N-[4-(3-phenyl-1,2,4-oxadiazol-5-yl)phenyl]benzene-1,4-diamine (Scheme IV, Step 4-2): Compound prepared according to procedure described in Example 1 from 4-(3-phenyl-1,2,4-oxadiazol-5-yl)aniline (Example 46, Step 3) and 4-dimethylaminobenzeneboronic acid. Crude product was purified by column chromatography over aluminum oxide using AcOEt/hexane (1:1) to yield 0.04 g (13.3%) of white solid.
LCMS (method B) RT = 3.08 min, MS (m/z ESI+) 357.2.
¹H NMR (300 MHz, CDCl₃): 8.15 (m, 2H), 8.00 (d, 2H), 7.50 (m, 3H), 7.14 (d, 2H), 6.87 (d, 2H), 6.77 (d, 2H), 5.86 (s br. 1H), 2.97 (s, 6H).
¹³C NMR (300 MHz, CDCl₃): 176.0, 168.6, 150.5, 148.4, 130.9, 129.9, 129.2, 128.7, 127.5, 127.4, 125.3, 113.4 (2C), 40.9.

### Industrial applicability

### PHARMACEUTICAL COMPOSITIONS

Herein are disclosed pharmaceutical compositions comprising a pharmaceutically acceptable carrier or diluent and, as active ingredient, a therapeutically effective amount of a compound according to the invention, presented with general Formula 1 or pharmaceutically acceptable salts, hydrates or solvates thereof.

The compounds of Formula 1 according to the invention, the pharmaceutically acceptable salts, hydrates or solvates thereof, or combination thereof may be formulated into various pharmaceutical forms for administration purposes. As appropriate compositions there may be cited all compositions usually employed for systemically administering drugs.

The amount of pharmaceutical compositions administered to the subject will depend on the type and severity of the disease or condition and on the characteristics of the subject, such as general health, age, sex, body weight, diet and tolerance to drugs. The skilled artisan will be able to determine appropriate dosages depending on these and other factors. Effective dosages for commonly used CNS drugs are well known to the skilled person. The total daily dose usually ranges from about 0.05-2000 mg.

Herein are disclosed pharmaceutical compositions which provide from about 0.01 to 1000 mg of the active ingredient per unit dose. The compositions may be administered by any suitable route. For example orally in the form of capsules and the like, parenterally in the form of solutions for injection, topically in the form of ointments, or lotions, ocularly in the form of eye-drops, rectally in the form of suppositories, intranasally or transcutaneously in the form of delivery system like patches.

To prepare the pharmaceutical compositions an effective amount of the particular compound, optionally pharmaceutically acceptable salts, hydrates or solvates thereof, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, particularly, for administration orally, rectally, percutaneously, by parenteral injection or by inhalation. For example, in preparing the compositions in oral dosage form, any of the usual pharmaceutical media may be employed such as, for example, Water, glycols, oils, alcohols and the like in the case of oral liquid preparations such as, for example, suspensions, syrups, elixirs, emulsions and solutions; or solid carriers such as, for example, starches, sugars, kaolin, diluents, lubricants, binders, disintegrating agents and the like in the case of powders, pills, capsules and tablets. Because of their ease in administration, tablets and capsules represent the most advantageous oral dosage unit forms in Which case solid pharmaceutical carriers are obviously employed.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association With the required pharmaceutical carrier. Examples of such unit dosage forms are tablets (including scored or coated tablets), capsules, pills, powder packets, wafers, suppositories, injectable solutions or suspensions and the like, and segregated multiples thereof. The quantity of active ingredient in a unit dose of composition is an effective amount and may be varied according to the particular treatment involved. It may be appreciated that it may be necessary to make routine variations to the dosage depending on the age and condition of the patient. The dosage will also depend on the route of administration which may be by a variety of routes including oral, aerosol, rectal, transdermal, subcutaneous, intravenous, intramuscular, intraperitoneal and intranasal.

Herein is disclosed a pharmaceutical composition comprising the compounds according to the invention and one or more other drugs in the treatment, prevention, control, amelioration, or reduction of risk of diseases or conditions for which compounds of Formula 1 or the other drugs may have utility as well as to the use of such a composition for the manufacture of a medicament.

### THERAPEUTIC USES

The compounds provided in this invention are positive allosteric modulators of III group of metabotropic receptors, in particular they are positive allosteric modulators of mGluR4. mGluR4 is expressed predominantly on the presynaptic membrane in the central nervous system where it is functions as an auto- and heteroreceptor to regulate the release of both GABA and glutamate. Furthermore, mGluR4 is also located in some postsynaptic locations. mGluR4 expressed in most areas of the brain, particularly in neurons responsible for the following functions: learning and memory; motor learning; regulation of voluntary movement and other functions; emotional responses; vision and olfaction; sleep and wakefulness; cerebellar functions and other.

The compounds of the present invention do not appear to bind to the glutamate recognition site, the orthosteric ligand site, but instead to an allosteric site within the seven transmembrane region of the receptor. In the presence of glutamate or an agonist of mGluR4, the compounds of this invention increase the mGluR4 response. The compounds provided in this invention are expected to have their effect at mGluR4 by virtue of their ability to increase the response of such receptors to glutamate or mGluR4 agonists, enhancing the response of the receptor. Hence, the present invention relates to a compound for use as a medicine, as well as to the use of a compound according to the invention or a pharmaceutical composition according to the invention for the manufacture of a medicament for treating or preventing a condition in a mammal, including a human, the treatment or prevention of which is affected or facilitated by the neuromodulatory effect of mGluR4 allosteric modulators, in particular positive mGluR4 allosteric modulators.

Also, the present invention relates to the use of a compound according to the invention or a pharmaceutical composition according to the invention for the manufacture of a medicament for treating, or preventing, ameliorating, controlling or reducing the risk of various neurological and psychiatric disorders associated with glutamate dysfunction in a mammal, including a human, the treatment or prevention of which is affected or facilitated by the neuromodulatory effect of mGluR4 positive allosteric modulators.

Where the invention is said to relate to the use of a compound or composition according to the invention for the manufacture of a medicament for e.g. the treatment of a mammal, it is understood that such use is to be interpreted in mal, comprising administering to a mammal in need of such e.g. a treatment, an effective amount of a compound or composition according to the invention.

An appropriate dosage level for the use of the mGluR4 positive allosteric modulators of the present invention as therapeutic agents will generally be about 0.01 to 500 mg per kg body weight of patient per day which can be administered in single or multiple doses. Preferably, the dosage level will be about 0.1 to about 250 mg/kg/day and more preferably about 0.5 to about 100 mg/kg/day. For oral administration, the compositions are preferably provided in the form of tablets containing 1.0 to 1000 mg of the active ingredient. The compounds may be administered on a regimen of 1 to 3 times per day, preferably once or twice daily. This dosage regimen may be adjusted to provide the optimal therapeutic response.

Exemplary diseases modulated by mGluR4, for which the compounds of the invention may useful, are central nervous system disorders for example: Parkinson's disease, parkinsonism and other disorders involving akinesia or bradykinesia; dystonia; Huntington's disease and other disorders involving involuntary movements and dyskinesias; schizophrenia and other psychotic disorders; pain; anxiety disorders; mood disorders (including depression, mania, bipolar disorders); addictive disorders; epilepsy; posttraumatic stress disorders; Alzheimer's disease; dementia and other form of neurodegeneration.

Because such positive allosteric modulators of mGluR4, including compounds of Formula 1, enhance the response of mGluR4 to glutamate, it is an advantage that the present methods utilize endogenous glutamate.

Because positive allosteric modulators of mGluR4, including compounds of Formula 1, enhance the response of mGluR4 to agonists, it is understood that the present invention extends to the treatment of neurological and psychiatric disorders associated with glutamate dysfunction by administering an effective amount of a positive allosteric modulator of mGluR4, including compounds of Formula 1, in combination with an mGluR4 agonist.

The compounds of the present invention may be utilized in combination with one or more other drugs in the treatment, prevention, control, amelioration, or reduction of risk of diseases or conditions for which compounds of Formula 1 or the other drugs may have utility, where the combination of the drugs together are safer or more effective than either drug alone.

### PHARMACOLOGY

The compounds provided in the present invention are allosteric modulators of III group of metabotropic glutamate receptors, subtype 4. As such, these compounds do not appear to bind to the orthosteric glutamate recognition site, and do not active the III group of mGluRs by themselves. Instead, the response to an endogenous ligand glutamate or other mGlu4 receptor agonists is increased when compounds of Formula 1 are present. Compounds of Formula 1 are expected to have their effect at III group of mGluRs, particularly subtype 4, by virtue of their ability to enhance the function of the receptor.

### ASSAY DESCRIPTION

### mGluR4 assay on T-Rex™-293 Cell line - expressing recombinant human mGlu4 receptor.

The compounds of the present invention are positive allosteric modulator of III group of metabotropic glutamate receptors, subtype 4. Their activity was examined on recombinant human mGlu4a receptor by detecting level of cyclic AMP in the presence of forskolin, adenylyl cyclase activator. The positive allosteric modulators provided in the present invention are expected to increase the functional activity and/or maximal efficacy of glutamate or other orthosteric agonists at mGluR4 receptor.

### Transfection and Cell culture

**Plazmid construct.** The cDNA fragment encoding human metabotropic receptor 4, variant 1 (accession number: NM_000841, NCBI Nucleotide Database) was cloned into the BamHI/NotI sites of pcDNA5/FRT/TO vector, under the control of a tetracycline regulated promoter (Invitrogen, Poland).

**Cell culture and transfection.** HEK293 cell line containing an inducible expression system (Flp-In T-Rex 293 cell line, Invitrogen, Poland) was maintained in Dulbecco's modified Eagle's medium supplemented with 10% fetal bovine serum, 2mM L-Glutamine (supplemented DMEM, LONZA, Poland), blastycidin and zeocin. T-Rex 293 cells were transfected with mixture of pTet-*h*mGluR4 and pOG44 (Invitrogen, Poland) plazmids and GeneJuice transfection reagent (Merck Millipore, Poland) according to the manufacturer's instructions. Cells were grown in supplemented DMEM with hygromycin B (75µg/ml) allowed selection of cells with stably integrated one copy of pTet-*h*mGluR4 plazmid. Expression of mGluR4 is induced by Tetracycline treatment.

**RT-PCR analysis of mGlu4 receptor expression.** Stable transfected cells were grown to 60% confluency with supplemented DMEM. Then 0,75µg/ml tetracycline was added and after 24h of GRM4 gene induction, cells were washed twice with PBS and collected in RNA isolation reagent (Trizol, Invitrogen, Poland). Then reverse transcription (1h, 37°C) and polymerase chain reaction (95°C 30s, 58°C 30s, 72°C 45s) was performed (3000 Thermocycler, Biometra, Poland). Results were visualized on 1% agarose gel propidium iodide stained.

**Western Blot analysis of mGlu4 receptor expression.** After 48 h of growth in medium with tetracycline, cells stable transfected with pTet-*h*mGluR4 plazmid were washed twice with PBS and solubilized in lysis buffer containing 20mM Tris-HCl, 100mM NaCl, 1mM EDTA, 0,5% Triton X-100, and protease inhibitor cocktail (Thermo Scientific, Poland) for 20 min on ice. After centrifugation at 20.000 x g for 15 min at 4°C, supernatants were mixed with 2 x Leammli buffer. 10µg of proteins from cell lysates were separated on 8% SDS-PAGE gels and transferred to nitrocellulose membranes according to standard Western Blot procedure. The blots were incubated with primary antibodies for 2h in RT (ab53088, AbCam, Poland) and secondary HRP conjugated antibodies for 1h in RT (12-348, Promega, Poland). Detection was performed using Pierce ECL western blotting substrate (Thermo Scientific, Poland) and analysis system (Fuji Film LAS-4000, Poland).

### Direct quantitative determination level of cyclic AMP by Homogenous Time - Resolved Fluorescence

**Cell preparation before assay.** Stable transfected cells were grown to 60% confluency with supplemented DMEM. Two days before experiment mGluR4 expression was induced by adding tetracycline in concentration 0,75µg/ml. 40h before experiment cells were incubated in a L-Glutamine free medium.

**Cyclic AMP measurement.** cAMP was measured quantitatively by cAMP dynamic 2 (Cisbio Biossays, France) according to manufacture instructions. cAMP dynamic 2 assay (Cisbio Biossays, France) is method based on homogenous time-resolved fluorescence. cAMP dynamic 2 is a competitive immunoassay between native cAMP produced by cells and the cyclic AMP labeled with, a fluorescence dye. The tracer binding is visualized by antibody anti - cAMP labeled with Eu³⁺ cryptate. The specific signal is inversely proportional to the concentration of cAMP in the sample.

Incubation of cells with all compounds was performed in 96 well plates. Each well were supplement with HHB containing 130 mM NaCl, 5,4 mM KCl, 1,8 mM CaCl₂, 0,8 mM MgSO₄, 0,9 mM NaH₂PO₄, 25 mM glucose and 20 mM HEPES, pH 7.4 with or without glutamate and with or without tested compound and forskolin (10 uM final concentration) in total volume of 50 ul. Assay was started by adding 50 ul of HHB buffer with cells to each well. Assay was performed in 37° C. After 5 min of incubation 10 ul of suspension containing 2000 cells were transferred to white 384 well plates (784080, Greiner bio-one) containing of 5 ul of cAMP-d2 conjugate in lysis buffer (cAMP dynamic 2, Cisbio Bioassays, France) in each well. Than was added 5 ul of cryptate conjugate in lysis buffer (cAMP dynamic 2, Cisbio Bioassays, France). After 1 h incubation in room temperature the fluorescence intensity of the assay plate were measured at 314 nm excitation, and 620 nm and 665 nm emission in Infinite M1000 (Tecan) microplate reader. Data were expressed as the ratio of 665 nm/620 nm. All liquid handling operations were performed using EVO 2000 system (Tecan, Switzerland).

Potentiation of the glutamate response of mGluR4 receptor by the invented compounds was observed as a decrease of EC₅₀ value for the glutamate in the presence of these molecules in the compare of EC₅₀ value in the absence of compounds. A decrease in the EC₅₀ for glutamate in the presence of tested compound was an indicator of degree of mGluR4 positive allosteric modulation at a given concentration of the sample compound. It also indicated (present as a % Glu Max) whether the sample compounds also affect the maximum activity of mGluR4 receptor.

The concentration response curves of representative compounds of the present invention were generated using the non-linear regression analysis program, GraphPad Prism version 5.03 (GraphPad Software, Inc., San Diego, USA). The curves were fitted to a four-parametric logistic equation: (Y=Bottom + (Top-Bottom)/(1+10^((LogEC₅₀-X)^{∗}Hill Slope) allowing the determination of EC₅₀ values.

### ACTIVITY OF SELECTED COMPOUNDS

The Table 3 below represents the mean EC₅₀ and % Glu Max obtained from at least three independent experiments of selected compounds performed in duplicates.

The "% Glu Max" is defined as a percent of activity mGluR4 receptor in the presence of tested compound and glutamate in concentration of the glutamic acid, which maximally activate mGluR4 receptor (100%).

### Table 3: Activity data for selected exemplary compounds

**Table 3**

| **Activity** | | **Example** |
|---|---|---|
| **EC₅₀ [µM]** | **% Glu Max** | |
| EC₅₀ < 0.3 µM | 104.7 - 118.0 | 27, 28, 30, 49, 39 |
| 0.3 mM < EC₅₀ <1 µM | 104.6 - 134.9 | 31, 50, 6, 26, 38, 40 |
| 1 mM < EC₅₀ < 3 µM | 99.2 - 118.6 | 17, 19, 22, 54, 42, 32, 44, 33, 41, 29, 46, 48, 47, 34 |

The results shown in Table 3 demonstrate activity of the compounds described in the present invention. Compounds listed in the Table 3 are positive allosteric modulators of mGluR4 in the aforementioned assay generally with an EC₅₀ for potentiation of less than about 3 µM. The disclosed compounds have activity in potentiating human recombinant mGluR4 receptor with EC₅₀ for potentiation of less than about 300 nM. The activity of human recombinant mGluR4 receptor to a concentration of glutamate is increased in the presence of compounds provided in the present invention and listed in Table 3. These compounds increase the functional activity and/or maximal efficacy of glutamate to stimulate receptor mGluR4 activity.

Thus, the positive allosteric modulators provided in the present invention are expected to increase the effectiveness of glutamate, or mGluR4 agonists.

Therefore, these compounds are expected to be useful for treatment of various neurological and psychiatric disorders associated with glutamate transmission dysfunction described to be treated herein and others that can be treated by such positive allosteric modulators.

The compounds of the present invention can be administered either alone, or in combination with other pharmaceutical agents effective in the treatment of conditions mentioned above.

## Claims

1. 1,2,4-oxadiazole derivatives selected from the group consisting of
N-{4-[5-(4-methoxyphenyl)-1,2,4-oxadiazol-3-yl]phenyl}pyridine-4-carboxamide,
N-{4-[5-(2-chlorophenyl)-1,2,4-oxadiazol-3-yl]-3-methoxyphenyl}pyridine-2-carboxamide;
N-{4-[5-(2-chloro-4-fluorophenyl)-1,2,4-oxadiazol-3-yl]-3-methoxyphenyl} pyridine-2-carboxamide;
N-{3-chloro-4-[5-(2-chloro-4-fluorophenyl)-1,2,4-oxadiazol-3-yl]phenyl}pyridine-2-carboxamide;
N-{4-[5-(2-chloro-4-fluorophenyl)-1,2,4-oxadiazol-3-yl]-3-fluorophenyl} pyridine-2-carboxamide;
N-{4-[5-(2-methoxyphenyl)-1,2,4-oxadiazol-3-yl]-3-fluorophenyl}pyridine-2-carboxamide;
N-[4-(5-phenyl-1,2,4-oxadiazol-3-yl)-3-trifluoromethyl)phenyl]pyridine-2-carboxamide;
N-{4-[5-(2-chlorophenyl)-1,2,4-oxadiazol-3-yl]-3-(trifluoromethyl)phenyl} pyridine-2-carboxamide;
N-[3-methyl-4-(5-phenyl-1,2,4-oxadiazol-3-yl)phenyl]pyridine-2-carboxamide;
N-{4-[5-(2-chlorophenyl)-1,2,4-oxadiazol-3-yl]-3-methylphenyl}pyridine-2-carboxamide;
N-{4-[5-(2-chlorophenyl)-1,2,4-oxadiazol-3-yl]-3-methoxyphenyl}-6-fluoropyridine-2-carboxamide;
5-chloro-N-{4-[5-(2-chlorophenyl)-1,2,4-oxadiazol-3-yl]-3-methoxyphenyl}-3-fluoropyridine-2-carboxamide;
6-chloro-N-{4-[5-(2-chlorophenyl)-1,2,4-oxadiazol-3-yl]-3-methoxyphenyl}pyridine-2-carboxamide
N-{4-[5-(2-chlorophenyl)-1,2,4-oxadiazol-3-yl]-3-methoxyphenyl} pyrimidine-4-carboxamide
N-{4-[5-(2-chlorophenyl)-1,2,4-oxadiazol-3-yl]-3-methoxyphenyl}thiophene-2-carboxamide;
6-fluoro-N-{4-[5-(2-fluorophenyl)-1,2,4-oxadiazol-3-yl]-3-methoxyphenyl} pyridine-2-carboxamide;
3,6-dichloro-N-{4-[5-(2-fluorophenyl)-1,2,4-oxadiazol-3-yl]-3-methoxy phenyl}pyridine-2-carboxamide;
N-{3-chloro-4-[5-(2-chlorophenyl)-1,2,4-oxadiazol-3-yl]phenyl}-6-fluoropyridine-2-carboxamide;
N-(2-chlorophenyl)-4-(5-phenyl-1,2,4-oxadiazol-3-yl)benzene-1-sulfonamide;
N-(2,4-difluorophenyl)-4-[5-(pyridin-2-yl)-1,2,4-oxadiazol-3-yl]benzene-1-sulfonamide;
N-[4-(3-phenyl-1,2,4-oxadiazol-5-yl)phenyl]benzamide;
3-chloro-N-[4-(3-phenyl-1,2,4-oxadiazol-5-yl)phenyl]benzamide;
4-methoxy-N-[4-(3-phenyl-1,2,4-oxadiazol-5-yl)phenyl]benzamide;
4-nitro-N-[4-(3-phenyl-1,2,4-oxadiazol-5-yl)phenyl]benzamide;
1-methyl-N-[4-(3-phenyl-1,2,4-oxadiazol-5-yl)phenyl]-1H-pyrrole-2-carboxamide;
or pharmaceutically acceptable salts, hydrates or solvates thereof, for use as positive allosteric modulator ligands of metabotropic glutamate receptors of group III subtype 4 (mGluR4) in the treatment of the central nervous disorders modulated by mGluR4 receptors.

2. The 1,2,4-oxadiazole derivatives for use according to Claim 1, either alone, or in combination with already existing drugs, wherein the central nervous disorders modulated by mGlu4 receptors is selected from Parkinson's disease, parkinsonism and other disorders involving akinesia or bradykinesia; dystonia; Huntington's disease and other disorders involving involuntary movements and dyskinesias; schizophrenia and other psychotic disorders; pain; anxiety disorders; mood disorders (including depression, mania, bipolar disorders); addictive disorders; epilepsy; posttraumatic stress disorders; Alzheimer's disease; dementia and other form of neurodegeneration.

## Patentansprüche

1. 1,2,4-Oxadiazolderivate ausgewählt aus der Gruppe bestehend aus
N-{4-[5-(4-Methoxyphenyl)-1,2,4-oxadiazol-3-yl]phenyl}pyridin-4-carboxamid,
N-{4-[5-(2-Chlorphenyl)-1,2,4-oxadiazol-3-yl]-3-methoxyphenyl}pyridin-2-carboxamid;
N-{4-[5-(2-Chlor-4-fluorphenyl)-1,2,4-oxadiazol-3-yl]-3-methoxyphenyl}pyridin-2-carboxamid;
N-{3-Chlor-4-[5-(2-chlor-4-fluorphenyl)-1,2,4-oxadiazol-3-yl]phenyl}pyridin-2-carboxamid;
N-{4-[5-(2-Chlor-4-fluorphenyl)-1,2,4-oxadiazol-3-yl]-3-fluorphenyl}pyridin-2-carboxamid;
N-{4-[5-(2-Methoxyphenyl)-1,2,4-oxadiazol-3-yl]-3-fluorphenyl}pyridin-2-carboxamid;
N-[4-(5-Phenyl-1,2,4-oxadiazol-3-yl)-3-trifluormethyl)phenyl]pyridin-2-carboxamid;
N-{4-[5-(2-Chlorphenyl)-1,2,4-oxadiazol-3-yl]-3-(trifluormethyl)phenyl}pyridin-2-carboxamid;
N-[3-Methyl-4-(5-phenyl-1,2,4-oxadiazol-3-yl)phenyl]pyridin-2-carboxamid;
N-{4-[5-(2-Chlorphenyl)-1,2,4-oxadiazol-3-yl]-3-methylphenyl}pyridin-2-carboxamid;
N-{4-[5-(2-Chlorphenyl)-1,2,4-oxadiazol-3-yl]-3-methoxyphenyl}-6-fluorpyridin-2-carboxamid;
5-Chlor-N-{4-[5-(2-chlorphenyl)-1,2,4-oxadiazol-3-yl]-3-methoxyphenyl}-3-fluorpyridin-2-carboxamid;
6-Chlor-N-{4-[5-(2-chlorphenyl)-1,2,4-oxadiazol-3-yl]-3-methoxyphenyl}pyridin-2-carboxamid;
N-{4-[5-(2-Chlorphenyl)-1,2,4-oxadiazol-3-yl]-3-methoxyphenyl}pyrimidin-4-carboxamid;
N-{4-[5-(2-Chlorphenyl)-1,2,4-oxadiazol-3-yl]-3-methoxyphenyl}thiophen-2-carboxamid;
6-Fluor-N-{4-[5-(2-fluorphenyl)-1,2,4-oxadiazol-3-yl]-3-methoxyphenyl}pyridin-2-carboxamid;
3,6-Dichlor-N-{4-[5-(2-fluorphenyl)-1,2,4-oxadiazol-3-yl]-3-methoxyphenyl}pyridin-2-carboxamid;
N-{3-Chlor-4-[5-(2-chlorphenyl)-1,2,4-oxadiazol-3-yl]phenyl}-6-fluorpyridin-2-carboxamid;
N-(2-Chlorphenyl)-4-(5-phenyl-1,2,4-oxadiazol-3-yl)benzen-1-sulfonamid;
N-(2,4-Difluorphenyl)-4-[5-(pyridin-2-yl)-1,2,4-oxadiazol-3-yl]benzen-1-sulfonamid,
N-[4-(3-Phenyl-1,2,4-oxadiazol-5-yl)phenyl]benzamid,
3-Chlor-N-[4-(3-phenyl-1,2,4-oxadiazol-5-yl)phenyl]benzamid,
4-Methoxy-N-[4-(3-phenyl-1,2,4-oxadiazol-5-yl)phenyl]benzamid;
4-Nitro-N-[4-(3-phenyl-1,2,4-oxadiazol-5-yl)phenyl]benzamid;
1-Methyl-N-[4-(3-phenyl-1,2,4-oxadiazol-5-yl)phenyl]-1H-pyrrol-2-carboxamid,
oder pharmazeutisch verträgliche Salze, Hydrate oder Solvate davon zur Verwendung als positive allosterische Modulatorliganden von metabotropen Glutamatrezeptoren des Subtyps 4 der Gruppe III (mGluR4) bei der Behandlung der durch mGluR4-Rezeptoren modulierten Zentralnervenstörungen.

2. Die 1,2,4-Oxadiazolderivate zur Verwendung nach Anspruch 1, entweder allein oder in Kombination mit bereits vorhandenen Arzneimitteln, wobei die durch mGlu4-Rezeptoren modulierten Zentralnervenstörungen aus Parkinson-Krankheit, Parkinsonismus und anderen Störungen mit Akinesie oder Bradykinesie; Dystonie; Huntington-Krankheit und andere Störungen mit unwillkürlichen Bewegungen und Dyskinesien; Schizophrenie und andere psychotische Störungen; Schmerzen; Angststörungen; Stimmungsstörungen (einschließlich Depressionen, Manie, bipolare Störungen); Suchtstörungen; Epilepsie; posttraumatische Belastungsstörungen; Alzheimer-Krankheit; Demenz und andere Formen der Neurodegeneration, ausgewählt werden.

## Revendications

1. Dérivés de 1,2,4-oxadiazole choisis dans le groupe constitué de
N-{4-[5-(4-méthoxyphényl)-1,2,4-oxadiazol-3-yl]phényl}pyridine-4-carboxamide,
N-{4-[5-(2-chlorophényl)-1,2,4-oxadiazol-3-yl]-3-méthoxyphényl}pyridine-2-carboxamide ;
N-{4-[5-(2-chloro-4-fluorophényl)-1,2,4-oxadiazol-3-yl]-3-méthoxyphényl}pyridine-2-carboxamide ;
N-{3-chloro-4-[5-(2-chloro-4-fluorophényl)-1,2,4-oxadiazol-3-yl]phényl}pyridine-2-carboxamide ;
N-{4-[5-(2-chloro-4-fluorophényl)-1,2,4-oxadiazol-3-yl]-3-fluorophényle}pyridine-2-carboxamide ;
N-{4-[5-(2-méthoxyphényl)-1,2,4-oxadiazol-3-yl]-3-fluorophényl}pyridine-2-carboxamide;
N-[4-(5-phényl-1,2,4-oxadiazol-3-yl)-3-trifluorométhyl)phényl]pyridine-2-carboxamide;
N-{4-[5-(2-chlorophényl)-1,2,4-oxadiazol-3-yl]-3-(trifluorométhyle)phényl}pyridine-2-carboxamide;
N-[3-méthyl-4-(5-phényl-1,2,4-oxadiazol-3-yl)phényl]pyridine-2-carboxamide ;
N-{4-[5-(2-chlorophényl)-1,2,4-oxadiazol-3-yl]-3-méthylphényl}pyridine-2-carboxamide ;
N-{4-[5-(2-chlorophényl)-1,2,4-oxadiazol-3-yl]-3-méthoxyphényl}-6-fluoropyridine-2-carboxamide ;
5-chloro-N-{4-[5-(2-chlorophényl)-1,2,4-oxadiazol-3-yl]-3-méthoxyphényl}-3-fluoropyridine-2-carboxamide ;
6-chloro-N-{4-[5-(2-chlorophényl)-1,2,4-oxadiazol-3-yl]-3-méthoxyphényl}pyridine-2-carboxamide
N-{4-[5-(2-chlorophényl)-1,2,4-oxadiazol-3-yl]-3-méthoxyphényl}pyrimidine-4-carboxamide
N-{4-[5-(2-chlorophényl)-1,2,4-oxadiazol-3-yl]-3-méthoxyphényl}thiophène-2-carboxamide ;
6-fluor-N-{4-[5-(2-fluorophényle)-1,2,4-oxadiazol-3-yl]-3-méthoxyphényl} pyridine-2-carboxamide ;
3,6-dichloro-N-{4-[5-(2-fluorophényle)-1,2,4-oxadiazol-3-yl]-3-méthoxy phényl}pyridine-2-carboxamide ;
N-{3-chloro-4-[5-(2-chlorophényl)-1,2,4-oxadiazol-3-yl]phényl}-6-fluoropyridine-2-carboxamide ;
N-(2-chlorophényl)-4-(5-phényl-1,2,4-oxadiazol-3-yl)benzène-1-sulfonamide ;
N-(2,4-difluorophényl)-4-[5-(pyridin-2-yl)-1,2,4-oxadiazol-3-yl]benzène-1-sulfonamide ;
N-[4-(3-phényl-1,2,4-oxadiazol-5-yl)phényl]benzamide ;
3-chloro-N-[4-(3-phényl-1,2,4-oxadiazol-5-yl)phényl]benzamide;
4-méthoxy-N-[4-(3-phényl-1,2,4-oxadiazol-5-yl)phényl]benzamide ;
4-nitro-N-[4-(3-phényl-1,2,4-oxadiazol-5-yl)phényl]benzamide ;
1-méthyl-N-[4-(3-phényl-1,2,4-oxadiazol-5-yl)phényl]-1H-pyrrole-2-carboxamide ;
ou leurs sels, hydrates ou solvates pharmaceutiquement acceptables, à utiliser comme ligands modulateurs allostériques positifs des récepteurs métabotropes du glutamate du sous-type 4 du groupe III (mGluR4) dans le traitement des troubles du système nerveux central modulés par les récepteurs mGluR4.

2. Dérivés de 1,2,4-oxadiazole à utiliser selon la revendication 1, soit seuls, soit en association avec des médicaments déjà existants, où les troubles du système nerveux central modulés par les récepteurs mGlu4 sont choisis parmi la maladie de Parkinson, le parkinsonisme et d'autres troubles impliquant une akinésie ou une bradykinésie ; la dystonie ; la maladie de Huntington et d'autres troubles impliquant des mouvements involontaires et des dyskinésies ; la schizophrénie et d'autres troubles psychotiques ; la douleur ; les troubles anxieux ; les troubles de l'humeur (y compris dépression, manie, troubles bipolaires) ; les troubles addictifs ; l'épilepsie ; les troubles de stress post-traumatique ; la maladie d'Alzheimer ; la démence et d'autres formes de neurodégénérescence.
